(19) Europäisches Patentamt / European Patent Office / Office européen des brevets

(11) **EP 3 864 177 B1**

(12) **EUROPEAN PATENT SPECIFICATION**

(45) Date of publication and mention
of the grant of the patent:
**25.02.2026 Bulletin 2026/09**

(21) Application number: **19870843.0**

(22) Date of filing: **09.10.2019**

(51) International Patent Classification (IPC):
**C12Q 1/6883** (2018.01)

(52) Cooperative Patent Classification (CPC):
**C12Q 1/6883; C12Q 2600/154**

(86) International application number:
**PCT/US2019/055444**

(87) International publication number:
**WO 2020/076983 (16.04.2020 Gazette 2020/16)**

(54) **DNA METHYLATION BASED BIOMARKERS FOR LIFE EXPECTANCY AND MORBIDITY**

AUF DNA-METHYLIERUNG BASIERENDE BIOMARKER FÜR LEBENSERWARTUNG UND
MORBIDITÄT

BIOMARQUEURS BASÉS SUR LA MÉTHYLATION DE L'ADN POUR L'ESPÉRANCE DE VIE ET LA
MORBIDITÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.10.2018 US 201862744010 P**

(43) Date of publication of application:
**18.08.2021 Bulletin 2021/33**

(73) Proprietor: **The Regents of the University of
California
Oakland CA 94607-5200 (US)**

(72) Inventors:
• **HORVATH, Stefan
Los Angeles, California 90049 (US)**
• **LU, Ake Tzu-Hui
Los Angeles, California 90024 (US)**

(74) Representative: **Murgitroyd & Company
165-169 Scotland Street
Glasgow G5 8PL (GB)**

(56) References cited:
**WO-A1-2012/162139      US-A1- 2013 129 668
US-A1- 2015 259 742**

• XU GAO ET AL: "DNA methylation changes of
whole blood cells in response to active smoking
exposure in adults: a systematic review of DNA
methylation studies", CLINICAL EPIGENETICS,
BIOMED CENTRAL LTD, LONDON, UK, vol. 7, no.
1, 16 October 2015 (2015-10-16), pages 113,
XP021230180, ISSN: 1868-7083, DOI: 10.1186/
S13148-015-0148-3
• VERSCHOOR CHRIS P ET AL: "DNA methylation
patterns are related to co-morbidity status and
circulating C-reactive protein levels in the
nursing home elderly", EXPERIMENTAL
GERONTOLOGY, ELSEVIER, AMSTERDAM, NL,
vol. 105, 12 October 2017 (2017-10-12), pages 47 -
52, XP085365760, ISSN: 0531-5565, DOI: 10.1016/
J.EXGER.2017.10.010
• LU AKE T ET AL: "DNA methylation GrimAge
strongly predicts lifespan and healthspan11",
vol. 11, no. 2, 21 January 2019 (2019-01-21),
pages 303 - 327, XP055928827, Retrieved from
the Internet <URL:https://www.aging-us.com/
article/101684/pdf> DOI: 10.18632/aging.101684
• YAN ZHANG ET AL: "DNA methylation
signatures in peripheral blood strongly predict
all-cause mortality", NATURE
COMMUNICATIONS, vol. 8, 17 March 2017
(2017-03-17), pages 14617, XP055465751, DOI:
10.1038/ncomms14617

EP 3 864 177 B1

Remarks:
The complete document including Reference
Table(s) and the Sequence Listing(s) can be
downloaded from the EPO website

Remarks:
The complete document including Reference
Table(s) and the Sequence Listing(s) can be
downloaded from the EPO website

**Description**

TECHNICAL FIELD

The invention relates to methods and materials for examining biological aging in individuals.

BACKGROUND OF THE INVENTION

Studies in invertebrates (yeast, worm, flies) have led to a long list of pharmacological agents that promise to intervene in different aspects of the aging process including stress response mimetics, anti-inflammatory interventions, epigenetic modifiers, neuroprotective agents, hormone treatments. While our arsenal of potential anti-aging interventions is brimming with highly promising candidates that delay aging in model organisms, it remains to be seen whether these interventions delay aging in human cells. To facilitate effective in vitro and *ex vivo* studies, there is a need for robust biomarkers of aging for human fibroblasts and other widely used cell types. One potential biomarker that has gained significant interest in recent years is DNA methylation (DNAm). Chronological time has been shown to elicit predictable hypo- and hyper-methylation changes at many regions across the genome. Several DNAm based biomarkers of aging have been developed including those using blood-based algorithms and the multi-tissue algorithms. These epigenetic age estimators exhibit statistically significant associations with many age-related diseases and conditions.

Recently developed DNA methylation-based biomarkers allow one to estimate the epigenetic age of an individual. For example, the pan tissue epigenetic clock, which is based on 353 dinucleotide markers, known as CpGs (-C-phosphate-G-), can be used to estimate the age of most human cell types, tissues, and organs (Horvath S. DNA methylation age of human tissues and cell types. Genome Biol. 2013; 14(R115). The estimated age, referred to as "DNA methylation age" (DNAm age), correlates with chronological age when methylation is assessed in certain cell types, tissues, and organs including whole blood, brain, breast, kidney, liver, lung, skin and saliva. Other reports described DNAm-based biomarkers that pertain to a single tissue (e.g. saliva or blood). Recent studies suggested that DNAm-based biomarkers of age capture aspects of biological age. For example, we and others have previously shown that individuals whose DNAm age was greater than their chronological age, i.e. individuals who exhibited epigenetic "age acceleration", were at an increased risk for death from all causes, even after accounting for known risk factors.

XU GAO ET AL: "DNA methylation changes of whole blood cells in response to active smoking exposure in adults: a systematic review of DNA methylation studies", CLINICAL EPIGENETICS, vol. 7, no. 1, 16 October 2015 (2015-10-16), page 113, is a review paper relating to DNA methylation changes of whole blood cells in response to active smoking exposure in adults in order to quantify long-term smoking exposure and evaluate the risks of smoking-induced diseases and reviews methods of determining the number of years in which an individual has smoked in their lifetime.

There is a need for improved methods of observing phenomena associated with epigenetic aging, independent of chronological age and traditional risk factors of mortality, as well as improved methods for observing the effects of one or more test agents on the epigenetic aging of human cells.

SUMMARY OF THE INVENTION

The invention disclosed herein provides methods and materials designed to observe DNA methylation levels at selected sites within the human genome. Using these methods and materials, embodiments of the invention provide a number of different biomarkers useful for predicting human lifespan, i.e. time to death, based on DNA methylation levels in genomic DNA obtained from samples such as blood tissue, blood cells, saliva, or buccal swabs. It will be appreciated that the scope of the invention is in accordance with the claims. Accordingly, there is provided a method for predicting lifespan of an individual as defined in claim 1. There is also provided a method of observing the effects of a test agent on genomic methylation associated epigenetic aging of human cells as defined in claim 8. Further features are provided in accordance with the dependent claims. The specification may include description of embodiments outside the scope of the claims provided as background and to assist in understanding the invention. As discussed in detail below, embodiments of the invention observe methylation levels at a variety sites within the human genome in order to obtain information on a variety of phenomena associated with aging such as life expectancy, mortality, and morbidity. Disclosure that focuses on the prediction of mortality and morbidity in humans show that these DNAm based biomarkers are highly robust and informative for a range of applications. As discussed in detail below, embodiments of the invention include methods of observing genomic methylation in an individual in order to obtain information on one or more physiological factors associated with an epigenetic age of the individual, as well as methods of observing the effects of one or more test agents on genomic methylation that is associated with the epigenetic aging of human cells.

Embodiments of the invention can be used to provide information that complements and enhances conventional biomarker assessments that are widely used in clinical applications. For example, aspects of the invention can be used to directly predict/prognosticate mortality, as well as provide further information on a host of age-related conditions such as cardiovascular disease, cancer risk, progression in neurodegeneration, and various measures of frailty. Aspects of the invention can also be used to estimate the plasma levels of a number of different proteins associated with aging. In addition, one aspect of the invention can be used to provide highly accurate information on the numbers of cigarettes smoked by an individual in their lifetime.

The biomarker embodiment disclosed herein and referred to as "DNAm GrimAge" is based on DNA methylation measurements at 1113 cytosine-phosphate-guanines (CpG) locations within the human genome. Significantly, this DNAm GrimAge methylation measurement at 1113 locations is collectively based on eight individual DNAm based biomarker assays at selected subsets of CpG locations, subsets of biomarkers that provide information on the numbers of cigarettes smoked by an individual (more precisely smoking packyears) as well as the *in vivo* plasma levels of: (1) adrenomedullin (ADM); (2) beta-2-microglobulin (B2M); (3) cystatin-C; (4) growth differentiation factor 15 (GDF15); (5) leptin; (6) plasminogen activator inhibitor 1 (PAI1); and (7) tissue inhibitor metalloproteinases 1 (TIMP1). Interestingly, plasma levels of ADM, B2M, cystatin C, and leptin relate to many age-related traits including cognitive functioning. For example, ADM levels are observed to be increased in individuals with hypertension and heart failure. Plasma B2M is a clinical biomarker associated with cardiovascular disease, kidney function, and inflammation. Plasma cystatin-C can be used to assess kidney function. Moreover, GDF-15 is involved in age-related mitochondrial dysfunction and PAI-1 plays a central role in a number of age-related subclinical and clinical conditions (and recent genetic studies link PAI-1 to lifespan).

Methods of obtaining information on one or more physiological factors associated with an age of an individual comprise obtaining genomic DNA from the individual, observing methylation of the genomic DNA in at least about 42 methylation markers from the group of methylation markers in SEQ ID NO: 1-SEQ ID NO: 1113, and then correlating observed methylation in the methylation markers with the one or more physiological factors associated with the age of an individual such that information on the one or more physiological factors associated with the age of an individual is obtained. Typically, the genomic DNA is obtained from human fibroblasts, keratinocytes, buccal cells, endothelial cells, lymphoblastoid cells, and/or cells obtained from blood, skin, dermis, epidermis or saliva. In typical embodiments of the invention, methylation is observed by a process comprising treatment of genomic DNA from the population of cells from the individual with bisulfite to transform unmethylated cytosines of CpG dinucleotides in the genomic DNA to uracil; and/or genomic DNA is hybridized to a complimentary polynucleotide sequence disposed on a microarray. Optionally in these embodiments, correlating observed methylation in the methylation markers comprises a regression analysis.

In addition, the invention observes specific constellations of markers in SEQ ID NO: 1-SEQ ID NO: 1113. For example, in the claimed invention, the one or more physiological factors associated with an age of an individual comprises predicted age (not recited in the claims) or lifespan of an individual, or the time to coronary heart disease in the individual (not recited in the claims), the method comprising observing methylation of the genomic DNA in 1113 methylation markers from the group of methylation markers in SEQ ID NO: 1-SEQ ID NO: 1113, and then correlating observed methylation in the 1113 methylation markers with predicted age or lifespan of an individual, or the time to coronary heart disease in the individual, such that information on predicted age or lifespan of an individual, or the time to coronary heart disease in the individual is obtained. The one or more physiological factors associated with an age of an individual comprises a number of years in which the individual has smoked in their lifetime, and the method comprises observing methylation of the genomic DNA in 172 methylation markers from the group of methylation markers in SEQ ID NO: 1-SEQ ID NO: 172, and then correlating observed methylation in the 172 methylation markers with the number of years in which an individual has smoked in their lifetime, such that information on the number of years in which the individual has smoked in their lifetime is obtained. Additionally, the one or more physiological factors associated with an age of an individual comprises plasma protein levels of tissue inhibitor metalloproteinase 1 in the individual, and the method comprises observing methylation of the genomic DNA in 42 methylation markers from the group of methylation markers in SEQ ID NO 173-SEQ ID NO: 214, and then correlating observed methylation in the 42 methylation markers with plasma protein levels of tissue inhibitor metalloproteinase 1 in an individual, such that information on plasma protein levels of tissue inhibitor metalloproteinase 1 in an individual is obtained. Additionally, the one or more physiological factors associated with an age of an individual comprises plasma protein levels of Cystatin-C in the individual, the method comprising observing methylation of the genomic DNA in 87 methylation markers from the group of methylation markers in SEQ ID NO: 215-SEQ ID NO: 301, and then correlating observed methylation in the 87 methylation markers with plasma protein levels of Cystatin-C in an individual, such that information on plasma protein levels of Cystatin-C in an individual is obtained. Additionally, the one or more physiological factors associated with an age of an individual comprises plasma protein levels of Beta-2-microglobulin in the individual, the method comprising observing methylation of the genomic DNA in 91 methylation markers from the group of methylation markers in SEQ ID NO 302-SEQ ID NO: 392, and then correlating observed methylation in the 91 methylation markers with plasma protein levels of Beta-2-microglobulin in an individual, such that information on plasma protein levels of Beta-2-microglobulin in an individual is obtained. Additionally, the one or more physiological factors associated with an age of an individual comprises plasma protein levels of growth differentiation factor 15 in the individual, the method comprising observing methylation of the genomic DNA in 137 methylation markers from the group of methylation markers in SEQ ID NO: 393-SEQ ID NO: 529, and then correlating observed methylation in the 137 methylation markers with plasma protein levels of growth differentiation factor 15 in an individual, such that information on plasma protein levels of growth differentiation factor 15 in an individual is obtained. Additionally, the one or more physiological factors associated with an age of an individual comprises plasma protein levels of adrenomedullin in the individual, the method comprising observing methylation of the genomic DNA in 186 methylation markers from the group of methylation markers in SEQ ID NO: 530-SEQ ID NO: 715, and then correlating observed methylation in the 186 methylation markers with plasma protein levels of adrenomedullin in an individual, such that information on plasma protein levels of adrenomedullin in an individual is obtained. Additionally, the one or more physiological factors associated with an age of an individual comprises plasma protein levels of Leptin in the individual, the method comprising observing methylation of the genomic DNA in 187 methylation markers from the group of methylation markers in SEQ ID NO: 716-SEQ ID NO: 902, and then correlating observed methylation in the 187 methylation markers with Leptin in an individual, such that information on plasma protein levels of Leptin in an individual is obtained. Additionally, the one or more physiological factors associated with an age of an individual comprises plasma protein levels of plasminogen activator inhibitor 1 in the individual, the method comprising observing methylation of the genomic DNA in 211 methylation markers from the group of methylation markers in SEQ ID NO: 903-SEQ ID NO: 1113, and then correlating observed methylation in the 211 methylation markers with plasminogen activator inhibitor 1 in an individual, such that information on plasma protein levels of plasminogen activator inhibitor 1 in an individual is obtained.

Yet another embodiment of the invention, as recited in claim 8, is a method of observing the effects of a test agent on genomic methylation associated epigenetic aging of human cells. The method comprise combining the test agent with human cells, observing methylation status in at least 172 of the methylation markers of SEQ ID NO: 1-SEQ ID NO: 172 in genomic DNA from the human cells, and then comparing the observations from (b) with observations of the methylation status in at least 172 of methylation markers of SEQ ID NO: 1-SEQ ID NO: 172 in genomic DNA from control human cells not exposed to the test agent such that effects of the test agent on genomic methylation associated epigenetic aging in the human cells is observed. Typically, the method comprises observing methylation of the genomic DNA in 172 methylation markers from the group of methylation markers in SEQ ID NO: 1-SEQ ID NO: 172; and/or observing methylation of the genomic DNA in 42 methylation markers from the group of methylation markers in SEQ ID NO: 173-SEQ ID NO: 214; and/or observing methylation of the genomic DNA in 87 methylation markers from the group of methylation markers in SEQ ID NO: 215-SEQ ID NO: 301; and/or observing methylation of the genomic DNA in 91 methylation markers from the group of methylation markers in SEQ ID NO: 302-SEQ ID NO: 392; and/or observing methylation of the genomic DNA in 137 methylation markers from the group of methylation markers in SEQ ID NO: 393-SEQ ID NO: 529; and/or observing methylation of the genomic DNA in 186 methylation markers from the group of methylation markers in SEQ ID NO: 530-SEQ ID NO: 715; and/or observing methylation of the genomic DNA in 187 methylation markers from the group of methylation markers in SEQ ID NO: 716-SEQ ID NO: 902; and/or observing methylation of the genomic DNA in 211 methylation markers from the group of methylation markers in SEQ ID NO 903-SEQ ID NO: 1113; and/or observing methylation of the genomic DNA in about 1113 methylation markers of SEQ ID NO: 1-SEQ ID NO: 1113. Optionally in these methods, a plurality of test agents are combined with the human cells *in vitro* (e.g. primary keratinocytes from a single or, alternatively, multiple donors. In illustrative embodiments of the invention, the test agent is a polypeptide, a polynucleotide or a compound having a molecular weight less than 3,000, 2,000, 1,000 or 500 g/mol.

In certain embodiments of the methods disclosed above, the genomic DNA can be obtained from human fibroblasts, keratinocytes, buccal cells, endothelial cells, lymphoblastoid cells, and/or cells obtained from blood, skin, dermis, epidermis or saliva. In typical embodiments of the invention, methylation is observed by a process comprising treatment of genomic DNA from the population of cells from the individual with bisulfite to transform unmethylated cytosines of CpG dinucleotides in the genomic DNA to uracil. Optionally in such methods, genomic DNA is hybridized to a complimentary sequence disposed on a microarray, and/or genomic DNA is amplified by a polymerase chain reaction process. In some embodiments of the invention, methylation in at least 75% of the respective groups of methylation markers in a constellation of markers is observed (e.g. a constellation of 172 markers, a constellation of 42 markers etc.). In certain embodiments of the invention, correlating observed methylation in the methylation markers comprises a regression analysis, for example one that also considers the sex of the individual, and/or the ethnicity/race of the individual. Optionally for example, correlating observed methylation in the methylation markers comprises a regression analysis selected to predict time to coronary heart disease in the individual.

As discussed below, the DNAm GrimAge biomarker disclosed herein can supplement and enhance existing conventional lifespan predictors. In addition, the subsets of DNAm based biomarkers (e.g. for smoking pack-years and protein plasma levels) have interesting applications in their own right. For example, the DNAm based surrogate biomarker of smoking pack-years could complement self-reported assessments of pack-years. The surprising fact that DNAm pack-years outperforms self-reported pack-years when it comes to lifespan prediction could reflect erroneous self-reporting or true biology: maybe DNAm pack-years is a superior measure of long-term exposure to smoke, or of biological response to it. Moreover, the DNAm based surrogate biomarkers of plasma protein levels disclosed herein can be used, for example, by epidemiologists who have access to stored DNA samples but no access to plasma samples.

Other objects, features and advantages of the present invention will become apparent to those skilled in the art from the following detailed description.

BRIEF DESCRIPTION OF THE DRAWINGS

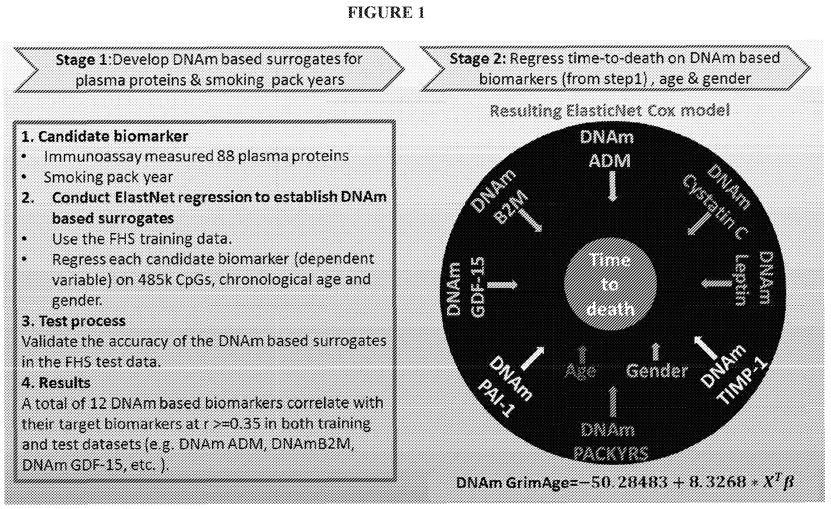

**Figure 1** provides a schematic of a flowchart for stages of the development and data from the DNAm GrimAge methodology disclosed herein. Surrogate DNAm based biomarkers for smoking pack-years and plasma protein levels were defined and validated using training and test data from the Framingham Heart study (stage 1). Only 12 out of 88 plasma proteins exhibited a correlation r >0.35 with their respective DNAm based surrogate marker in the test data. In stage 2, time-to-death (due to all-cause mortality) was regressed on chronological age, sex, and DNAm based biomarkers of smoking pack-years and the 12 above mentioned plasma protein levels. The ElasticNet regression model automatically selected the following covariates: chronological age (Age), sex (Female), and DNAm based surrogates for smoking pack-years (DNAm PACKYRS), adrenomedullin levels (DNAm ADM), beta-2 microglobulin (DNAm B2M), cystatin C (DNAm Cystatin C), growth differentiation factor 15 (DNAm GDF-15), leptin (DNAm Leptin), plasminogen activation inhibitor 1 (DNAm PAI-1), tissue inhibitor metalloproteinase 1 (DNAm TIMP-1). The linear combination of the covariate values $X^T\beta$ was linearly transformed to be in units of years. Technically speaking DNAm GrimAge is a mortality risk estimator. Metaphorically speaking, it can be interpreted as a DNAm based estimator of biological age or as another epigenetic clock.

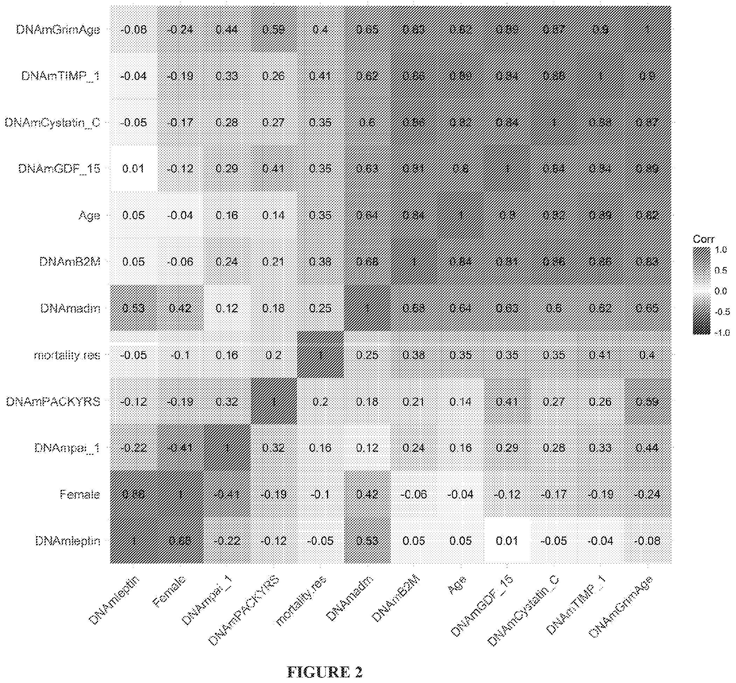

**Figure 2** provides a heat map of pairwise correlations of DNAm based biomarkers.

The heat map codes the pairwise Pearson correlations of select variables (surrounding the definition of DNAm GrimAge) in the test data from the Framingham Heart Study (N=625). DNAm GrimAge is defined as a linear combination of chronological age (Age), sex (Female takes on the value 1 for females and 0 otherwise), and eight DNAm based surrogate markers for smoking pack-years (DNAm PACKYRS), adrenomedullin levels (DNAm ADM), beta-2 microglobulin (DNAm B2M), cystatin C (DNAm Cystatin C), growth differentiation factor 15 (DNAm GDF-15), leptin (DNAm Leptin), plasminogen activation inhibitor 1 (DNAm PAI-1), issue inhibitor metalloproteinase 1 (DNAm TIMP-1). The figure also includes an estimator of mortality risk, mortality.res, which can be interpreted as a measure of "excess" mortality risk compared to the baseline risk in the test data. Formally, mortality.res is defined as the deviance residual from a Cox regression model for time-to-death due to all-cause mortality. The rows and columns of the Figure are sorted according to a hierarchical clustering tree. The shades of color (blue, white, and red) visualize correlation values from -1 to 1. Each square reports the Pearson correlation coefficient.

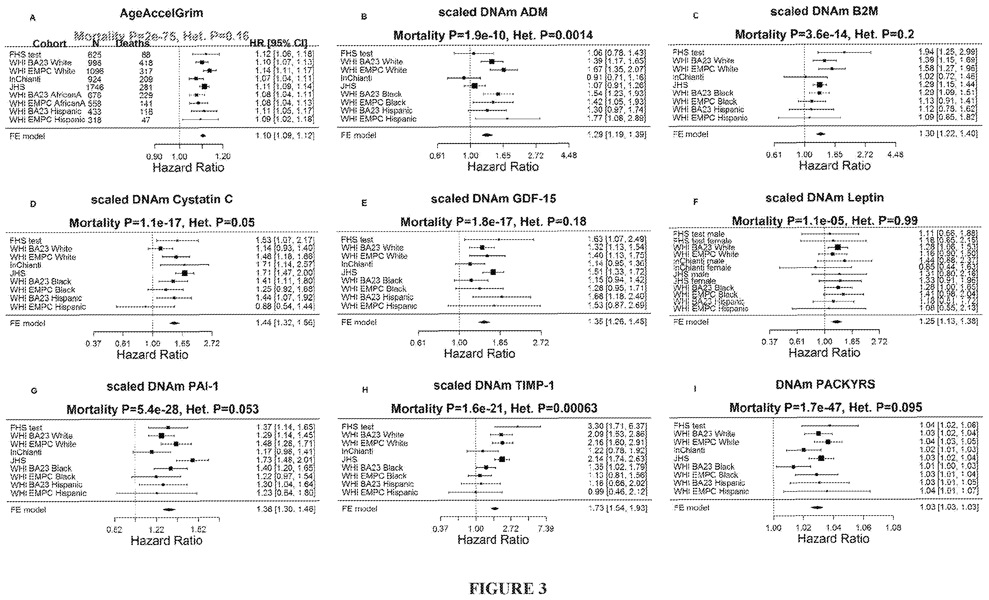

**Figures 3A-3I** show data from meta-analysis forest plots for predicting time-to-death due to all-cause mortality. Each panel reports a meta-analysis forest plot for combining hazard ratios predicting time-to-death based on a DNAm based biomarker (reported in the figure heading) across different strata formed by racial group within cohort. **Figure 3A** shows results for AgeAccelGrim. Each row reports a hazard ratio (for time-to-death) and a 95% confidence interval resulting from a Cox regression model in each of 9 strata (defined by cohort and racial groups). Results for (age-adjusted) DNAm based surrogate markers of **(Figure 3B)** adrenomedullin (ADM), **(Figure 3C)** beta-2 microglobulin (B2M), **(Figure 3D)** cystatin C (Cystatin C), **(Figure 3E)** growth differentiation factor 15 (GDF-15), **(Figure 3F)** leptin, **(Figure 3G)** plasminogen activation inhibitor 1 (PAI-1), **(Figure 3H)** tissue inhibitor metalloproteinase 1 (TIMP-1) and **(Figure 3I)** smoking pack-years (PACKYRS). The sub-title of each panel reports the meta-analysis p-value and a p-value for a test of heterogeneity Cochran Q test (Het.). In Figure 3A, each hazard ratio (HR) corresponds to a one-year increase in AgeAccelGrim. In Figures 3B-3H, each hazard ratio corresponds to an increase in one-standard deviation. In Figure 3I, hazard ratios correspond to a 1-year increase in pack-years. The most significant meta-analysis P value (here AgeAccelGrim) is marked in red. A *non*-significant Cochran Q test p-value is desirable because it indicates that the hazard ratios don't differ significantly across the strata. For example, the hazard ratios associated with AgeAccelGrim exhibit insignificant heterogeneity across the strata (Cochran Q test $P_{I2}$=0.16).

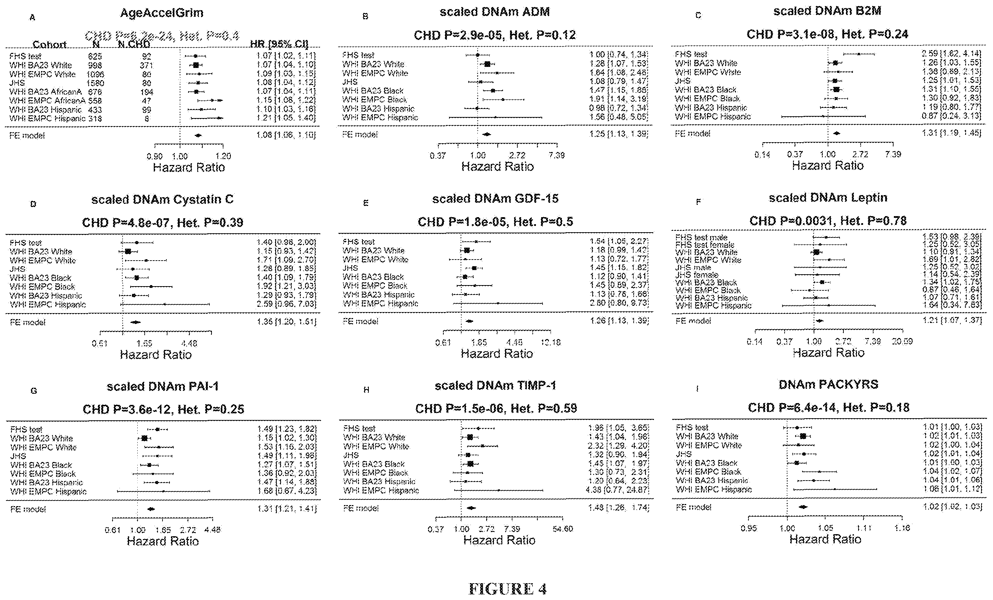

**Figures 4A-4I** show data from meta-analysis forest plots for predicting time-to-coronary heart disease. Each panel reports a meta-analysis forest plot for combining hazard ratios predicting time to CHD and the DNAm based biomarker (reported in the figure heading) across different strata formed by racial groups within cohorts. **Figure 4A** shows results for AgeAccelGrim. Each row reports a hazard ratio (for time-to-CHD) and a 95% confidence interval resulting from a Cox regression model in each of 9 strata (defined by cohort and racial groups). Results for (age adjusted) DNAm based surrogate markers of **(Figure 4B)** adrenomedullin (ADM), **(Figure 4C)** beta-2 microglobulin (B2M), **(Figure 4D)** cystatin C (Cystatin C), **(Figure 4E)** growth differentiation factor 15 (GDF-15), **(Figure 4F)** leptin, **(Figure 4G)** plasminogen activation inhibitor 1 (PAI-1), **(Figure 4H)** tissue inhibitor metalloproteinase 1 (TIMP-1) and **(Figure 4I)** smoking pack-years (PACKYRS). The sub-title of each panel reports the meta-analysis p-value and a p-value for a test of heterogeneity Cochran Q test (Het.). In Figure 4A, each hazard ratio (HR) corresponds to a one-year increase in AgeAccelGrim. In Figures 4B-4H, each hazard ratio corresponds to an increase in one-standard deviation. In Figure 4I, hazard ratios correspond to a one unit increased in DNAm pack-years. The most significant meta-analysis P value (here AgeAccelGrim) is marked.

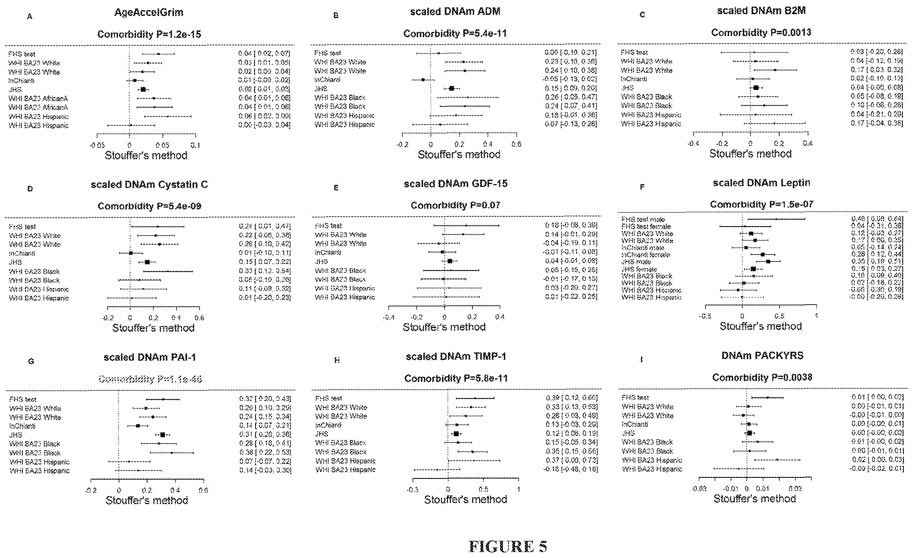

**Figures 5A-5I** show data from meta-analysis of associations with total number of age-related conditions. Each panel reports a meta-analysis forest plot for combining regression coefficients between the comorbidity index and the DNAm based biomarker (reported in the figure heading) across different strata, which are formed by racial group within cohort. **Figure 5A** shows a meta-analysis of the regression slope between AgeAccelGrim and the comorbidity index. Analogous results for (age adjusted) DNAm based surrogate markers of **(Figure 5B)** adrenomedullin (ADM), **(Figure 5C)** beta-2 microglobulin (B2M), **(Figure 5D)** cystatin C (Cystatin C), **(Figure 5E)** growth differentiation factor 15 (GDF-15), **(Figure 5F)** leptin, **(Figure 5G)** plasminogen activation inhibitor 1 (PAI-1), **(Figure 5H)** tissue inhibitor metalloproteinase 1 (TIMP-1) and **(Figure 5I)** smoking pack-years (PACKYRS). The individual study results were combined using fixed effect meta-analysis (reported in the panel heading). Cochran Q test for heterogeneity across studies (Het.). The effect sizes correspond to one year of age acceleration in panel A, one pack-year in panel I and one standard deviation in other panels for DNAm proteins. The estimate with the most significant meta P value is marked in red.

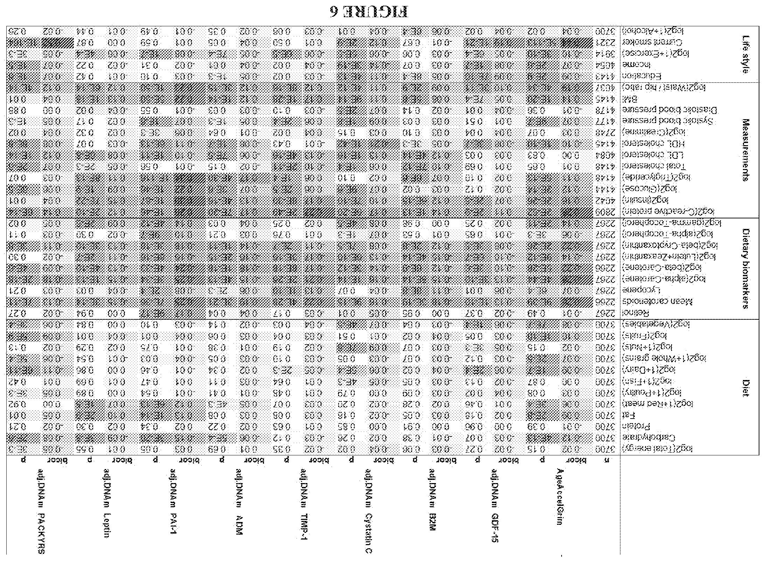

**Figure 6** provides a grid showing cross sectional correlations between DNAm biomarkers and lifestyle factors. Robust correlation coefficients (biweight midcorrelation [39]) between 1) AgeAccelGrim and its eight age-adjusted underlying DNAm based surrogate biomarkers and 2) 38 variables including self-reported diet, 9 dietary biomarkers, 12 variables related to metabolic traits and central adiposity, and 5 life style factors. The analysis was performed on the WHI cohort in up to 4200 postmenopausal women.

DETAILED DESCRIPTION OF THE INVENTION

In the description of embodiments, reference may be made to the accompanying figures which form a part hereof, and in which is shown by way of illustration a specific embodiment in which the invention may be practiced. Many of the techniques and procedures described or referenced herein are well understood and commonly employed by those skilled in the art. Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art.

All publications mentioned herein are disclose and describe aspects, methods and/or materials in connection with the cited publications. For example, Lu et al., Aging (Albany NY). 2019 Jan 21;11(2):303-327. doi: 10.18632/aging 101684; PCT Patent Application No.: PCT/US2019/034829, U.S. Patent Publication 20150259742, U.S. Patent App. No. 15/025,185, titled "METHOD TO ESTIMATE THE AGE OF TISSUES AND CELL TYPES BASED ON EPIGENETIC MARKERS", filed by Stefan Horvath; U.S. Patent App. No. 14/119,145, titled "METHOD TO ESTIMATE AGE OF INDIVIDUAL BASED ON EPIGENETIC MARKERS IN BIOLOGICAL SAMPLE", filed by Eric Villain et al.; and Hannum et al. "Genome-Wide Methylation Profiles Reveal Quantitative Views Of Human Aging Rates." Molecular Cell. 2013; 49(2):359-367 and patent US2015/0259742,.

The invention disclosed herein and recited in the claims provide novel and powerful biomarker predictors of life expectancy, mortality, and morbidity based on DNA methylation levels. Our discoveries surrounding the prediction of mortality and morbidity show that the DNAm based biomarkers disclosed herein are highly robust and informative for a range of applications. Embodiments of the DNAm based biomarkers disclosed herein can provide complementary information that enhances and supplements traditional biomarker assessments that are widely used in clinical applications. For example, embodiments of the invention can be used to directly predict/prognosticate mortality, and further information relating to a host of age-related conditions such as cardiovascular disease, cancer risk, progression in neurodegeneration, and various measures of frailty.

The invention includes methods of observing the effects of one or more test agents on genomic methylation associated epigenetic aging of human cells. Typically, these methods comprise combining the test agent(s) with human cells (e.g. for specified period of time such as at least 1-7 days, 1-3 weeks, 1-6 months or the like), and then observing methylation status in at least 172 of the methylation markers of SEQ ID NO: 1-SEQ ID NO: 172 in genomic DNA from the human cells, and then comparing the observations from (b) with observations of the methylation status in at least 172 of methylation markers of SEQ ID NO: 1-SEQ ID NO: 172 in genomic DNA from control human cells not exposed to the test agent such that effects of the test agent on genomic methylation associated epigenetic aging in the human cells is observed. Typically, the method comprises observing methylation of the genomic DNA in about 172 methylation markers from the group of methylation markers in SEQ ID NO: 1-SEQ ID NO: 172; and/or observing methylation of the genomic DNA in about 42 methylation markers from the group of methylation markers in SEQ ID NO: 173-SEQ ID NO: 214; and/or observing methylation of the genomic DNA in about 87 methylation markers from the group of methylation markers in SEQ ID NO: 215-SEQ ID NO: 301; and/or observing methylation of the genomic DNA in about 91 methylation markers from the group of methylation markers in SEQ ID NO: 302-SEQ ID NO: 392; and/or observing methylation of the genomic DNA in about 137 methylation markers from the group of methylation markers in SEQ ID NO: 393-SEQ ID NO: 529; and/or observing methylation of the genomic DNA in about 186 methylation markers from the group of methylation markers in SEQ ID NO: 530-SEQ ID NO: 715; and/or observing methylation of the genomic DNA in about 187 methylation markers from the group of methylation markers in SEQ ID NO: 716-SEQ ID NO: 902; and/or observing methylation of the genomic DNA in about 211 methylation markers from the group of methylation markers in SEQ ID NO: 903-SEQ ID NO: 1113; and/or observing methylation of the genomic DNA in about 1113 methylation markers of SEQ ID NO: 1-SEQ ID NO: 1113. Optionally in these methods, a plurality of test agents are combined with the human cells *in vitro* (e.g. primary keratinocytes from a single or, alternatively, multiple donors. In illustrative embodiments of the invention, the test agent is a polypeptide, a polynucleotide or a compound having a molecular weight less than 3,000, 2,000, 1,000 or 500 g/mol.

The invention includes a number of different biomarkers useful for predicting human lifespan, i.e. time to death, based on DNA methylation levels in blood tissue, blood cells, saliva, or buccal swabs. One biomarker embodiment, referred to as "DNAm GrimAge", can be described on two levels. First, it is based on DNA methylation measurements at 1113 locations, called cytosine-phosphate-guanines (CpGs) in the human genome. Second, at a higher level, these DNA methylation measurements at 1113 locations are further based on eight individual DNAm based biomarkers that measure i) the numbers of cigarettes smoked (more precisely smoking packyears) and ii) the plasma levels of the following seven proteins: (1) adrenomedullin (ADM); (2) beta-2-microglobulin (B2M); (3) cystatin-C; (4) growth differentiation factor 15 (GDF15); (5) leptin; (6) plasminogen activator inhibitor 1 (PAI1); and (7) tissue inhibitor metalloproteinases 1 (TIMP1).

Embodiments of the invention further include, for example, methods of obtaining information on one or more physiological factors associated with an age of an individual. These methods comprise obtaining genomic DNA from the individual, observing methylation of the genomic DNA in at least about 42 methylation markers from the group of methylation markers in SEQ ID NO: 1-SEQ ID NO: 1113, and then correlating observed methylation in the methylation markers with the one or more physiological factors associated with the age of an individual such that information on the one or more physiological factors associated with the age of an individual is obtained. Typically, the genomic DNA is obtained from human fibroblasts, keratinocytes, buccal cells, endothelial cells, lymphoblastoid cells, and/or cells obtained from blood, skin, dermis, epidermis or saliva. In typical embodiments of the invention, methylation is observed by a process comprising treatment of genomic DNA from the population of cells from the individual with bisulfite to transform unmethylated cytosines of CpG dinucleotides in the genomic DNA to uracil; and/or genomic DNA is hybridized to a complimentary polynucleotide sequence disposed on a microarray. Optionally in these embodiments, correlating observed methylation in the methylation markers comprises a regression analysis.

In this context, certain embodiments of the invention observe specific constellations of markers in SEQ ID NO: 1-SEQ ID NO: 1113. For example, in one embodiment of the invention, the one or more physiological factors associated with an age of an individual comprises predicted age or lifespan of an individual, or the time to coronary heart disease in the individual, the method comprising observing methylation of the genomic DNA in about 1113 methylation markers from the group of methylation markers in SEQ ID NO: 1-SEQ ID NO: 1113, and then correlating observed methylation in the about 1113 methylation markers with predicted age or lifespan of an individual, or the time to coronary heart disease in the individual, such that information on predicted age or lifespan of an individual, or the time to coronary heart disease in the individual is obtained. In the invention as claimed, the one or more physiological factors associated with an age of an individual comprises a number of years in which the individual has smoked in their lifetime, and the method comprises observing methylation of the genomic DNA in about 172 methylation markers from the group of methylation markers in SEQ ID NO: 1-SEQ ID NO: 172, and then correlating observed methylation in the about 172 methylation markers with the number of years in which an individual has smoked in their lifetime, such that information on the number of years in which the individual has smoked in their lifetime is obtained. Additionally, the one or more physiological factors associated with an age of an individual comprises plasma protein levels of tissue inhibitor metalloproteinase 1 in the individual, and the method comprises observing methylation of the genomic DNA in about 42 methylation markers from the group of methylation markers in SEQ ID NO: 173-SEQ ID NO: 214, and then correlating observed methylation in the about 42 methylation markers with plasma protein levels of tissue inhibitor metalloproteinase 1 in an individual, such that information on plasma protein levels of tissue inhibitor metalloproteinase 1 in an individual is obtained. Additionally, the one or more physiological factors associated with an age of an individual comprises plasma protein levels of Cystatin-C in the individual, the method comprising observing methylation of the genomic DNA in about 87 methylation markers from the group of methylation markers in SEQ ID NO: 215-SEQ ID NO: 301, and then correlating observed methylation in the about 87 methylation markers with plasma protein levels of Cystatin-C in an individual, such that information on plasma protein levels of Cystatin-C in an individual is obtained. Additionally, the one or more physiological factors associated with an age of an individual comprises plasma protein levels of Beta-2-microglobulin in the individual, the method comprising observing methylation of the genomic DNA in about 91 methylation markers from the group of methylation markers in SEQ ID NO: 302-SEQ ID NO: 392, and then correlating observed methylation in the about 91 methylation markers with plasma protein levels of Beta-2-microglobulin in an individual, such that information on plasma protein levels of Beta-2-microglobulin in an individual is obtained. Additionally, the one or more physiological factors associated with an age of an individual comprises plasma protein levels of growth differentiation factor 15 in the individual, the method comprising observing methylation of the genomic DNA in about 137 methylation markers from the group of methylation markers in SEQ ID NO: 393-SEQ ID NO: 529, and then correlating observed methylation in the about 137 methylation markers with plasma protein levels of growth differentiation factor 15 in an individual, such that information on plasma protein levels of growth differentiation factor 15 in an individual is obtained. Additionally, the one or more physiological factors associated with an age of an individual comprises plasma protein levels of adrenomedullin in the individual, the method comprising observing methylation of the genomic DNA in about 186 methylation markers from the group of methylation markers in SEQ ID NO: 530-SEQ ID NO: 715, and then correlating observed methylation in the about 186 methylation markers with plasma protein levels of adrenomedullin in an individual, such that information on plasma protein levels of adrenomedullin in an individual is obtained. Additionally, the one or more physiological factors associated with an age of an individual comprises plasma protein levels of Leptin in the individual, the method comprising observing methylation of the genomic DNA in about 187 methylation markers from the group of methylation markers in SEQ ID NO: 716-SEQ ID NO: 902, and then correlating observed methylation in the about 187 methylation markers with Leptin in an individual, such that information on plasma protein levels of Leptin in an individual is obtained. Additionally, the one or more physiological factors associated with an age of an individual comprises plasma protein levels of plasminogen activator inhibitor 1 in the individual, the method comprising observing methylation of the genomic DNA in about 211 methylation markers from the group of methylation markers in SEQ ID NO: 903-SEQ ID NO: 1113, and then correlating observed methylation in the about 211 methylation markers with plasminogen activator inhibitor 1 in an individual, such that information on plasma protein levels of plasminogen activator inhibitor 1 in an individual is obtained. In this context, the DNAm surrogate marker of PAI-1 level stands out when it comes to associations with type 2 diabetes status, glucose-, insulin-, triglyceride levels and measures of adiposity (body mass index and waist-to-hip ratio).

The DNAm GrimAge biomarker disclosed herein outperforms all considered alternative DNAm based predictors of lifespan and time-to-coronary heart disease. The two-stage construction of DNAm GrimAge (via the use of DNAm based surrogate biomarkers for plasma proteins and smoking) is admittedly involved but well worth the effort for the following reason. First, the resulting lifespan predictor outperforms all of the considered alternative biomarkers including those that were constructed by directly regressing lifespan on DNA methylation (such as our novel biomarker DNAm Mortality). Second, DNAm GrimAge outperforms other DNAm based biomarkers when it comes to predicting time to CHD. Third, DNAm GrimAge can be readily interpreted as a linear combination of age, sex, and surrogate biomarkers for plasma proteins and smoking pack-years. Fourth, the DNAm GrimAge estimate has an intuitive interpretation as physiological age since it is in units of years.

We expect that our other surrogate DNAm based biomarkers (for smoking pack-years and PAI-1 levels) will find interesting applications in their own right. We demonstrate that only a small fraction of plasma proteins (we focused on 7 out of 88) can be imputed based on DNA methylation levels. In the Framingham heart study (FHS) data, the measurement of the plasma proteins (exam 7) preceded the measurement of blood methylation data (exam 8) by 6.6 years, which suggests that long term exposure to elevated/decreased plasma levels are associated with subsequent changes in methylation levels. However, the elucidation of cause-and-effect relationships between certain plasma proteins and DNA methylation changes will require future longitudinal cohort studies and mechanistic studies. Our DNAm based surrogate biomarkers of plasma protein levels could also be interesting to epidemiologists who have access to stored DNA samples but no access to plasma samples. Strong literature support links the selected plasma proteins (used in the construction of GrimAge) to various age-related conditions: ADM levels are increased in individuals with hypertension and heart failure. Plasma B2M is a clinical biomarker associated with cardiovascular disease, kidney function, and inflammation. Plasma cystatin-C can be used to assess kidney function. Overall, ADM, B2M, cystatin C, and leptin relate to many age-related traits including cognitive functioning. GDF-15 is involved in age-related mitochondrial dysfunction. PAI-1 plays a central role in a number of age-related subclinical and clinical conditions[9], and recent genetic studies link PAI-1 to lifespan. The tissue inhibitor of metalloproteinases, TIMP-1, plays an anti-apoptotic function.

Despite their obvious strengths, it is unlikely that DNAm based biomarkers will replace existing clinical biomarkers (such as glucose levels, lipid levels, blood pressure) when it comes to informing patient care. Rather, we expected that these biomarkers will complement existing clinical biomarkers when it comes to evaluating anti-aging interventions in vivo and in vitro. Since DNAm captures important properties of the DNA molecule, these DNAm biomarkers are expected to be proximal to innate aging processes.

The DNAm GrimAge biomarker will enhance existing conventional lifespan predictors. Our DNAm based surrogate biomarker of smoking pack-years could complement self-reported assessments of pack-years. The surprising fact that DNAm pack-years outperforms self-reported pack-years when it comes to lifespan prediction could reflect erroneous self-reporting or true biology: maybe DNAm pack-years is a superior measure of long-term exposure to smoke, or of biological response to it.

Beyond lifespan prediction, AgeAccelGrim (and several of its underlying surrogate biomarkers) relate to most age-related conditions (metabolic syndrome, comorbidity, markers of inflammation such as C-reactive protein levels) in the expected way: increased values are associated with a grim condition. Similarly, higher values of AgeAccelGrim (and several DNAm based surrogate markers) are associated with a blood cell composition that is indicative of older individuals and shorter telomere length. While the reported associations are statistically highly significant, the magnitude of the underlying correlations is relatively small. In light of these data, it is unlikely that AgeAccelGrim is simply a marker of immunosenescence. Future studies will need to evaluate the hypothesis that AgeAccelGrim is associated with several hallmarks of aging including immunosenescence, the decline in hematopoietic stem cells, and cell intrinsic epigenetic changes.

While AgeAccelGrim stands out when it comes to lifespan prediction and prediction of time-to-CHD, our (age-adjusted) DNAm surrogate marker of PAI-1 level stands out when it comes to associations with type 2 diabetes status, glucose-, insulin-, triglyceride levels and measures of adiposity (body mass index and waist-to-hip ratio). Inflammation and metabolic conditions are associated with AgeAccelGrim, age-adjusted DNAm PAI-1 and age-adjusted DNAm TIMP-1. Our dietary analysis reveals that vegetable consumption is associated with slower epigenetic aging which echoes previous results. Using the invention disclosed herein, future longitudinal cohort studies can investigate the influence of various stress factors on AgeAccelGrim. Future genetic studies of AgeAccelGrim promise to identify the underlying genetic variants. Overall, our set of novel DNAm based biomarkers has great potential to monitor and evaluate interventions applied to age-related conditions.

Overview of the two-stage approach for defining DNAm GrimAge

We used a novel two-stage approach for defining a novel DNAm based predictor of life span (time to death due to all cause mortality). In stage 1, we defined a collection of surrogate DNAm biomarkers of physiological risk factors and stress factors. In stage 2, we combined these biomarkers into a single composite biomarker of lifespan (DNAm GrimAge). DNAm GrimAge is calibrated to be in units of years, i.e. it can be interpreted as DNA methylation based estimate of physiological age.

We first sought to develop DNA methylation based surrogate biomarkers of plasma protein levels since plasma proteins such as adrenomedullin, C-reactive protein, plasminogen activation inhibitor 1 (PAI-1), and growth differentiation factor 15 (GDF15) accompany important physiological processes and are associated with age-related conditions [12, 13]. Further, we defined a novel DNAm based estimator of smoking pack-years since smoking is a significant risk factor of mortality and morbidity.

The DNAm based biomarkers defined in stage 1 are attractive surrogate biomarkers for epidemiological studies because a) they lend themselves for imputing plasma protein levels and b) they allow checking self reported questionnaire entries surrounding smoking pack-years based on DNA methylation levels. The surrogate biomarkers have limitations, but it is exciting that stored DNA samples (collected decades ago) will allow one to impute plasma levels for a select group of plasma proteins.

In stage 2, we used these surrogate DNAm biomarkers to define a composite biomarker DNAm GrimAge that outperforms existing DNAm based biomarkers of lifespan. To demonstrate that DNAm GrimAge outperforms existing DNAm based predictors of lifespan we carried out a large-scale meta-analysis (involving more than 7000 Illumina array measurements). We also characterized the resulting DNAm GrimAge estimate with respect to lifestyle factors and a host of age-related conditions, e.g. we demonstrate that our DNAm based biomarkers predict time to cardiovascular disease. Finally, we demonstrate that DNAm GrimAge and several underlying surrogate biomarkers (e.g. DNAm PAI-1, DNAm GDF15) are associated with age-related changes in blood cell composition and leukocyte telomere length.

Practicing the invention of DNAm GrimAge

To use the epigenetic biomarker, one needs to extract DNA from cells or fluids, e.g. human blood cells, whole blood, peripheral blood mononuclear cells, and saliva (e.g. buccal skin cells). Next, one needs to measure DNA methylation levels in the underlying signature of 1113 CpGs (epigenetic markers) that are being used in the mathematical algorithm. The algorithm leads to an "age" (for each sample or human subject). The higher the value, the higher the risk of death and disease.

## FURTHER ILLUSTRATIVE ASPECTS AND EMBODIMENTS OF THE INVENTION

Novel molecular biomarkers of aging, such as those termed "DNAm age", "epigenetic age" or "apparent methylomic aging rate" allow one to prognosticate mortality, are interesting to gerontologists (aging researchers), epidemiologists, medical professionals, and medical underwriters for life insurances. Exclusively clinical biomarkers such as lipid levels, body mass index, blood pressures have a long and successful history in the life insurance industry. By contrast, molecular biomarkers of aging have rarely been used.

DNA methylation refers to chemical modifications of the DNA molecule. Technological platforms such as the Illumina Infinium microarray or DNA sequencing-based methods have been found to lead to highly robust and reproducible measurements of the DNA methylation levels of a person. There are more than 28 million CpG loci in the human genome. Consequently, certain loci are given unique identifiers such as those found in the Illumina CpG loci database (see, e.g. Technical Note: Epigenetics, CpG Loci Identification ILLUMINA Inc. 2010). These CG locus designation identifiers are used herein. In this context, one embodiment of the invention is a method of obtaining information useful to observe biomarkers associated with a phenotypic age of an individual by observing the methylation status of one or more of the 1113 methylation marker specific GC loci that are identified in Table 9 below.

The term "epigenetic" as used herein means relating to, being, or involving a chemical modification of the DNA molecule. Epigenetic factors include the addition or removal of a methyl group which results in changes of the DNA methylation levels.

The term "nucleic acids" as used herein may include any polymer or oligomer of pyrimidine and purine bases, preferably cytosine, thymine, and uracil, and adenine and guanine, respectively. The present invention contemplates any deoxyribonucleotide, ribonucleotide or peptide nucleic acid component, and any chemical variants thereof, such as methylated, hydroxymethylated or glucosylated forms of these bases, and the like. The polymers or oligomers may be heterogeneous or homogeneous in composition, and may be isolated from naturally-occurring sources or may be artificially or synthetically produced. In addition, the nucleic acids may be DNA or RNA, or a mixture thereof, and may exist permanently or transitionally in single-stranded or double-stranded form, including homoduplex, heteroduplex, and hybrid states.

The term "methylation marker" as used herein refers to a CpG position that is potentially methylated. Methylation typically occurs in a CpG containing nucleic acid. The CpG containing nucleic acid may be present in, e.g., in a CpG island, a CpG doublet, a promoter, an intron, or an exon of gene. For instance, in the genetic regions provided herein the potential methylation sites encompass the promoter/enhancer regions of the indicated genes. Thus, the regions can begin upstream of a gene promoter and extend downstream into the transcribed region.

The term "gene" as used herein refers to a region of genomic DNA associated with a given gene. For example, the region can be defined by a particular gene (such as protein coding sequence exons, intervening introns and associated expression control sequences) and its flanking sequence. It is, however, recognized in the art that methylation in a particular region is generally indicative of the methylation status at proximal genomic sites. Accordingly, determining a methylation status of a gene region can comprise determining a methylation status of a methylation marker within or flanking about 10 bp to 50 bp, about 50 to 100 bp, about 100 bp to 200 bp, about 200 bp to 300 bp, about 300 to 400 bp, about 400 bp to 500 bp, about 500 bp to 600 bp, about 600 to 700 bp, about 700 bp to 800 bp, about 800 to 900 bp, 900 bp to 1kb, about 1 kb to 2 kb, about 2 kb to 5 kb, or more of a named gene, or CpG position.

The phrase "selectively measuring" as used herein refers to methods wherein only a finite number of methylation marker or genes (comprising methylation markers) are measured rather than assaying essentially all potential methylation marker (or genes) in a genome. For example, in some aspects, "selectively measuring" methylation markers or genes comprising such markers can refer to measuring no more than 211, 187, 137, 91, or 42 different methylation markers or genes comprising methylation markers in Table 9.

The invention described herein provides novel and powerful predictors of life expectancy, mortality, and morbidity based on DNA methylation levels. In this context, it is critical to distinguish clinical from molecular biomarkers of aging. Clinical biomarkers such as lipid levels, blood pressure, blood cell counts have a long and successful history in clinical practice. By contrast, molecular biomarkers of aging are rarely used. However, this is likely to change due to recent breakthroughs in DNA methylation-based biomarkers of aging. Since their inception in 2013, DNA methylation (DNAm) based biomarkers of aging promise to greatly enhance biomedical research, clinical applications, patient care, and even medical underwriting when it comes to life insurance policies and other financial products. They will also be more useful for clinical trials and intervention assessment that target aging, since they are more proximal to the biological changes that characterize the aging process compared to upstream clinical read outs of health and disease status.

It is critical to distinguish clinical from molecular biomarkers of aging. Clinical biomarkers such as lipid levels, blood pressure, blood cell counts have a long and successful history in clinical practice. By contrast, molecular biomarkers of aging are rarely used. However, this is likely to change due to recent breakthroughs in DNA methylation-based biomarkers of aging. Since their inception, DNA methylation (DNAm) based biomarkers of aging promise to greatly enhance biomedical research, clinical applications, patient care, and even medical underwriting when it comes to life insurance policies and other financial products. They will also be more useful for clinical trials and intervention assessment that target aging, since they are more proximal to the biological changes that characterize the aging process compared to upstream clinical read outs of health and disease status.

The disclosure presented herein surrounding the prediction of mortality and morbidity show that DNAm based biomarkers are highly robust and informative for a range of applications. DNAm age can not only be used to directly predict/prognosticate mortality but also relate to a host of age-related conditions such as heart disease risk, cancer risk, dementia status, cardiovascular disease and various measures of frailty. Further embodiments and aspects of the invention are discussed below.

DNA methylation of the methylation markers can be measured using various approaches, which range from commercial array platforms (e.g. from IlluminaTM) to sequencing approaches of individual genes. This includes standard lab techniques or array platforms. A variety of methods for detecting methylation status or patterns have been described in, for example U.S. Pat. Nos. 6,214,556, 5,786,146, 6,017,704, 6,265,171, 6,200,756, 6,251,594, 5,912,147, 6,331,393, 6,605,432, and 6,300,071 and US Patent Application Publication Nos. 20030148327, 20030148326, 20030143606, 20030082609 and 20050009059. Other array-based methods of methylation analysis are disclosed in U.S. patent application Ser. No. 11/058,566. For a review of some methylation detection methods, see, Oakeley, E. J., Pharmacology & Therapeutics 84:389-400 (1999). Available methods include, but are not limited to: reverse-phase HPLC, thin-layer chromatography, SssI methyltransferases with incorporation of labeled methyl groups, the chloracetaldehyde reaction, differentially sensitive restriction enzymes, hydrazine or permanganate treatment (m5C is cleaved by permanganate treatment but not by hydrazine treatment), sodium bisulfite, combined bisulphate-restriction analysis, and methylation sensitive single nucleotide primer extension.

The methylation levels of a subset of the DNA methylation markers disclosed herein are assayed (e.g. using an Illumina™ DNA methylation array or using a PCR protocol involving relevant primers). To quantify the methylation level, one can follow the standard protocol described by Illumina™ to calculate the beta value of methylation, which equals the fraction of methylated cytosines in that location. The invention can also be applied to any other approach for quantifying DNA methylation at locations near the genes as disclosed herein. DNA methylation can be quantified using many currently available assays which include, for example:

a) Molecular break light assay for DNA adenine methyltransferase activity is an assay that is based on the specificity of the restriction enzyme DpnI for fully methylated (adenine methylation) GATC sites in an oligonucleotide labeled with a fluorophore and quencher. The adenine methyltransferase methylates the oligonucleotide making it a substrate for DpnI. Cutting of the oligonucleotide by DpnI gives rise to a fluorescence increase.

b) Methylation-Specific Polymerase Chain Reaction (PCR) is based on a chemical reaction of sodium bisulfite with DNA that converts unmethylated cytosines of CpG dinucleotides to uracil or UpG, followed by traditional PCR. However, methylated cytosines will not be converted in this process, and thus primers are designed to overlap the CpG site of interest, which allows one to determine methylation status as methylated or unmethylated. The beta value can be calculated as the proportion of methylation.

c) Whole genome bisulfite sequencing, also known as BS-Seq, is a genome-wide analysis of DNA methylation. It is based on the sodium bisulfite conversion of genomic DNA, which is then sequencing on a Next-Generation Sequencing (NGS) platform. The sequences obtained are then re-aligned to the reference genome to determine methylation states of CpG dinucleotides based on mismatches resulting from the conversion of unmethylated cytosines into uracil.

d) The HpaII tiny fragment Enrichment by Ligation-mediated PCR (HELP) assay is based on restriction enzymes' differential ability to recognize and cleave methylated and unmethylated CpG DNA sites.

e) Methyl Sensitive Southern Blotting is similar to the HELP assay but uses Southern blotting techniques to probe gene-specific differences in methylation using restriction digests. This technique is used to evaluate local methylation near the binding site for the probe.

f) ChIP-on-chip assay is based on the ability of commercially prepared antibodies to bind to DNA methylation-associated proteins like MeCP2.

g) Restriction landmark genomic scanning is a complicated and now rarely-used assay is based upon restriction enzymes' differential recognition of methylated and unmethylated CpG sites. This assay is similar in concept to the HELP assay.

h) Methylated DNA immunoprecipitation (MeDIP) is analogous to chromatin immunoprecipitation. Immunoprecipitation is used to isolate methylated DNA fragments for input into DNA detection methods such as DNA microarrays (MeDIP-chip) or DNA sequencing (MeDIP-seq).

i) Pyrosequencing of bisulfite treated DNA is a sequencing of an amplicon made by a normal forward primer but a biotinylated reverse primer to PCR the gene of choice. The Pyrosequencer then analyses the sample by denaturing the DNA and adding one nucleotide at a time to the mix according to a sequence given by the user. If there is a mismatch, it is recorded and the percentage of DNA for which the mismatch is present is noted. This gives the user a percentage methylation per CpG island.

In certain embodiments of the invention, the genomic DNA is hybridized to a complimentary sequence (e.g. a synthetic polynucleotide sequence) that is coupled to a matrix (e.g. one disposed within a microarray). Optionally, the genomic DNA is transformed from its natural state via amplification by a polymerase chain reaction process. For example, prior to or concurrent with hybridization to an array, the sample may be amplified by a variety of mechanisms, some of which may employ PCR. See, for example, PCR Technology: Principles and Applications for DNA Amplification (Ed. H. A. Erlich, Freeman Press, NY, N.Y., 1992); PCR Protocols: A Guide to Methods and Applications (Eds. Innis, et al., Academic Press, San Diego, Calif., 1990); Mattila et al., Nucleic Acids Res. 19, 4967 (1991); Eckert et al., PCR Methods and Applications 1, 17 (1991); PCR (Eds. McPherson et al., IRL Press, Oxford); and U.S. Pat. Nos. 4,683,202, 4,683,195, 4,800,159, 4,965,188, and 5,333,675. The sample may be amplified on the array. See, for example, U.S. Pat. No. 6,300,070.

In addition to using art accepted modeling techniques on data obtained from embodiments of the invention (e.g.

regression analyses), embodiments of the invention can utilize a variety of art accepted technical processes. For example, in certain embodiments of the invention, a bisulfite conversion process is performed so that cytosine residues in the genomic DNA are transformed to uracil, while 5-methylcytosine residues in the genomic DNA are not transformed to uracil. Kits for DNA bisulfite modification are commercially available from, for example, MethylEasyTM (Human Genetic SignaturesTM) and CpGenomeTM Modification Kit (ChemiconTM). See also, WO04096825A1, which describes bisulfite modification methods and Olek et al. Nuc. Acids Res. 24:5064-6 (1994), which discloses methods of performing bisulfite treatment and subsequent amplification. Bisulfite treatment allows the methylation status of cytosines to be detected by a variety of methods. For example, any method that may be used to detect a SNP may be used, for examples, see Syvanen, Nature Rev. Gen. 2:930-942 (2001). Methods such as single base extension (SBE) may be used or hybridization of sequence specific probes similar to allele specific hybridization methods. In another aspect the Molecular Inversion Probe (MIP) assay may be used.

The CpG sites discussed herein are found in Table 9 below. The Illumina method takes advantage of sequences flanking a CpG locus to generate a unique CpG locus cluster ID with a similar strategy as NCBI's refSNP IDs (rs#) in dbSNP (see, e.g. Technical Note: Epigenetics, CpG Loci Identification ILLUMINA Inc. 2010).

## EXAMPLE 1: ILLUSTRATIVE ASPECTS AND WORKING EMBODIMENTS OF THE INVENTION

DNAm levels have been used to build accurate composite biomarkers of chronological age [1-6]. DNAm-based age (epigenetic age) estimators, include an estimator developed by Horvath [1], based on 353 CpGs in different somatic tissues, and an estimator developed by Hannum [2], based on 71 CpGs in leukocytes. These estimators predict lifespan after adjusting for chronological age and other risk factors [7-9]. Moreover, they are associated with a host of age-related conditions [10-13]. These DNAm-based age estimators highly correlate with chronological age (r>0.9), but they are less accurate in predicting mortality, healthspan, and various age-related morbidities [10]. Several DNAm-based biomarkers are better mortality predictors because they explicitly use lifespan (time to death) in their construction [3, 4]. For example, Zhang et al (2017) combined mortality associated CpGs [4] into an overall mortality risk score. Levine et al (2018) developed a mortality predictor, DNAm PhenoAge, by regressing a phenotypic measure of mortality risk on CpGs [3].

Many analytical strategies are possible for building mortality predictors from DNAm data. The direct approach involves regressing time-to-death (due to all-cause mortality) on DNAm levels. Here we describe a two-stage procedure: In stage 1, we defined DNAm-based surrogate biomarkers of smoking pack-years and select plasma proteins which have been associated with mortality or morbidity. In stage 2, we regressed time-to-death on these DNAm based surrogate biomarkers. The mortality risk estimate of the regression model is linearly transformed so that it is in units of age. We coin this DNAm-based biomarker of mortality "DNAm GrimAge" because high values are grim news when it comes to lifespan. Using validation data from three ethnic groups, we demonstrate that DNAm GrimAge stands out in terms of its predictive ability for time-to-death due to all-cause mortality and time-to-coronary heart disease. An age-adjusted version of DNAm GrimAge, which can be regarded as a new measure of epigenetic age acceleration (AgeAccelGrim), is associated with a host of age-related conditions, lifestyle factors, biomarkers of immunosenescence, and leukocyte telomere length.

### Overview of the two-stage approach for defining DNAm GrimAge

We constructed the DNAm GrimAge in two-stages. First, we defined surrogate DNAm biomarkers of physiological risk factors and stress factors. These include the following plasma proteins: adrenomedullin, C-reactive protein, plasminogen activation inhibitor 1 (PAI-1), and growth differentiation factor 15 (GDF15) [14, 15]. In addition, given that smoking is a significant risk factor of mortality and morbidity, we also used DNAm-based estimator of smoking pack-years. Second, we combined these biomarkers into a single composite biomarker of lifespan, DNAm GrimAge, which is expressed in units of years. We then performed a large-scale meta-analysis (involving more than 7000 Illumina array measurements), showing that DNAm GrimAge is a better predictor of lifespan that present DNAm-based predictors.

Our studies reveal a surprising finding: sometimes, the DNAm based surrogate biomarkers (e.g. for smoking pack-years) is a better predictors of mortality than the underlying observed (self-reported) biomarker. We also correlated DNAm GrimAge with lifestyle factors and a host of age-related conditions, e.g. we demonstrate that our DNAm based biomarkers predict time to cardiovascular disease. Finally, we demonstrate that DNAm GrimAge is associated with age-related changes in blood cell composition and leukocyte telomere length.

### Training and test data from the Framingham Heart Study

We correlated levels of 88 plasma protein variables (measured using an immunoassay) with DNAm array data generated from the same blood samples of n=2,356 individuals from the Framingham heart study (FHS) Offspring Cohort [16] (note 1 below). We randomly split the FHS data into a training set (70% of the FHS pedigrees, N= 1731 individuals from 622 pedigrees) and a test data set (30% pedigrees, N=625 individuals from 266 pedigrees, (Table 3). The mean age of individuals donating DNA for the training set was 66 years. The mean age of individuals in the test dataset was 67. These participants had a similar demographic profile, smoking history, and number of years' follow-up as those used for the training set **(Table 3).**

**Stage 1: DNAm-based surrogate biomarkers of plasma proteins and smoking pack-years**

We used the training data to define DNAm-based surrogate markers of 88 plasma protein variables and smoking pack-years. We restricted the analysis to CpGs that are present on both the Illumina Infinium 450K array and the new Illumina EPIC methylation array in order to ensure future compatibility. Each of the 88 plasma protein variables (dependent variable) was regressed on chronological age, sex, and the CpGs levels in the training data using an elastic net regression model, which automatically selected a subset of CpGs (typically fewer than about 200 CpGs) whose linear combination best predicted the corresponding plasma level in the training data (Methods). For example, the DNAm levels of 137 CpGs and 211 CpGs allowed us to estimate the plasma levels of GDF15 and PAI-1, respectively. The predicted DNAm values of GDF15 and PAI-1 can be used as surrogate markers for the observed plasma levels. In general, we denote DNAm-based surrogate markers of plasma proteins and smoking pack-years by adding the prefix "DNAm" to the respective variable name, e.g. DNAm pack-years (Fig. 1 and Table 4).
Not all of the available 88 plasma protein could be successfully imputed based on DNAm data. Only 12 (out of 88) plasma proteins exhibited a correlation coefficient greater than 0.35 between the observed plasma levels and their respective DNAm-based surrogate marker in the test data set, (Table 1). We focused on these 12 DNAm surrogate biomarkers in stage 2. Additionally, we constructed a DNAm-based surrogate of self-reported smoking pack-years, DNAm pack-years, based on a linear combination of 172 CpGs.

**Stage 2: Constructing a composite biomarker of lifespan based on surrogate biomarkers**

In stage 2, we developed a predictor of mortality by regressing time-to-death due to all-cause mortality (dependent variable) on the following covariates: the DNAm-based estimator of smoking pack-years, chronological age at the time of the blood draw, sex, and the 12 DNAm-based surrogate biomarkers of plasma protein levels. The ElasticNet Cox regression model automatically selected the following covariates: DNAm pack-years, age, sex, and the following 7 DNAm based surrogate markers of plasma proteins: adrenomedullin (ADM), beta-2-microglobulim (B2M), cystatin C (Cystatin C), GDF-15, leptin (Leptin), PAI-1, and tissue inhibitor metalloproteinases 1 (TIMP-1), (Table 4). DNAm-based biomarkers for smoking pack-years and the 7 plasma proteins are based on typically fewer than about 200 CpGs each, totaling 1,113 unique CpGs (Table 4). Details on the plasma proteins can be found in Note 2 below.
The linear combination of covariates resulting from the ElasticNet. Cox regression model can be interpreted as an estimate of the logarithm of the hazard ratio of mortality. We linearly transformed this parameter into an age estimate, i.e., DNAm GrimAge, by performing a linear transformation whose slope and intercept terms were chosen by forcing the mean and variance of DNAm GrimAge to match that of chronological age in the training data (Methods, Fig. 1). In independent test data, DNAm GrimAge is calculated without estimating any parameter because the numeric values of all parameters were chosen in the training data. Following the terminology from previous articles on DNAm-based biomarkers of aging, we defined a novel measure of epigenetic age acceleration, AgeAccelGrim, which, by definition, is *not* correlated (r=0) with chronological age. Toward this end, we regressed DNAm GrimAge on chronological age using a linear regression model and defined AgeAccelGrim as the corresponding raw residual (i.e. the difference between the observed value of DNAm age minus its expected value). Thus, a positive (or negative) value of AgeAccelGrim indicates that the DNAm GrimAge is higher (or lower) than expected based on chronological age.
Unless indicated otherwise, we used AgeAccelGrim (rather than DNAm GrimAge) in association tests of age-related conditions because age was a confounder in these analyses. For the same reason, we also used age-adjusted versions of our DNA based surrogate markers (for smoking pack-years and the seven plasma protein levels). In general, all association tests were adjusted for chronological age and, when required, other confounders (such as sex, Methods).

Pairwise correlations between DNAm GrimAge and surrogate biomarkers

Using the test data from the FHS, we calculated pairwise correlations between DNAm GrimAge and its underlying variables (Fig. 2 and Table 4). DNAm GrimAge is highly correlated with DNAm TIMP-1 (r=0.90) and chronological age (r=0.82). An estimate of excess mortality risk (called mortality residual *mortality.res*) exhibits higher positive correlations with both DNAm GrimAge and DNAm TIMP_1 $(r \sim 0.40)$ than with chronological age $(r \sim 0.35,$ Fig. 2), in keeping with our later finding that these DNAm biomarkers are better predictors of lifespan than chronological age. With the exception of DNAm Leptin, all of the DNAm based biomarkers exhibited positive correlations with the measure of excess mortality risk $(0.41 \geq r \geq 0.16,$ Fig. 2). With the exception of DNAm Leptin, all DNAm based surrogate biomarkers exhibited moderate to strong pairwise correlations with each other.

DNAm Leptin is elevated in females consistent with what has been reported in the literature [17, 18]. After stratifying by sex, we find that plasma leptin levels increase weakly with age ($r$=0.18 and P=2.1E-3 in males; $r$=0.19, P=4.8E-4 in females).

Predicting time-to-death in validation data

To evaluate whether our novel DNAm based biomarkers are better predictors of lifespan than chronological age, we analyzed N=7,375 Illumina methylation arrays generated from blood samples of 6,935 individuals comprising 3 ethnic/racial groups: 50% European ancestry (Caucasians), 40% African Americans, and 10% Hispanic ancestry (Table 2, Methods). The data came from different cohort studies: test data from the FHS, BA23 and EMPC study from the Women's Health Initiative (WHI), the InCHIANTI cohort study, and African Americans from the Jackson Heart Study (JHS). We stratified each cohort by race/ethnicity (resulting in 9 strata) to avoid confounding and to ascertain whether the mortality predictors apply to each group separately.

The mean chronological age at the time of the blood draw was 63.0 years. The mean follow-up time (used for assessing time-to-death due to all-cause mortality) was 13.7 years. Since chronological age is one of the component variables underlying DNAmGrimAge, it is not surprising that the latter is highly correlated with age in each of the study cohorts ($r \geq 0.79$).

While each (age-adjusted) component variable underlying DNAm GrimAge is a significant predictor of lifespan (Fig. 3), DNAm pack-years (meta-analysis P=1.7F-47) and DNAm PAI-1(P=5.4E-28) exhibit the most significant meta-analysis P-values. The fixed effects meta-analysis P-values reveal that AgeAccelGrim stands out when it comes to lifespan prediction (meta-analysis P=2.0E-75, Fig. 3A). The same applies when the analysis is restricted to never-smokers or to former/current smokers. AgeAccelGrim remains a highly significant predictor of lifespan after restricting the analysis to never-smokers (N=3,988, meta-analysis P=1.1E-16) or to former/current smokers (P=3.5E-33).

DNAm based surrogates sometimes outperform observed biomarkers

Our DNAm-based surrogate biomarker for smoking pack-years has to surprising properties. First, it predicts lifespan in never-smokers (P=1.6E-6). Second, the surrogate marker is a more significant predictor of lifespan than self-reported pack-years: P=8.5E-5 for DNAm marker versus P=2.1E-3 for observed pack-years in in the FHS test data; similarly, P=5.3E-4 versus 0.18 in the InChianti Study (Table 5). The superior predictive performance of DNAm based surrogate biomarkers vis-à-vis their observed counter parts also applies to PAI-1 plasma levels (P=8.7E-4. for the DNAm marker versus P=0.074 for the observed levels), TIMP-1 (P=3.8E-4 for the DNAm marker versus P=0.017), and to a lesser extent to cystatin C (P=0.019 for the DNAm estimator versus P=0.054 for the observed level, (Table 6).

Mortality prediction based on observed plasma protein levels

The AgeAccelGrim is a composite biomarker derived from DNAm-based surrogate biomarkers of plasma protein levels and smoking pack-years. This begs the question whether a predictor of lifespan based on observed plasma protein levels and self-reported smoking pack-years, outperforms its DNAm-based analog. Analogous to our construction of DNAm GrimAge, we used a Cox regression model to regress-time to-death on the observed plasma protein levels and self-reported pack-year in the training data (Methods). The resulting mortality risk estimator (defined as weighted average of the observed biomarkers) was linearly transformed into units of years. The resulting predictor, i.e., *observed* GrimAge, and its age-adjusted version. i.e., *observed* AgeAccelGrim, were compared in the FHS, showing similar HRs *(observed* AgeAccelGrim HR=1.10, P=3.2E-7; *observed* AgeAccelGrim HR= 1.12, P=8.6E-5, Table 7). Overall, this comparison shows that DNAm levels in general and our DNAm-based surrogate biomarkers in particular capture a substantial proportion of the information captured by the 7 selected plasma proteins and self-reported smoking pack-years. Since our study focuses on DNAm based biomarkers, we will only consider DNAm based biomarkers in the following.

Age-related conditions

Our Cox regression analysis of time to coronary heart disease (CHD), reveals that AgeAccelGrim is highly predictive of incident CHD (HR=1.07, P=6.2E-24 and $P_{IS}$=0.4, Fig. 4A). As expected, several underlying DNAm-based surrogate biomarkers also predict incident CHD notably our age-adjusted versions of DNAm smoking pack-years (HR=1.02, P=6.4E-14) and DNAm PAI-1 (HR=1.31 per SD, P=3.6E-12). Similarly, time-to-congestive heart failure (CHF) is associated with AgeAccelGrim (HR=1.10 and P=4.9E-9), age adjusted DNAm cystatin C (HR=2.02 and P=2.0E-10) and DNAm PAI-1 (HR=1.58 and P=8.9E-10).

Cross sectional studies reveal that AgeAccelGrim is associated with hypertension (odds ratio [OR]=1.04 and P= 5.1E-13), type 2 diabetes (OR=1.02 and P=0.01), and physical functioning (Stouffer P=3.8E-13). All of the reported associations are in the expected directions, e.g. higher values of AgeAccelGrim are associated with lower physical functioning levels. In women, early age at menopause is associated with significantly higher values of AgeAccelGrim (P=1.6E-12) and to a lesser extent with all of the age-adjusted versions of the DNAm based surrogate markers, notably DNA cystatin C (P=2.2E-6) and DNAm GDF-15 (P=1.3E-5).

DNAm plasminogen activation inhibitor 1

AgeAccelGrimAge outperforms (age-adjusted versions of) DNAm smoking pack-years and the 7 DNAm-based surrogate markers of plasma protein levels when it comes to predicting time-to-death or time-to-coronary heart disease (Figs. 3 & 4). However, age-adjusted DNAm PAI-1 outperforms AgeAccelGrim for several age-related traits, notably the comorbidity index (defined as total number of age-related conditions) where Stouffer's meta-analysis P value for DNAm PAI-1 (P=1.1E-46) is more significant than that for AgeAccelGrim (P=1.2E-15, Fig. 5). Similar to AgeAccelGrim, higher levels of age-adjusted DNAm PAI-1 are associated with hypertension status, type 2 diabetes status, time-to-CHD (Fig. 4), time-to-CHF, and early age at menopause and lower levels are associated with disease free status (Stouffer P=2.9E-11) and better physical functioning (Stouffer P=1.2E-12).

Heritability analysis

We used pedigree based polygenic models (Methods) to arrive at heritability estimates of AgeAccelGrim and our other biomarkers. Significant heritability estimates can be observed for AgeAccelGrim ($h^2$ =0.30, P=0.022) and observed AgeAccelGrim ($h^2$ =0.37, P=0.0060 , Table 8). Similarly, several of our DNAm based surrogate biomarkers (for PAI1, B2M, ADM, GDF1 5) and their observed counterparts are highly heritable (Table 8), e.g. DNAm PAI-1 ($h^2$ =0.34 and P=7.1E-3), observed PAI-1 levels ($h^2$ =0.51, P=6.2E-4), DNAm Beta 2 microglobulin levels ($h^2$ =0.45 , P=2.4E-3), and observed B2M ($h^2$ =0.34 , P=3.3E-3). Overall, these results show that many DNAm based biomarkers are under considerable genetic control.

AgeAccelGrim versus other epigenetic measures of age acceleration

Using the same validation datasets (N=7,375 arrays), we compared DNAm GrimAge with three widely-used DNA-based biomarkers of aging: DNAm age estimator based on different somatic tissues by Horvath (2013) [1],
[0071] The DNAm age estimator based on leukocytes by Hannum (2013) [2] and the DNAm PhenoAge estimator by Levine (2018) [3]. The respective age adjusted measures of epigenetic age acceleration will be denoted as AgeAccel (or AgeAccelerationResidual), AgeAccelHannum, and AgeAccelPheno following the notation of previous publications. The four epigenetic measures of age acceleration (including AgeAccelGrim) are in units of year. AgeAccelGrim exhibits moderate positive correlations with each of the three alternative measures of epigenetic age acceleration ($0.17 \le r \le 0.45$) and the strongest correlation with AgeAccelPheno. AgeAccelGrim stands out with respect to the meta-analysis P-values for predicting time-to-death: AgeAccelGrim (P=2.0E-75, HR=1.10), AgeAccel (Meta P=8.9E-5, HR=1.02), AgeAccel-Hannum (Meta P=6.8E-16, HR=1.04), AgeAccelPheno (Meta P=3.5E-36, HR=1.05). The results remain qualitatively the same after restricting the analysis to never-smokers or former/current smokers.
Similarly, AgeAccelGrim stands out when comparing individuals in the top 20% percentile of epigenetic age acceleration to those in the bottom 20% percentile (Stouffer meta-analysis P= 2.7E-39), AgeAccelPheno (P=3.0E-20), AgeAccelHannum (P=4.9E-6), and AgeAccel (P=0.14).
When it comes to significant associations with the comorbidity index, age-adjusted DNAm PAI-1( $P_{DHAm\ PAI\text{-}1}$ =1.1E-46, Fig. 5) outperforms all other DNAm-based biomarkers including AgeAccelGrim ( $P_{AgeAccelGrim}$ =1.2E-15) and AgeAccelPheno ($P_{AgeAccelPheno}$ =2.7E-18).
AgeAccelGrim is more informative than AgeAccelPheno when it comes to predicting time to CHD ($P_{AgeAccelGrim}$ =6.2E-24 and $HR_{AgeAccelGrim}$=1.07 versus $P_{AgeAccelPheno}$ = 1.7E-8 and $HR_{AgeAccelPheno}$ =1.03) even after stratifying the analysis by smoking status.
AgeAccelGrim greatly outperforms the other 3 measures of epigenetic age acceleration including predicting time to (any) cancer (AgeAccelGrim P= 1.3E-12 versus AgeAccelPheno P=2.7E-3) and as related to an inverse association with early age at menopause in women (AgeAccelGrim P=1.6E-12 versus AgeAccel P=2.2E-3). A sensitivity analysis reveals that the latter finding remains qualitatively the same even after removing the InChianti cohort, which exhibited the strongest negative association between epigenetic age acceleration and age at menopause.

Multivariate Cox models adjusting for traditional risk factors

Our above-mentioned Cox regression models adjusted for age at blood draw (baseline), batch, pedigree, and intra subject correlation as needed. We also fit multivariate Cox regression models that included additional covariates assessed at baseline: body mass index, educational level, alcohol intake, smoking pack-years, prior history of diabetes, prior history of cancer, and hypertension status (Methods). Even after adjusting for these known risk factors for morbidity, AgeAccelGrim remained a highly significant predictor of lifespan (P=5.7E-29) and time-to-CHD (P=3.7E-11) and outperformed previously published measures of epigenetic age acceleration.

Stratified analyses

We evaluated AgeAccelGrim and underlying DNAm biomarkers in different strata characterized by age (younger/older than 65 years), body mass index (obese versus non-obese), educational attainment, prevalent condition at baseline such as prior history of cancer, type 2 diabetes, or hypertension. In all of these strata, AgeAccelGrim remains a significant predictor of time-to-death and time-to-CHD. Further, AgeAccelGrim outperforms existing DNAm based biomarkers of aging in all strata except for one (comprised of n=281 individuals with a prior history of cancer).

These subgroup analysis results also confirm that epigenetic age acceleration is an independent predictor of earlier mortality even after adjusting for possible confounders and within major subgroups of the population. With few exceptions, we found that DNAm based PAI-1, TIMP-1 and pack-years remained highly significant in each stratum.

Exceptionally fast/slow agers

The DNAm GrimAge estimate has an intuitive interpretation as physiological age since it is in units of years. However, if someone is 8 years older than expected, that does not mean that this person has on average an 8-year shorter life expectancy. Rather, lifespan calculations need to account for the hazard ratio associated with AgeAccelGim as indicated in the following. It is a statistical co-incidence that the hazard ratio associated with one-year increase in AgeAccelGrim is the same in strata comprised of never-smokers (HR=1.10), former/current smokers (HR=1.10), and among all individuals combined (HR=1.10). This allows us to evaluate the mortality risks in exceptionally fast and slow agers (according to AgeAccelGrim) irrespective of their smoking status. The top $5^{th}$ percentile and the 95% percentile of AgeAccelGrim corresponds to -7.5 years and + 8.3 years. A person in the top $95^{th}$ percentile of AgeAccelGrim (=8.3 years) faces a hazard of death that is twice that of the average person in their str,arm (whose AgeAccelGrim equals 0). Specifically, fast aging status is associated with a hazard ratio of $HR=2.2=1.10^{8.3}$. Conversely, a slow ager in the bottom $5^{th}$ percentile (-7.5 years) faces a hazard of death that is half that of the average person in their stratum, $HR=0.49= 1.10^{-7.5}$.

DNAm GrimAge versus single stage estimators of lifespan

DNAm GrimAge was built using a novel two-stage approach that critically depended on the development of DNAm-based surrogate biomarkers. To justify the utility of this indirect approach, we compared DNAm GrimAge with several DNAm-based lifespan predictors that were developed by directly regressing lifespan on DNAm data (referred to as single stage mortality predictors). To this end, we developed a new mortality predictor, DNAm Mortality (in year units) by directly regressing time-to-death (due to all-cause mortality) on CpGs in the FHS training data. DNAm Mortality was calculated as linear combination of 59 CpGs. The direct approach entailed the constructions of DNAm Mortality, an ElasticNet Cox regression model, and linear transformation of the mortality risk to ensure that the values of DNAm Mortality are in units of years (Methods). Further, we also evaluated the published mortality predictor from Zhang [4] which, remarkably, is based on only 10 CpGs (Methods). The latter two (single-stage) lifespan predictors are highly correlated with each other (r=0.77 in the FHS test data).

The novel age-adjusted DNAm Mortality estimator (HR=1.07, P=3.0E-44) and both versions of Zhang's mortality risk estimator (continuous HR=1.02, P=4.2E-39) lead to a less significant meta-analysis P-value for lifespan prediction than AgeAccelGrim (HR=1.10, P=2.0E-75). AgeAccelGrim also stands out in terms of its meta-analysis P-value for predicting time-to-CHD (AgeAccelGrim P=1.1E-20, AgeAccelMortality P=1.8E-11, AgeAccelZhang P=1.9E-10).

Because smoking is a major risk factor, it is useful to characterize the different lifespan predictors in terms of their correlation with DNAm pack-years. Age-adjusted DNAm pack-years exhibits positive correlations with both DNAm Mortality and Zhang's mortality predictor ($r \geq 0.55$). The connection of single stage mortality predictors to smoking can also be observed at the CpG level. DNAm Mortality, Zhang's mortality predictor, and DNAm pack-years explicitly use CpG cg05575921 (in the AHRR gene on chromosome 5p15.33), which has previously been highlighted by epigenome-wide association studies of cumulative smoking exposure [4, 19]. Overall, these results suggest that the two single-stage lifespan predictors relate more strongly to cumulative smoking exposure than AgeAccelGrim.

Association with blood cell composition

DNAm data allow one to estimate several abundance measures of blood cells as described in (Methods)[20, 21]. We previously showed that DNAm biomarkers of aging, which capture age-related changes in blood cell composition are better predictors of lifespan than those that are independent of blood cell counts [9]. Therefore, we hypothesized that several of our novel DNAm biomarkers would exhibit significant correlations with these imputed measures of blood cell composition. This is indeed the case as can be seen from our large-scale meta-analysis across the validation data. AgeAccelGrim is significantly associated with a decrease in naive CD8+ T cells (r=-0.22, P=9.2E-62), CD4+T cells (r=-0.21, P=1.8E-57), and B cells (r=-0.18, P=9.7E-43) and with an increase in granulocytes/neutrophils (r=0.24, P=1.5E-74) and plasma blasts (r=0.22, P=7.3E-63). While these results demonstrate that AgeAccelGrim is associated with an age-related decline in immune system functioning, our cross-sectional analysis does not allow us to dissect cause-and-effect relationships.

Age-adjusted DNAm TIMP-1 exhibits the most significant correlations with the measures of blood cell composition (e.g. proportion of granulocytes r=0.36, P=2.7E-172) followed by age adjusted DNAm Cystatin C (proportion of CD4+ T cells counts r=-0.33, P=3.4E-142). Although many of our DNAm biomarkers are correlated with blood cell counts, this does not mean that these measures only capture changes in blood cell composition. This point is illustrated by the finding that measures of blood cell composition only correlate weakly with our age-adjusted DNAm surrogate markers of smoking pack-years (strongest correlation r=-0.14) and PAI-1 levels (strongest correlation r=0.17) even though both biomarkers are strongly associated with mortality risk and age-related conditions as shown above.

Association with leucocyte telomere length

Leukocyte telomere length (LTL) has been found to be predictive of mortality and cardiovascular disease.

Our meta-analysis reveals a statistically significant but weak negative correlation between LTL and AgeAccel-Grim ($r$= -0.12 and meta P= 3.3E-10) across data from the FHS, WHI (BA23 sub-study) and JHS (total N =2,702, 27% White and 73% African American). Similarly, LTL exhibits (weak) negative correlations with our DNAm based surrogate biomarkers for GDF-15 ($r$= -0.10, meta P= 3.4E-7), DNAm PAI-1 ($r$= -0.10, meta P= 5.1E-8) and smoking pack-years ($r$= -0.09 and meta P= 2.9E-6).

Diet, education, and life style factors

Several previous measures of epigenetic age acceleration in blood have been shown to exhibit statistically significant but weak correlations with lifestyle factors and biomarkers of metabolic syndrome [3, 22]. Here we revisited these cross-sectional studies in the WHI (comprising approximately 4000 postmenopausal women, Methods) with our novel measures of AgeAccelGrim and its underlying DNAm-based surrogate biomarkers (Fig. 6).

All of our (age-adjusted) DNAm-based biomarkers correlate with plasma biomarkers measuring vegetable consumption, but AgeAccelGrim (robust correlation coefficient r=-0.26, P=9E-39, Fig. 6) and DNAm PAI-1 (r=-0.25, P=7E-36) stand out in terms of their strong relationship with mean carotenoid levels (Fig. 6). Similar, but far less significant negative associations, could be observed for self-reported measures of fruit-, vegetable-, and dairy intake. The following results were unexpected because they had not been observed with previous DNAm-based biomarkers of aging: (self-reported) proportion of carbohydrate consumption was associated with lower AgeAccelGrim (robust correlation r=-0.12, P=4E-13) and DNAm PAI-1 (r=-0.15, P=3E-20). Conversely, an increased proportion of fat intake (but not protein intake) was associated with increased AgeAccelGrim (r=0.09, P=2E-8) and DNAm PAI-1 (r=0.13, P=1E-14). Measures of lipid metabolism, triglyceride levels and HDL cholesterol levels, were significantly correlated with AgeAccelGrim (r=0.11 and r=-0.10, respectively) and even more so with (age adjusted) DNAm PAI-1 levels (r=0.3,4 and r=-0.11). Similarly, measures of glucose metabolism, insulin- and glucose levels, exhibited positive correlations with AgeAccelGrim (r=0.16 and r=0.12, respectively) and with (age adjusted) DNAm PAI-1 levels (r=0.30 and r=0.22).

Similar to what we observed with previous DNAm based biomarkers of aging, plasma C-reactive protein levels exhibited comparatively strong positive correlations with DNAm-based biomarkers, particularly AgeAccelGrim (r=0.28, P=2E-52), DNAm TIMP-1 (r=0.27, P=2E-49), and DNAm PAI-1 (r=0.26, P=1E-46).

Measures of adiposity, BMI and waist-to-hip ratio, are associated with increased AgeAccelGrim, age-adjusted DNAm PAI-1, and other DNAm based surrogate biomarkers. Higher education and income are associated with lower AgeAccelGrim (P=2E-9 and P=2E-6). AgeAccelGrim stands out when it comes to detecting a beneficial effect of physical exercise (r=-0.10, P=3E-10).

Several of our results in the WHI could be replicated in a smaller data (N< 625 individuals) from the FHS test dataset that included lipid and metabolic biomarker data. In the FHS, hemoglobin A1C and albumin levels (in urine) exhibited significant positive correlations with AgeAccelGrim, age-adjusted DNAm PAI-1 ($0.10 \leq r \leq 0.12$ and $1.4E-07 \leq P \leq 2.3E-03$), and to a lesser extent with our other DNAm based surrogate biomarkers.

DNAm-based biomarkers are strong predictors of aging [10, 23]. Several recent articles describe DNAm-based biomarkers for measuring tissue age and for predicting lifespan. This work shows that DNAm GrimAge, which in essence is as a linear combination of age, sex, and surrogate biomarkers for plasma proteins and smoking pack-years, outperforms all other DNAm-based predictors of lifespan and CHD. Moreover, the DNAm-based biomarkers of smoking and PAI-1 might be used independently of DNAm GrimAge to test a host of hypotheses linking smoking and PAI-1 to aging and its related diseases. The DNAm based surrogate biomarker of smoking might complement self-reported assessments of pack-years. The surprising fact that DNAm pack-years outperforms self-reported pack-years in predicting lifespan could reflect erroneous self-reporting. Alternatively, DNAm pack-years may capture intrinsic variation across individuals in the lasting biological damage that results from smoking, i.e., inter-individual sensitivities to smoking.

Notably, only a small fraction of plasma proteins (7 out of 88) could be imputed in this work based on DNAm. In the FHS data, the measurement of the plasma proteins (exam 7) preceded the measurement of blood DNAm data (exam 8) by 6.6 years, suggesting that DNAm is not just a snapshot of the status of these proteins at the time of blood collection. That said, the elucidation of cause-and-effect relationships between plasma proteins and DNAm will require future longitudinal cohort studies and mechanistic evaluations. Our DNAm-based surrogate biomarkers of plasma protein levels may be leveraged by researchers who rely on bio-banked DNA samples without the availability of plasma samples.

Strong evidence supports links between plasma proteins used in the construction of GrimAge and various age-related conditions: ADM levels are increased in individuals with hypertension and heart failure[24]. Plasma B2M is a clinical biomarker associated with cardiovascular disease, kidney function, and inflammation [25]. Plasma cystatin-C is used to assess kidney function [26]. Overall, ADM, B2M, cystatin C, and leptin relate to many age- related traits including cognitive functioning [27-29]. GDF-15 is involved in age-related mitochondrial dysfunction [29]. PAI-1 plays a central role in a number of age-related subclinical and clinical conditions [30], and recent genetic studies link PAI-1 to lifespan [31]. The tissue inhibitor of metalloproteinases, TIMP-1, plays an anti-apoptotic function [32].

While AgeAccelGrim stands out when it comes to lifespan prediction and prediction of time-to-CHD, our (age-adjusted), DNAm surrogate marker of PAI-1 level stands out when it comes to associations with type 2 diabetes status, glucose-, insulin-, triglyceride levels and measures of adiposity (body mass index and waist-to-hip ratio).

Inflammation and metabolic conditions are associated with AgeAccelGrim, age-adjusted DNAm PAI-1 and age-adjusted DNAm TIMP-1.

Despite their obvious strengths, DNAm-based biomarkers are unlikely to replace existing clinical indices, e.g., blood glucose, blood lipids, blood pressure, etc., in medical practice. Rather, these biomarkers might complement existing clinical biomarkers when evaluating the individual's overall 'aging' status. Since DNAm captures important properties of the DNA molecule, these DNAm biomarkers are proximal to innate aging processes [10].

Finally, beyond lifespan prediction, AgeAccelGrim (and several of its underlying surrogate biomarkers) relate to most age-related conditions (metabolic syndrome, comorbidity, markers of inflammation such as C-reactive protein levels) in the expected way, i.e., increased values are associated with deleterious effect. Similarly, higher values of AgeAccelGrim (and several DNAm-based surrogate markers) are associated with a blood cell composition that is indicative of older individuals and shorter telomere length. While the reported associations are statistically significant, the magnitude of the underlying correlations is relatively small in keeping with the concept that aging is multi-factorial and perhaps the most complex of all human traits.

## METHODS

### Study cohort

To establish DNAm based estimators and DNAm GrimAge, we used 2,356 individuals composed of 888 pedigrees from the FHS cohort [16], a large-scale longitudinal study started in 1948, initially investigating risk factors for cardiovascular disease (CVD). The FHS cohort contains medical history and measurements, immunoassays at exam 7, and blood DNA methylation profiling at exam 8. The technology of immunoassay was based on Luminex xMAP assay, an extension of the enzyme-linked immunosorbent assay (ELISA) performed with multiple analyte-specific capture antibodies bound to a set of fluorescent beads. The DNA methylation profiling was based on the Illumina Infinium HumanMethylation450K BeadChip.

We assigned 70% pedigrees (1731 individuals/622 pedigrees) to the training process and the remaining 30% of pedigrees (625 individuals/266 pedigrees) to the FHS test data (Table 3). The training dataset was used to build the DNAm based surrogate markers for plasma proteins, smoking pack-years, and the composite biomarker DNAm GrimAge.

### Validation data from 5 cohorts

Our validation analyses involved 7,375 Illumina arrays measuring blood methylation levels in N=6,935 individuals from five independent cohorts: the FHS test dataset (N=625), WHI BA23 (N=2107), WHI EMPC study (N=1972), JHS (N=1747), and InChianti (N=924 from 1 to 2 longitudinal measures on 484 individuals, Table 2). All the statistical analyses were adjusted for the correlation structure due to pedigree effects or repeated measurements as described below.

Estimation of surrogate DNAm based biomarkers

We developed estimators for plasma proteins based on blood methylation data. We leveraged immunoassay measurements in the FHS which profiled 88 plasma protein biomarkers (in units of pg/mL), including cardiovascular disease related plasma proteins such as C-reactive protein[33] and growth differentiation factor 15 (GDF-15)[34]. For each protein marker, missing values were imputed by the respective median value. The median missing rate was < 0.3%. Next the resulting observed plasma levels were regressed on DNAm data in the FHS training data.

Each plasma protein was regressed on the CpGs using the elastic net regression model implemented in the R package *glmnet*. Ten-fold cross validation was performed in the FHS training data to specify the underlying tuning parameter $\lambda$.

We required the predicted variable associated with the target variable with $\geq 0.35$ correlation in both training and test datasets. Only 12 out of 88 proteins exhibited a correlation greater than 0.35 between observed plasma levels and their respective DNAm based estimators in the FHS test data (Table 1). The missing rates of the 12 ImmunoAssay proteins were less than 0.7%. The correlation estimates have a distribution of $0.64\pm0.12$ [0.43, 0.86] (mean$\pm$SD [range]) in the training dataset and a distribution of $0.43\pm0.09$ [0.35, 0.66] in the test dataset.

### DNA methylation data

The Illumina 450K Infinium array profiled almost 486k CpG markers across the whole genome. Methylation beta values were generated using the Bioconductor *minfi* package with background correction. Our analysis focused on the subset of 450,161 CpGs that are also available on the Illumina EPIC array.

### Smoking Pack-Years

The variable "smoking pack-years" attempts to measure the cumulative amount of cigarettes consumed by the smoker. It is calculated by the number of packs of cigarettes smoked per day multiplied by the number of years the person smoked. We computed smoking pack-years using the information up to exam 8 in the FHS cohort.

### Definition of DNAm GrimAge

We again used an elastic net Cox regression model[35] to regress time-to-death (due to all-cause mortality) since exam 7 on the 12 DNAm based surrogate markers for plasma proteins and on DNAm PACKYR, chronological age, and sex. In the training dataset, we performed 10-fold cross validation to specify the value of the tuning parameter $\lambda$.

### Linearly transform DNAm GrimAge into units of years

The final ElasticNet Cox model listed in Table 4 results in an uncalibrated DNAm GrimAge estimate, which can be interpreted as the linear combination of the covariates, $X^T \beta$, or alternatively as the logarithm of the hazard ratio, log[ $h(t)/h_0(t)$] = $X^T \beta$, where $h_0(t)$ is the baseline hazard at time. The linear combination , $X^T \beta$ , can be interpreted as an uncalibrated version of DNAm GrimAge. To facilitate an intuitive interpretation as a physiological age estimator, we linearly transformed it so that the resulting estimate would be in units of years. Toward this end, we imposed the following requirement: the mean and variance of the resulting value, DNAm GrimAge, should be the same as the mean and variance of the age variable in the FHS training data (exam 7). This resulted in the following transformation

$$\text{DNAm GrimAge} = -50.28483 + 8.3268 * X^T \beta.$$

Observed DNAm GrimAge model

Our finalized DNAm GrimAge is composed of 7 DNAm proteins, DNAm pack-years, age and gender, yielding DNAm based age acceleration measure: AgeAccelGrim (in units of year). We fitted a Cox regression model based on the observed proteins, self-report pack-years, age, and gender, using the FHS training dataset. This yielded the observed value of GrimAge. We computed age acceleration measures based on the observed and DNAm based GrimAge and compared their performances as risk factors for mortality, using the FHS training and test datasets, respectively.

Statistical models used in validation analysis

Validation analysis was performed on 7,735 observations across 6,395 individuals (Table 2) coming from five datasets: the FHS test dataset (N=625), WHI BA23 (N=2107), WHI EMPC (N=1972), Jackson Heart Study (JHS, N=1747), and InChianti study (N=924 from 1 to 2 longitudinal measures on 484 individuals, Table 2). Our validation analysis involved i) Cox regression for time to death, for time-to-CHD, and for time to coronary heart failure, ii) linear regression for our DNAm based measures (independent variable) associated with and number of age-related conditions (dependent variable) and physical function score, respectively, iii) linear regression for age at menopause (independent variable) associated with our DNAm measure, iv) logistic regression analysis for estimating the odds ratios of our DNAm based measure associated with any cancer, hypertension, type 2 diabetes, emphysema, and disease free status. The variable of "number of age-related conditions" includes arthritis, cataract, cancer, CHD, CHF, emphysema, glaucoma, lipid condition, osteoporosis, type 2 diabetes, etc. In our validation analysis, we used AgeAccelGrim (the age-adjusted measure of DNAm GrimAge), and used the scaled measures of seven DNAm surrogates for plasma proteins based on the mean and standard deviation (SD) of the FHS training dataset such that the effect size was approximately corresponding to one SD. All the models were adjusted for age, and adjusted for batch effect as needed. To avoid the bias due to familial correlations from pedigrees in the FHS cohort or the intra subject correlations from the repeated measures, we accounted for the correlations accordingly in all the analyses in the following. In Cox regression analysis, we used robust standard errors, the Huber sandwich estimator, implemented in R *coxph* function. We used linear mixed models with a random intercept term, implemented in *lme* R function. We used generalized estimation equation models (GEE), implemented in R *gee* function, for our logistic regression models. Additional covariates related to demographic characteristics, psychosocial behaviors and clinical covariates were adjusted in multivariate Cox models analysis. The additional covariates include BMI (category), education attainment (category), alcohol consumption (gram/day), self-report smoking pack-years, three medical covariates: status of cancer, hypertension and type 2 diabetes at baseline. The categories associated with BMI ranges are a) 18.5 -25 (normal), b) 25 to 30 (over), and c) >30 (obese). The categories associated with education attainment are a) less than high school, b) high school degree, c) some college, and d) college degree and above. Both smoking pack-years and education variables were not available in the JHS cohort. Smoking category (never, former and current) was used in the analysis using the JHS cohort.

Meta-analysis

Unless specified, we used fixed effect models weighted by inverse variance to combine the results across validation study sets into a single estimate by using the *metafar* R function. A few statistics were combined via Stouffer's method due to different scaling of effect sizes across study sets, such as number of age-related conditions, disease free status and physical function scores.

Heritability analysis

We conducted heritability analysis using the polygenic models defined in SOLAR [36] to estimate narrow sense $h^2$. The robust polygenic model (with t-dist option) was used to estimate heritability of AgeAccelGrim and DNAm based proteins. Heritability is defined as the total proportion of phenotypic variance attributable to genetic variation in the polygenic model. All the analysis were adjusted for age (except AgeAccelGrim) and gender and were performed in R solarius[37].

Two stage estimate of mortality versus a single stage estimate of mortality

To develop our single stage mortality estimator, DNAm Mortality, we used ElasticNet Cox regression to regress time-to-death on the CpG markers, chronological age and sex in the FHS training data. We used the same options in the training process (i.e., 10-fold cross validation for choosing the lambda tuning parameter). The final estimator for DNA Mortality is a linear combination of 59 CpGs and chronological age. We used the same method for calibration as we did for DNAm GrimAge and to arrive at an estimate in units of years (DNAm Mortality). A secondary analysis was to compare with the two estimators of mortality developed by Zhang (on the basis of 10 CpGs)[4]. One estimator is a composite score of these 10 CpGs with weights determined by Cox regression with lasso penalty. Using the same method, we calibrated this estimate in units of year (arriving at DNAmZhang) in order to ensure a fair comparison with our other predictors of lifespan. The other Zhang estimator was defined as the total scores of the 10 CpGs with aberrant methylation[4], referred as DNAmZhangScore (range from 0 to 10). Of the 10 CpGs, cg06126421 and cg23665802 were absent in the JHS cohort and were replaced by the medians based on the FHS training data.

AgeAccelGrim versus blood cell composition

The imputed blood cell abundance measures were related to DNAm Grim Age models using the validation study sets: FHS test, WHI BA23, JHS, and InChianti, involving n=6,003 individuals. The following imputed blood cell counts were analyzed: B cell, naive CD4+ T, CD4+ T, naive GD8+ T, CD8+ T, exhausted cytotoxic CD8+ T cells (defined as CD8 positive CD28 negative CD45R negative), plasma blasts, natural killer cells, monocytes, and granulocytes. The blood cell composition imputation of the naive T cells, exhausted T cells, and plasma blasts was based on the Horvath method [38]. The remaining cell types were imputed using the Houseman method [21]. To avoid confounding by age, we used AgeAccelGrim and adjusted all DNAm based surrogate biomarkers by chronological age (by forming residuals). The correlation results were combined across studies via the same fixed effect models.

Diet, exercise, education, and lifestyle factors

We performed a robust correlation analysis (biweight midcorrelation, bicor [39]) between 1) AgeAccelGrim and its eight age-adjusted components and 2) 38 variables including 12 self-reported dietary variables, behavioral variables, 9 dietary biomarkers, 12 variables related to metabolic related traits and central adiposity, and 5 life style factors. We combined the individuals from the WHI BA23 and WHI EMPC, up to ~4000 postmenopausal women. The bicor analysis was conducted in the whole sample and in the three groups stratified by ethnicity. Blood biomarkers were measured from fasting plasma collected at baseline. Food groups and nutrients are inclusive, including all types and all preparation methods, e.g. folic acid includes synthetic and natural, dairy includes cheese and all types of milk. The individual variables are explained in [22].

## EXAMPLE 1 REFERENCES

1. Horvath S: DNA methylation age of human tissues and cell types. Genome Biol 2013, 14.

2. Hannum G, Guinney J, Zhao L, Zhang L, Hughes G, Sadda S, Klotzle B, Bibikova M, Fan JB, Gao Y, et al: Genome-wide methylation profiles reveal quantitative views of human aging rates. Mol Cell 2013, 49:359-367.

3. Levine ME, Lu AT, Quach A, Chen BH, Assimes TL, Bandinelli S, Hou L, Baccarelli AA, Stewart JD, Li Y, et al: An epigenetic biomarker of aging for lifespan and healthspan. Aging (Albany NY) 2018.

4. Zhang Y, Wilson R, Heiss J, Breitling LP, Saum KU, Schottker B, Holleczek B, Waldenberger M, Peters A, Brenner H: DNA methylation signatures in peripheral blood strongly predict all-cause mortality. Nat Commun 2017, 8:14617.

5. Durso DF, Bacalini MG, Sala C, Pirazzini C, Marasco E, Bonafé M, do Valle ÍF, Gentilini D, Castellani G, Faria AMC, et al: Acceleration of leukocytes' epigenetic age as an early tumor and sex-specific marker of breast and colorectal cancer. Oncotarget 2017, 8:23237-23245.

6. Lin Q, Weidner CI, Costa IG, Marioni RE, Ferreira MR, Deary IJ, Wagner W: DNA methylation levels at individual age-associated CpG sites can be indicative for life expectancy. Aging (Albany NY) 2016, 8:394-401.

7. Marioni R, Shah S, McRae A, Chen B, Colicino E, Harris S, Gibson J, Henders A, Redmond P, Cox S, et al: DNA methylation age of blood predicts all-cause mortality in later life. Genome Biol 2015, 16:25.

8. Perna L, Zhang Y, Mons U, Holleczek B, Saum KU, Brenner H: Epigenetic age acceleration predicts cancer, cardiovascular, and all-cause mortality in a German case cohort. Clin Epigenetics 2016, 8:64.

9. Chen BH, Marioni RE, Colicino E, Peters MJ, Ward-Caviness CK, Tsai PC, Roetker NS, Just AC, Demerath EW, Guan W, et al: DNA methylation-based measures of biological age: meta-analysis predicting time to death. Aging (Albany NY) 2016, 8:1844-1865.

10. Horvath S, Raj K: DNA methylation-based biomarkers and the epigenetic clock theory of ageing. Nat Rev Genet 2018, 19:371-384.

11. Zheng SC, Widschwendter M, Teschendorff AE: Epigenetic drift, epigenetic clocks and cancer risk. Epigenomics 2016, 8:705-719.

12. Jung M, Pfeifer GP: Aging and DNA methylation. BMC Biology 2015, 13:1-8.

13. Nwanaji-Enwerem JC, Weisskopf MG, Baccarelli AA: Multi-tissue DNA methylation age: Molecular relationships and perspectives for advancing biomarker utility. Ageing Res Rev 2018, 45:15-23.

14. Ignjatovic V, Lai C, Summerhayes R, Mathesius U, Tawfilis S, Perugini MA, Monagle P: Age-related differences in plasma proteins: how plasma proteins change from neonates to adults. PLoS One 2011, 6:e17213.

15. Ridker PM, Buring JE, Cook NR, Rifai N: C-reactive protein, the metabolic syndrome, and risk of incident cardiovascular events an 8-year follow-up of 14 719 initially healthy American women. Circulation 2003, 107:391-397.

16. Dawber TR, Meadors GF, Moore FE, Jr.: Epidemiological approaches to heart disease: the Framingham Study. Am J Public Health Nations Health 1951, 41:279-281.

17. Considine RV, Sinha MK, Heiman ML, Kriauciunas A, Stephens TW, Nyce MR, Ohannesian JP, Marco CC, McKee LJ, Bauer TL, et al.: Serum immunoreactive-leptin concentrations in normal-weight and obese humans. N Engl J Med 1996, 334:292-295.

18. Rosenbaum M, Nicolson M, Hirsch J, Heymsfield SB, Gallagher D, Chu F, Leibel RL: Effects of gender, body composition, and menopause on plasma concentrations of leptin. J Clin Endocrinol Metab 1996, 81:3424-3427.

19. Gao X, Jia M, Zhang Y, Breitling LP, Brenner H: DNA methylation changes of whole blood cells in response to active smoking exposure in adults: a systematic review of DNA methylation studies. Clin Epigenetics 2015, 7:113.

20. Horvath S, Gurven M, Levine ME, Trumble BC, Kaplan H, Allayee H, Ritz BR, Chen B, Lu AT, Rickabaugh TM, et al: An epigenetic clock analysis of race/ethnicity, sex, and coronary heart disease. Genome Biol 2016, 17:171.

21. Houseman E, Accomando W, Koestler D, Christensen B, Marsit C, Nelson H, Wiencke J, Kelsey K: DNA methylation arrays as surrogate measures of cell mixture distribution. BMC Bioinformatics 2012, 13:86.

22. Quach A, Levine ME, Tanaka T, Lu AT, Chen BH, Ferrucci L, Ritz B, Bandinelli S, Neuhouser ML, Beasley JM, et al: Epigenetic clock analysis of diet, exercise, education, and lifestyle factors. Aging (Albany NY) 2017.

23. Jylhava J, Pedersen NL, Hagg S: Biological Age Predictors. EBioMedicine 2017, 21:29-36.

24. Wong HK, Cheung TT, Cheung BM: Adrenomedullin and cardiovascular diseases. JRSM Cardiovasc Dis 2012, 1.

25. Liabeuf S, Lenglet A, Desjardins L, Neirynck N, Glorieux G, Lemke HD, Vanholder R, Diouf M, Choukroun G, Massy ZA, European Uremic Toxin Work G: Plasma beta-2 microglobulin is associated with cardiovascular disease in uremic patients. Kidney Int 2012, 82:1297-1303.

26. Ferguson TW, Komenda P, Tangri N: Cystatin C as a biomarker for estimating glomerular filtration rate. Curr Opin Nephrol Hypertens 2015, 24:295-300.

27. Larrayoz IM, Ferrero H, Martisova E, Gil-Bea FJ, Ramirez MJ, Martinez A: Adrenomedullin Contributes to Age-Related Memory Loss in Mice and Is Elevated in Aging Human Brains. Front Mol Neurosci 2017, 10:384.

28. Smith LK, He Y, Park JS, Bieri G, Snethlage CE, Lin K, Gontier G, Wabl R, Plambeck KE, Udeochu J, et al: beta2-microglobulin is a systemic pro-aging factor that impairs cognitive function and neurogenesis. Nat Med 2015, 21:932-937.

29. Fujita Y, Taniguchi Y, Shinkai S, Tanaka M, Ito M: Secreted growth differentiation factor 15 as a potential biomarker for mitochondrial dysfunctions in aging and age-related disorders. Geriatr Gerontol Int 2016, 16 Suppl 1:17-29.

30. Cesari M, Pahor M, Incalzi RA: Plasminogen activator inhibitor-1 (PAI-1): a key factor linking fibrinolysis and age-related subclinical and clinical conditions. Cardiovasc Ther 2010, 28:e72-91.

31. Khan SS, Shah SJ, Klyachko E, Baldridge AS, Eren M, Place AT, Aviv A, Puterman E, Lloyd-Jones DM, Heiman M, et al: A null mutation in <em>SERPINE1</em> protects against biological aging in humans. Science Advances 2017, 3.

32. Ashutosh, Chao C, Borgmann K, Brew K, Ghorpade A: Tissue inhibitor of metalloproteinases-1 protects human neurons from staurosporine and HIV-1-induced apoptosis: mechanisms and relevance to HIV-1-associated dementia. Cell Death Dis 2012, 3:e332.

33. Ridker PM: High-sensitivity C-reactive protein: potential adjunct for global risk assessment in the primary prevention of cardiovascular disease. Circulation 2001, 103:1813-1818.

34. Andersson C, Enserro D, Sullivan L, Wang TJ, Januzzi JL, Jr., Benjamin EJ, Vita JA, Hamburg NM, Larson MG, Mitchell GF, Vasan RS: Relations of circulating GDF-15, soluble ST2, and troponin-I concentrations with vascular function in the community: The Framingham Heart Study. Atherosclerosis 2016, 248:245-251.

35. Zou H, Hastie T: Regularization and variable selection via the elastic net (vol B 67, pg 301, 2005). Journal of the Royal Statistical Society Series B-Statistical Methodology 2005, 67:768-768.

36. Almasy L, Blangero J: Multipoint quantitative-trait linkage analysis in general pedigrees. Am J Hum Genet 1998, 62:1198-1211.

37. Ziyatdinov A, Brunel H, Martinez-Perez A, Buil A, Perera A, Soria JM: solarius: an R interface to SOLAR for variance component analysis in pedigrees. Bioinformatics 2016, 32:1901-1902.

38. Horvath S, Levine AJ: HIV-1 infection accelerates age according to the epigenetic clock. J Infect Dis 2015.

39. Langfelder P, Horvath S: WGCNA: an R package for weighted correlation network analysis. BMC Bioinformatics 2008, 9:559.

## EXAMPLE 2: DATASETS AND MATERIALS OF THE INVENTION

### Note 1: Description of datasets

Our study involved four cohorts: the Framingham Heart Study (FHS) offspring cohort, the Women's Health Initiative (WHI), Jackson Heart Study (JHS), and Invecchiare in Chianti, aging in the Chianti area (InChianti). We established our DNAm GrimAge model using the individuals from the FHS cohort. Of the FHS cohort, 1731 individuals were used for the training process in establishing the DNAm GrimAge model and 625 individuals were used for the test process at stage 1. Validation analysis was performed on 7,735 observations across 6,395 individuals coming from five independent datasets: the FHS test dataset (N=625), WHI BA23 (N=2107), WHI ENIPC (N=1972), JHS (N=1747), and InChianti study (N=924 from 1 to 2 longitudinal measures on 484 individuals). Below we describe each study cohort/datasets in more detail.

## Framingham Heart Study Cohort

The FHS cohort[1] is a large-scale longitudinal study started in 1948, initially investigating the common factors of characteristics that contribute to cardiovascular disease (CVD), https://www.framinghamheartstudy.org/index.php. The study at first enrolled participants living in the town of Framingham, Massachusetts, who were free of overt symptoms of CVD, heart attack or stroke at enrollment. In 1971, the study started FHS Offspring Cohort to enroll a second generation of the original participants' adult children and their spouses (n= 5124) for conducting similar examinations[2]. Participants from the FHS Offspring Cohort were eligible for our study if they attended both the seventh and eighth examination cycles and consented to having their molecular data used for study. We used the 2,356 participants from the group of Health/Medical/Biomedical (IRB, MDS) consent and available for both Immunoassay array DNA methylation array data. The FHS data are available in dbGaP (accession number: phs000363.v16.p10 and phs000724.v2.p9).

We computed the total of number age-related conditions based on dyslipidemia, hypertension, cardiovascular disease (including coronary heart disease [CHD] or congestive heart failure [CHF]), type 2 diabetes, cancer and arthritis. Time to CHD or time to CHF was truncated at zero if it occurred before exam 8. Deaths among the FHS participants that occurred prior to January 1, 2013 were ascertained using multiple strategies, including routine contact with participants for health history updates, surveillance at the local hospital and in obituaries of the local newspaper, and queries to the National Death Index. Death certificates, hospital and nursing home records prior to death, and autopsy reports were requested. When cause of death was undeterminable, the next of kin were interviewed. The date and cause of death were reviewed by an endpoint panel of 3 investigators.

*DNA methylation quantification* Peripheral blood samples were collected at the 8th examination. Genomic DNA was extracted from buffy coat using the Gentra Puregene DNA extraction kit (Qiagen) and bisulfite converted using EZ DNA Methylation kit (Zymo Research Corporation). DNA methylation quantification was conducted in two laboratory batches using the Illumina Infinium HumanMethylation450 array (Illumina). Methylation beta values were generated using the Bioconductor *minfi* package with Noob background correction[3].

## Women's Health Initiative

The WHI is a national study that enrolled postmenopausal women aged 50-79 years into the clinical trials (CT) or observational study (OS) cohorts between 1993 and 1998[4,5]. We included 4,079 WHI participants with available phenotype and DNA methylation array data: 2,107 women from "*Broad Agency Award 23*" (WHI BA23) and 1,972 women from "*Epigenetic Mechanisms of PM-Mediated CVD Risk*" (WHI EMPC). WHI BA23 focuses on identifying miRNA and genomic biomarkers of coronary heart disease (CHD), integrating the biomarkers into diagnostic and prognostic predictors of CHD and other related phenotypes, and other objectives can be found in https://www.whi.org/researchers/data/WHIStudies/StudySites/BA23/Pages/home.aspx. WHI EMPC is a study of epigenetic mechanisms underlying associations between ambient particulate matter (PM) air pollution and cardiovascular disease [6]. WHI EMPC and BA23 span three WHI sub-cohorts including GARNET, WHIMS and SHARe. 936 EMPC participants were not in any of the WHI GWAS (either GARNET, WHIMS, SHARe, MOPMAP, HIPFX, or GECCO). The largest overlap was with SHARE & GARNET. There was almost no overlap with WHIMS & MOPMAP.

The total number of age-related conditions was based on Alzheimer's disease, amyotrophic lateral sclerosis, arthritis, cancer, cataract, CVD, glaucoma, emphysema, hypertension, and osteoporosis.

*DNA methylation quanlificatiott for BA23* In brief, bisulfite conversion using the Zymo EZ DNA Methylation Kit (Zymo Research, Orange, CA, USA) as well as subsequent hybridization of the HumanMethylation450k Bead Chip (Illumina, San Diego, CA), and scanning (iScan, Illumina) were performed according to the manufacturers protocols by applying standard settings. DNA methylation levels ($\beta$ values) were determined by calculating the ratio of intensities between methylated (signal A) and un-methylated (signal B) sites. Specifically, the $\beta$ value was calculated from the intensity of the methylated (M corresponding to signal A) and un-methylated (U corresponding to signal B) sites, as the ratio of fluorescent signals $\beta = Max(M,0)/[Max(M,0)+Max(U,0)+100]$. Thus, $\beta$ values range from 0 (completely un-methylated) to 1 (completely methylated).

*DNA methylation quantification for WHI EMPC* Illumina Infinium HumanMethylation450 BeadChip data from the Northwestern University Genomics Core Facility for WHI EMPC participants sampled in stages 1a, 1b, and 2 were quality controlled, normalized and batch adjusted. Beta-mixture quantile normalization was implemented using BMIQ [7] and empirical Bayes methods of batch adjustment for stage and plate were implemented in ComBat [8].

*Lifestyle factors and dietary assessment in the Women's Health Initiative (WHI)*

WHI participants completed self-administered questionnaires at baseline which provided personal information on a wide range of topics, including sociodemographic information (age, education, race, income), and current health behaviors (recreational physical activity, tobacco and alcohol exposure, and diet). Participants also visited clinics at baseline where certified Clinical Center staff collected blood specimens and measured anthropometrics (weight, height, hip and waist circumferences) and blood pressures (systolic, diastolic). Body mass index and waist to hip ratio were calculated from these measurements.

Dietary intake was assessed at baseline using the WHI Food Frequency Questionnaire [9]. Briefly, participants were asked to report on dietary habits in the past three months, including intake, frequency, and portion sizes of foods or food groups, along with questions concerning topics such as food preparation practices and types of added fats. Nutrient intake levels were then estimated from these responses. For current drinker, we use the threshold of more than one serving equivalent (14g) within the last 28 days.

## Jackson Heart Study

The JHS is a large, population-based observational study evaluating the etiology of cardiovascular, renal, and respiratory diseases among African Americans residing in the three counties (Hinds, Madison, and Rankin) that make up the Jackson, Mississippi metropolitan area[10]. Data and biologic materials have been collected from 5306 participants, including a nested family cohort of 1,498 members of 264 families. The age at enrollment for the unrelated cohort was 35-84 years; the family cohort included related individuals >21 years old. Participants provided extensive medical and social history, had an array of physical and biochemical measurements and diagnostic procedures, and provided genomic DNA during a baseline examination (2000-2004) and two follow-up examinations (2005-2008 and 2009-2012). The study population is characterized by a high prevalence of diabetes, hypertension, obesity, and related disorders. Annual follow-up interviews and cohort surveillance are ongoing. In our analysis, we used the visits at baseline from 1747 individuals as part of project JHS ancillary study ASN0104, available with both phenotype and DNA methylation array data. Total numbers of age-related conditions were based on hypertension, type 2 diabetes, kidney dysfunction based on ever dialysis, and CVD.

*DNA methylation quantification* Peripheral blood samples were collected at the baseline. Methylation beta values were generated using the Bioconductor *minfi* package with Noob background correction[3].

## Invecchiare in Chianti, aging in the Chianti area (InChianti)

The InChianti (Invecchiare in Chianti, aging in the Chianti area) cohort is a representative population-based study of older persons enrolling individuals aged 20 years and older from two areas in the Chianti region of Tuscany, Italy, http://incluantistudy.net/wp/. One major goal of the study is to translate epidemiological research into geriatric clinical tools, ultimately advancing clinical applications in older persons. Of the cohort, 924 observations from 484 individuals with both phenotype information and DNA methylation data were including in our studies. The observations were collected from baseline in 1998 and the third follow-up visit in 2007. All participants provided written informed consent to participate in this study. The study complied with the Declaration of Helsinki. The Italian National Institute of Research and Care on Aging Institutional Review Board approved the study protocol. We computed the total number of age-related conditions based on cancer, hypertension, myocardial infarction, Parkinson's disease, stroke and type 2 diabetes.

*DNA methylation quantification.* Genomic DNA was extracted from buffy coat samples using an AutoGen Flex and quantified on a Nanodrop1000 spectrophotometer prior to bisulfite conversion. Genomic DNA was bisulfite converted using Zymo EZ-96 DNA Methylation Kit (Zymo Research Corp., Irvine, CA) as per the manufacturer's protocol. CpG methylation status of 485,577 CpG sites was determined using the Illumina Infinium HumanMethylation450 BeadChip (Illumina Inc., San Diego, CA) as per the manufacturer's protocol and as previously described[11]. Initial data analysis was performed using GenomeStudio 2011.1 (Model M Version 1.9.0, Illumina Inc.). Threshold call rate for inclusion of samples was 95%. Quality control of sample handling included comparison of clinically reported sex versus sex of the same samples determined by analysis of methylation levels of CpG sites on the X chromosome[11]. Methylation beta values were generated using the Bioconductor *minfi* package with Noob background correction[3].

## Note 2. DNAm based surrogates for plasma proteins

The model of DNAm GrimAge is composed of seven DNAm based plasma proteins, DNAm based pack years, age and gender. Below we briefly describe the seven plasma proteins. **ADM (adrenomedullin)** is a vasodilator peptide hormone. Plasma ADM, initially isolated from adrenal gland, is increased in individuals with hypertension and heart failure[12]. A recent study showed that ADM was involved in age-related memory loss in mice and aging human brains[13].

**B2M (Beta-2 microglobulin)** is a component of major histocompatibility complex class 1 (MHC I) molecular. Plasma B2M is a clinical biomarker associated with cardiovascular disease, kidney function, inflammation severity[14]. B2M is a pro-aging factor associated with cognitive and regenerative function in aging process and suggests B2M may be targeted therapeutically in old age [15]. A previous study showed that systemic B2M accumulation in aging blood promoted age-related cognitive dysfunction and impairs mouse models[15].

**Cystatin C or cystatin 3** (formerly gamma trace, post-gamma-globulin, or neuroendocrine basic polypeptide) is mainly used as a biomarker of kidney function. Plasma cystatin-C is a clinical relevant biomarker indicating kidney function[16]. Cystatin-C seems plays a role in cardiovascular disease [17] or amyloid deposition associated with Alzheimer's disease [18].

**GDF-15 (growth differentiation factor 15)** is one of transforming growth factor beta subfamily. GDF-15 has been implicated in aging and age- related disorders. It also plays a role in age-related mitochondria dysfunction[19].

**Leptin** is a hormone predominantly in adipose cells. Leptin plays a role in regulating energy balance by inhibiting hunger and is implicated in Alzheimer's disease[20].

**Plasminogen activator inhibitor antigen type 1(PAI-1)** is the major inhibitor of tissue-type plasminogen activator and unokinase plasminogen activator. PAI-1, released in response to inflammation process, plays a central role in a number of age-related subclinical (i.e., inflammation, atherosclerosis, insulin resistance) and clinical conditions (i.e., obesity, comorbidities)[21].

**TIMP-1 or TIMP metallopeptidase inhibitor 1** is a tissue inhibitor of metalloproteinases. It is also involves chromatin structures, promoting cell proliferation in a wide range of cell types, and may also have an anti-apoptotic function[22].

<u>Estimation of blood cell counts based on DNAm levels</u>

We estimated blood cell counts using two different software tools. First, Houseman's estimation method [23] was used to estimate the proportions of CD8+ T cells, CD4+ T, natural killer, B cells, and granulocytes (mainly neutrophils). Second, the Horvath blood cell estimation method, implemented in the advanced analysis option of the epigenetic clock software [24,25], was used to estimate the percentage of exhausted CD8+ T cells (defined as CD28-CD45RA-), the number (count) of naive CD8+ T cells (defined as CD45RA+CCR7+) and plasma blasts cells. We and others have shown that the estimated blood cell counts have moderately high correlations with corresponding flow cytometric measures [23,26].

**TABLES**

Table 1: Reproducibility and age correlations of DNAm based surrogate biomarkers

| Correlation ( r ) | Training (N=1731) | | Test (N=625) | |
|---|---|---|---|---|
| | Observed biomarker | Age | Observed biomarker | Age |
| **DNAm based surrogate** | | | | |
| adrenomedullin | 0.65 | 0.63 | 0.38 | 0.64 |
| beta-2-microglobulin | 0.62 | 0.83 | 0.43 | 0.85 |
| CD56 | 0.86 | 0.17 | 0.36 | 0.17 |
| ceruloplasmin | 0.56 | 0.04 | 0.49 | -0.02 |
| cystatin-C | 0.58 | 0.81 | 0.39 | 0.83 |
| EGF fibulin-like ECM protein1 | 0.59 | 0.72 | 0.41 | 0.87 |
| growth differentiation factor 15 | 0.74 | 0.71 | 0.53 | 0.81 |
| leptin | 0.68 | 0.06 | 0.35 | 0.05 |
| myoglobin | 0.50 | -0.04 | 0.38 | 0.03 |
| plasminogen activator inhibitor 1 | 0.69 | 0.19 | 0.36 | 0.16 |
| serum paraoxonase/arylesterase 1 | 0.57 | -0.22 | 0.51 | -0.22 |
| tissue Inhibitor Metalloproteinases 1 | 0.43 | 0.92 | 0.35 | 0.90 |
| smoking pack-years | 0.79 | 0.17 | 0.66 | 0.13 |

**Legend**

This table reports the correlation coefficients between the observed marker (i.e. observed plasma protein level or self-reported smoking pack-years) and its respective DNAm based surrogate marker in 1) the FHS training data and 2) the FHS test data. Each of the DNA based surrogate biomarkers (rows) leads to a correlation $r \geq 0.35$ in both training and test datasets (columns 2 and 4). DNAm based pack-years is highly correlated with the self-report pack-years in both training and test datasets ($r \geq 0.66$). The table also reports the correlation coefficients between the DNAm based surrogate biomarkers (rows) and chronological age in the FHS training and test data (columns 3 and 5).

Table 2: Overview of the cohorts used in the validation analysis

| Study | N | Female | Age | Smoking status | | | Pack-years | Years of Follow-up |
| | | | | Never | Former | Current | | |
|---|---|---|---|---|---|---|---|---|
| FHS* test | 625 | 53% | 66.9±8.64 [61,73] | 37% | 52% | 10% | 14.7±19.91 [0,23] | 7.7±1.78 [7.3,8.8] |
| WHI BA23 | 2107 | 100% | 65.3±7.1 [60,70.9] | 52% | 36% | 10% | 9.5±18.55 [0,12.5] | 16.9±4.63 [15.8,19.9] |
| WHI EMPC | 1972 | 100% | 63.3±7.03 [57.9,68.7] | 52% | 38% | 9% | 9±17.27 [0,12.5] | 18±4.02 [17.9,20.1] |
| JHS | 1747 | 63% | 56.2±12.31 [46.5,65.4] | 65% | 21% | 14% | NA | 11.7±2.55 [11.2,13.1] |
| InChianti** | 924 (484) | 54% | 67±16.64 [60,78] | 57% | 29% | 14% | 10.3±17.33 [0,16.8] | 5.4±4.84 [0.1,9.3] |

NA=not available.

Quantitative variables are presented in the format of mean ±SD [25th, 75th].

*The distribution of age is based on exam 8.

**The statistics are based on the number of 924 observations across 484 individuals.

**Legend**

This table summarizes the characteristics of 6,935 individuals (corresponding to 7,375 Illumina arrays) from five independent cohorts that were used in our validation analysis. For example, up to two longitudinal measurements were available for each of 484 individuals in the InChianti cohort.

Table 3 Characteristics of the Framingham Heart Study

Description of the Framingham Heart Study (FHS) Offspring Cohort that was used to train and test DNAm GrimAge. We assigned 70% of pedigrees to the training dataset and the remaining 30% pedigrees to the test dataset.

| | Training | Test |
| | N=1731 | N=625 |
|---|---|---|
| $N_{pedigree}$ | 622 | 266 |
| Female | 54% | 53% |
| Smoking | | |
| Never | 41% | 37% |
| Former | 51% | 52% |
| Current | 8% | 10% |
| Age | | |
| exam 7 | 59.6±9.05 [53;67] | 60.3±8.59 [54;66] |
| exam 8 | 66.2±9.08 [59;73] | 66.9±8.64 [61;73] |
| PACK years | 13.8±20.17 [0;22] | 14.7±19.91 [0;23] |
| Follow-up (years) | | |
| since exam 7 | 14.5±1.69 [14;15.6] | 14.4±1.82 [13.9;15.6] |
| since exam 8 | 7.9±1.65 [7.5;8.9] | 7.7±1.78 [7.3;8.8] |

Quantitative variables are presented in the format of mean $\pm$ one standard deviation, SD and the interval: [25th, 75th] percentile.

Immunoassay array was profiled at exam 7;
DNA methylation array was profiled at exam 8.

Table 4: Multivariate regression model for estimating DNA GrimAge

Coefficient values for computing the uncalibrated version of DNAm GrimAge based on the underlying covariates (rows): chronological age, sex (an indicator of female), and eight DNA methylation (DNAm) based variables (rows). The columns report the name of the covariate (e.g. DNAm based biomarker), its abbreviation, regression coefficient, and the number of underlying CpGs that underlie the surrogate biomarker (defined in stage 1). As noted, this model yields uncalibrated DNAm GrimAge. The finalized DNAm GrimAge is based on transforming the raw variable into a distribution in units of year (see **Methods**).

| Covariate | Abbreviation | Coefficients | Number of CpGs |
|---|---|---|---|
| DNAm adrenomedullin | DNAm ADM | 0.007903 | 186 |
| DNAm beta-2-microglobulin | DNAm B2M | 4.59E-7 | 91 |
| DNAm cystatin-C | DNAm Cystatin C | 3.5E-6 | 87 |
| DNAm growth differentiation factor 15 | DNAm GDF-15 | 0.000349 | 137 |
| DNAm leptin | DNAm Leptin | -7.3E-6 | 187 |
| DNAm plasminogen activator inhibitor 1 | DNAm PAI-1 | 2.56E-5 | 211 |
| DNAm tissue inhibitor metalloproteinases 1 | DNAm TIMP-1 | 0.000144 | 42 |
| DNAm pack years | DNAm PACKYRS | 0.030398 | 172 |
| Chronological age | Age | 0.030082 | -- |
| Female | Female | -0.22847 | -- |

Table 5: Comparing self-reported versus DNAm based estimates of smoking pack years predicting time-to-death.

Two Cox regression models for time-to-death (due to all-cause mortality) were used to evaluate the predictive power of self-reported smoking pack years and its DNAm based surrogate biomarker, respectively. The survival analysis was conducted across five datasets from four independent study cohorts (as detailed in the first column). The column report the variables, the sample size, the total number of deaths during the follow-up period, the hazard ratio associated with a 1 unit increase in the variable, and the corresponding Cox regression p-value. To compare the performance of the two variables, one should focus on the p-value as opposed to the hazard ratio (because the latter depends on the distribution of the underlying variable). Surprisingly, this comparative analysis indicates that the DNAm based surrogate biomarker of smoking pack years leads to a more significant (smaller) Cox regression p-value than that of the self-reported variable.

| Data | Pack years | N | # death | HR | P |
|---|---|---|---|---|---|
| FHS train | DNAm surrogate | 1729 | 219 | 1.04 | 8.10E-23 |
| | Self-reported | 1729 | 219 | 1.02 | 1.12E-11 |
| FHS test | DNAm surrogate | 625 | 88 | 1.04 | 8.51E-5 |
| | Self-reported | 625 | 88 | 1.01 | 2.13E-3 |
| WHI BA23 | DNAm surrogate | 2107 | 765 | 1.02 | 7.17E-12 |
| | Self-reported | 2029 | 734 | 1.01 | 7.03E-12 |
| WHI EMPC | DNAm surrogate | 1972 | 505 | 1.03 | 2.1E-19 |
| | Self-reported | 1904 | 484 | 1.02 | 6.4E-19 |

(continued)

| Data | Pack years | N | # death | HR | P |
|---|---|---|---|---|---|
| InChianti | DNAm surrogate | 924 | 209 | 1.02 | 5.33E-4 |
| | Self-reported | 924 | 209 | 1.01 | 1.78E-1 |
| HR=hazard ratio | | | | | |

Table 6: Comparing ImmunoAssay versus DNAm based estimates of plasma proteins wrt. predicting time-to-death.

Two Cox regression models for time-to-death (due to all-cause mortality) were used to evaluate the predictive power of our study plasma proteins measured by ImmunoAssay versus its DNAm based surrogate biomarker, respectively. The survival analysis was adjusted for chronological age, center, and family structure, conducted in the FHS training (N/number of death =1729/219) and test (N/number of death=625/88) datasets, respectively. For each plasma protein, we list the hazard ratios (HR) and the corresponding Cox regression p-values of the protein levels based on ImmunoAssay measure and DNA methylation estimate, respectively. All the test variables were standardized based on the FHS training dataset such that each HR corresponds to an increase in one-standard deviation. To compare the performance of the two variables, one should focus on the p-value as opposed to the hazard ratio (because the latter depends on the distribution of the underlying variable).

The analysis for leptin levels was stratified by gender due to its highly correlation with gender.

| Protein | data | HR* | | P-value | |
|---|---|---|---|---|---|
| | | ImmunoAssay | DNAm | ImmunoAssay | DNAm |
| Adrenomedullin | training | 1.23 | 1.36 | 4.53E-4 | **3.85E-7** |
| | test | 1.33 | 1.06 | 2.92E-2 | 7.23E-1 |
| Beta-2 Microglobulin | training | 1.31 | 1.51 | 7.42E-8 | 9.87E-8 |
| | test | 1.22 | 1.94 | 1.33E-2 | **2.92E-3** |
| Cystatin C | training | 1.25 | 1.53 | 1.32E-4 | **1.44E-8** |
| | test | 1.13 | 1.53 | 5.40E-2 | **1.92E-2** |
| GDF-15 | training | 1.24 | 1.20 | 3.82E-5 | 2.58E-3 |
| | test | 1.83 | 1.63 | 6.86E-13 | 2.32E-2 |
| Leptin | training male | 1.28 | 2.23 | 1.03E-1 | **9.12E-6** |
| | test male | 0.99 | 1.11 | 9.74E-1 | 6.83E-1 |
| | training female | 0.93 | 0.97 | 4.81E-1 | 8.38E-1 |
| | test female | 1.15 | 1.18 | 3.25E-3 | 5.91E-1 |
| PAI-1 | training | 1.14 | 1.31 | 3.95E-2 | **3.63E-6** |
| | test | 1.19 | 1.37 | 7.42E-2 | **8.69E-4** |
| TIMP-1 | training | 1.33 | 2.61 | 1.38E-5 | **1.06E-8** |
| | test | 1.29 | 3.30 | 1.71E-2 | **3.79E-4** |

*in units of one SD.
The p value based on a DNAm variable was marked in bold if it was more significant than the one based on the corresponding ImmunoAssay variable.
GDF-15=growth differentiation factor 15; PAI-1=plasminogen activation inhibitor 1; TIMP-1=tissue inhibitor metalloproteinase 1.

Table 7: Mortality prediction in the FHS based on DNAm- and observed versions of AgeAccelGrim

The fact that AgeAccelGrim is a composite biomarker based on DNAm based surrogate biomarkers of plasma protein levels and smoking pack-years begs the question whether a predictor of lifespan based on observed values, i.e.

observed plasma protein levels and self-reported smoking pack-years, outperforms its DNAm based analog? To our surprise, relatively little is gained by using observed values as will be shown in the following. Analogous to our construction of DNAm GrimAge, we used a Cox regression model to regress-time to-death on the observed plasma protein levels and self-reported pack-year in the training data (Methods). As before, the resulting mortality risk estimator (defined as linear combination of the observed biomarkers) was linearly transformed into units of years. The resulting predictor will be denoted as observed GrimAge and its age-adjusted version as observed AgeAccelGrim. The table reports the predictive accuracy for the two age acceleration measures (based on observed values versus DNAm based surrogates) when it comes to predicting time to death (due to all-cause mortality). The table reports the sample size (N), the number of deaths during the follow up period, the hazard ratio associated with a 1 unit increase in the variable, the Z statistic, and the Cox regression p value. To compare the performance of the two variables, one should focus on the p-value as opposed to the hazard ratio (because the latter depends on the distribution of the underlying variable).

Rows correspond to the results in the training data and the test data of the FHS. An unbiased comparison in the test from the FHS reveals that the hazard ratio associated with observed AgeAccelGrim (HR=1.10, P=3.2E-7) is similar to that of its DNAm based analog AgeAccelGrim (HR= 1.12, P=8.6E-5).

The results in the training data of the FHS are biased and should be ignored.

| FHS | AgeAccelGrim | N | # death | HR | z | P |
|---|---|---|---|---|---|---|
| Training (N=1731) | Observed | 1729 | 219 | 1.12 | 10.54 | 5.65E-26 |
| | DNAm based | 1729 | 219 | 1.14 | 11.85 | 2.15E-32 |
| Test (N=625) | Observed | 625 | 88 | 1.10 | 5.11 | 3.18E-7 |
| | DNAm based | 625 | 88 | 1.12 | 3.93 | 8.55E-5 |
| HR=hazard ratio. | | | | | | |

Table 8: Heritability analysis of observed and DNAm based variables

Robust polygenic model was performed to estimate heritability using the FHS test pedigree datasets. We estimated the heritability of observed and DNAm based AgeAccelGrim and it associated proteins. The observed AgeAccelGrim was defined in manuscript and the observed protein levels were based on ImmunoAssay measures. Additional analysis was applied to other epigenetic measures of age acceleration: age-adjusted DNAm PhenoAge (AgeAccelPheno)[28], age-adjusted DNAm age based on Hannum et al. (AgeAccelHannum) [27] and age adjusted DNAm age based on Horvath (AgeAccelResidual)[24].

| | Heritability | | P value | |
|---|---|---|---|---|
| Trait | ImmunoAssay | DNAm | ImmunoAssay | DNAm |
| AgeAccelGrim | 0.37 | 0.30 | 6.0E-3 | 2.2E-2 |
| AgeAccelPheno | NA | 0.11 | NA | 2.5E-1 |
| AgeAccelHannum | NA | 0.19 | NA | 7.7E-2 |
| AgeAccelerationResidual | NA | 0.44 | NA | 1.8E-3 |
| Adrenomedullin | 0.42 | 0.38 | 3.7E-3 | 2.9E-3 |
| Beta 2 microglobulin | 0.34 | 0.45 | 3.3E-3 | 2.4E-3 |
| Cystatin C | 0.32 | 0.09 | 2.9E-2 | 3.1E-1 |
| GDF-15 | 0.24 | 0.21 | 6.0E-2 | 9.1E-2 |
| Leptin male | 0.58 | 0.31 | 4.8E-2 | 1.8E-1 |
| Leptin females | 0.34 | 0.38 | 1.1E-1 | 1.3E-1 |
| PAI-1 | 0.34 | 0.51 | 7.1E-3 | 6.2E-4 |
| TIMP-1 | 0.31 | 0.24 | 2.0E-2 | 9.7E-2 |
| GDF-15=growth differentiation factor 15; PAI-1=plasminogen activation inhibitor 1; TIMP-1=tissue inhibitor metalloproteinase 1;NA= not applicable. | | | | |

**Table 9: CpG Methylation Sites Associated with one or more physiological factors associated with an age of an individual**

| cg identifier | DNA Sequence | Sequence ID Number |
|---|---|---|
| cg20462449 | CGGACCGTTTGGCCCCTCTGTGACCCAAGTCAGCCTCGGCGAAGTGCTTT | 1 |
| cg19802390 | CGCGGCCAGGCAGCGAACCCCACTAGGAGGCACCCCAGGCCCCTGGGCTT | 2 |
| cg14084907 | CGCGCCTGGGCCCCCAGGACCTCTTGCAGACGGAGTCCCTCAGACAGTG | 3 |
| cg23251761 | GGTTGCTTCCTTTTGTGCCCCCGGGGGTCAGGAATCCCAGGTTTCTCCG | 4 |
| cg14825555 | CTGAGGCCACCTGGGGCTGGGGATCCCACTCTTCTTGCAGCTGTTGAGCG | 5 |
| cg04967775 | AGGGCCCCCTGGGCCTGTACCAAGGTCTGCGACAGATGGGTGGGGAGCCG | 6 |
| cg05817517 | GCCTGGCAGAATCAGGAGCCGGGGGAGTAGGGCTCCCGGAAAGTTTATCG | 7 |
| cg00500789 | GAGGCAGTGGGCGGGGGCTGAAGCTGCAGGTCTGCGCAGAGGGCGGGGCTGCG | 8 |
| cg23800435 | GTGGGCGGGGGCTGAAGCTGCAGGTCTGGCAGAGGGCGGGGCTGCGGCAGCG | 9 |
| cg08871545 | CGCAAGCATGAAGCGTTTAACTGTGCAACACAAATCAACAACCTGGGCTC | 10 |
| cg04135242 | CGCACCCACTGAATCAGAGAAGCTGTAGGAGCGGGAGCCAGTGGTTTGCATT | 11 |
| cg25189904 | CGCTGCCAAACCCCTACACGAAAGTTTGGTTCTGTTCCAGAAACTATGGA | 12 |
| cg16511983 | GGACAAAAAATCCTCGGTGGCGGGGCAAAAAGTCGCGGTGACAAAAAGCG | 13 |
| cg12067764 | TTTGAAGCCCTTATGTCTATGGTAATCTGTTACAGCCCCCGCAGGAAACG | 14 |
| cg09396704 | CGGGCACCTTGCTGTCGCCCTGCGCCCCACCTGCCCTTGGCTGCC | 15 |
| cg17440248 | CGTGAATTGAGATGTCATGCCATTGTAAGATTTTAAAAAGGGAAGGACA | 16 |
| cg01491219 | CGGGCGCGCACTGCACAATGAACATGCAAAACACCTTCAACTGCACTTGCT | 17 |
| cg02356786 | CCATAGCGGTGGGCCTTTGGCGACGTCAGAGGCGCGGGTGTTCGCCTACG | 18 |
| cg19012696 | AGCTCCTGCCGTGGTGGTGAAAATATTGTGGACTGCTTGGCTGACTACACG | 19 |
| cg17886420 | CGTGCTCAGCTGATAAAATGCTAAATAGCATCAATGCTGACCATGCTTCT | 20 |
| cg12465010 | CGTGTGTACACATGTATGTCTTTCAAGCAAGGCTATGTATTACTCAGAAC | 21 |
| cg15777335 | GTCAGGGAGATAAGTCCCTTTGCGGAATGTCAAGACGGTGAGCAGGCGCG | 22 |
| cg03634479 | CGCTTCTTAAGGGAAAGGCATGAGGTGGGACAGGCAAACCTTGGCAAGTG | 23 |
| cg13458005 | CGGGCAGGCATCCCCTCTACCTGACCGGCTTCTTCGGAGGATACATCTTGG | 24 |
| cg14371731 | ATTAGCGGAGCGCCTCCGCCTATGATTGGCTTCGCCCGGGAAGCTGGAGACG | 25 |

| cg identifier | DNA Sequence | Sequence ID Number |
|---|---|---|
| cg23322172 | CGGGGGCTCTTCCTGCTCAAGCTGGGACTTAGCCTGCTCATGTTGTTGGC | 26 |
| cg02089135 | CTTTTTTTGGCATTGGAATTGGCAAGTGGCTGCCCTTGTCTGTTACAACG | 27 |
| cg05747812 | CGGGGTGCAGGTCAGGGCTCCAGGTGTCACCTGCCTGTCCACACGGGGCT | 28 |
| cg11019008 | CGCCGTGTCCCTCACGCTGGCTTTCAGAAGGCTTCACGCAAGTGGGCTCA | 29 |
| cg02580250 | CGGGTGCTGTTATCACCCCAATTTCACCAATGAGGAAAGAGGTGCAGAGA | 30 |
| cg24869272 | GTGGTTTTCTACCTGGACCTCTCCTTCATCTTCCTCCTAGAACTGAAGCG | 31 |
| cg23938637 | GCTGTGGCGTCTCCAACTACACTGACTGGTTCGAGGTGTACAACGCCACG | 32 |
| cg09690118 | CGTGGACTTCCCATCCCCTGGACCCCACGGCGGGGACAAGGAAACCTACA | 33 |
| cg01518025 | AGCCTCACTGAGGAAGTGACTGTCCCAGGCTCTAGAGGTGCCTGAGGGCG | 34 |
| cg06809342 | AGACGGGGAGGCCGGGCAGAGACGCTCCTCACCTCCCAGACAGGGTTGCG | 35 |
| cg00161556 | CGACCCTTTGTGGAGGGCACTGGAGGAGCTGGCATTTAAGCCCTGTCCAG | 36 |
| cg06911753 | AATGCGTCGCAGTCAAGACAGCGCAGGGGCCTGGGCTGCCGGGCGCTGCG | 37 |
| cg04265051 | CAAGGGCACACAGCGAGGCAGTTTCAGGGCGGGCAGCCTGGGGCCCCACG | 38 |
| cg16385335 | CGGGGCAGTGCTTTCCCAGAAGGATTGCTCGGCATCCTGCCCTTCCCAGA | 39 |
| cg11660018 | CGCACAGCACTGAGTGCTGTAAGGAAATGCAAAGCATTGGTGGCCCTGCC | 40 |
| cg23771366 | CGGTGCCTCCAAAGAACAGAAACCTGTATTCCCATGGCAGGGCCACCAAT | 41 |
| cg12051762 | TCTCAAACTCGGGGCCAGAGAACAGTGAAGTAGGAGCAGCCGTAAGTCCG | 42 |
| cg10130564 | CGCCCCGGCCCGCTCCATCTCCAAAGCATGCAGAGAATGTCTCGGCAGCC | 43 |
| cg19174059 | GCACACACAGCAGCAGCACTGCCACCCAGAGCAGGGCTAAGATGAACACG | 44 |
| cg04832325 | CGGGTATTACTGCCATCGTCGTACCAGCAGGGAAACAAACTGAGACTCCG | 45 |
| cg08161922 | CGCGGCTCCATACTATTGCCCAGTGTTTTCCGAGCATCTCCATCCTTGGC | 46 |
| cg00417288 | CAGTCCGTCATCTCATCTGGGGAAACTGTTTGCCTTTTTCTGATACTACG | 47 |
| cg21368094 | CGTGTGCACATGCGCACTCTCATATGCCACCCCATGTGCCTTTAGTCCAC | 48 |
| cg05661164 | CAGGAGATGCCGCGGTAGGGGGCGGCGGTGATGCAGCAGCGGCTTGGCCG | 49 |
| cg09244872 | CGGGTGCCCAGGAGCCCACAAAACGGCCAGAGGAGAAATGCTTTCAAAGG | 50 |
| cg22277972 | CGGCCTTCTGGCTGGCAAGCCCCTGTGGGCCAGGAAGCTCAGACCTAACC | 51 |

(continued)

| cg identifier | DNA Sequence | Sequence ID Number |
|---|---|---|
| cg13406893 | AGTCACTGAATCATGAGTGACGGTAACATAAATGCCCATCGGGGGAAACG | 52 |
| cg12384004 | ACTCGCCCTGATGTTCATTGGGGTTCAAAATAATCAAGTTCTGCAGAACG | 53 |
| cg17116694 | GGCCAAGGCAGGCGGCGGCTGGGAGGCGTAGGTTGTAGCGAGCCGAGATCACG | 54 |
| cg25939203 | ACGATGTTGGCTCACTGCATCCTCCACCTTCTGGGTTCAAGCGATTCTCG | 55 |
| cg02922734 | GTCATCCCCGGGGAGAGGCTCTCAATGGGGAGTCCCATCTGCAGGCTGCG | 56 |
| cg01873886 | CGGGTTCCTTTAAAGTGCTGCTAGCGCGCACTCGCCCTCTCAGCGTTGC | 57 |
| cg01637125 | CAAGGCCACTGCAATTTTTGGGATCTAGTGAGTCTGTAGGAAATGTAACG | 58 |
| cg10920224 | TGAGGTCAGGCGTGCCCTTGCGTAGCACAATCGCGGGGAGGGGTGGCTCG | 59 |
| cg18425651 | GCATGTGGCATGGGTGGGGTGGGCCAGCACGCTACAGGGGCTTCCTATGCG | 60 |
| cg13316619 | CGGAAGAGGCTCCAGTGTCCTCAGCAGACATGCTTTATTGGTTAAAGCA | 61 |
| cg23047825 | AAGCTTCAGTGAATGTCCCCCGCTTCTAAGATCCTCTTCTTTTTGTCACG | 62 |
| cg08923669 | CGCCAGGCTCGCGATGCCTCTACACAAGTATCCCGTGTGGCTCTGGAAGC | 63 |
| cg06259664 | CGGGGAGAACCTTCTGTGCAGGCCCGGCGCTGCTCCACTCCAGGAAAGAAA | 64 |
| cg05090127 | GAGGCAGGGCAGGCAGACCTGCAGTCACTCCTCTGCAGAGTCGCACACCG | 65 |
| cg01960979 | CGCAGAGAAGGAAATAAACTTGTTCAAAAGAACCTACTGGGGTGTGTGTAC | 66 |
| cg06213463 | GGGAGTGGGCTGGCAGTGCGCGGCACAGCGGGGGAGTGGGTCGTGCACG | 67 |
| cg01773760 | TCGTTCCTCATGAAAACATCTTGAGTCCAGGATCTCCAATTGCACTCACG | 68 |
| cg01130056 | TTCTCTCAAAACTCATCACCCACTTTTCTTTAACTGTTTGTTATAAGACG | 69 |
| cg03974717 | CGGACCTAGACTCCAACAGTGACAGCTCTGAGTAAAGAAGTCGCCGAAGC | 70 |
| cg26573797 | GAAGGAGAAGCACCCGGAGCCGGGGGCTCCTGGTGTCTACTGCAAAAGCG | 71 |
| cg06127256 | TGGCCGGGATCCCGTGCTTGGCATACAGCCGGTCTTGTTCTCATTGTCCG | 72 |
| cg03574306 | TTCATGGGTGTGGTAGCTTGTGAGACCGGTCTTGTTCTCATTGTCCG | 73 |
| cg08439518 | TTTAACCTTTCCAGGCCTCAGTTTCTTCATCTGTTCAGTGGAAATGATCG | 74 |
| cg11235787 | TAGTTTTCACCACCCTGCCTGGAGCAGCACAGCCAATATTGGCAGACTCG | 75 |
| cg20361594 | CGCAAGCCGGCCCCTCACCCAGTATATTGATAGGGCCATTGAGGGGTTCCG | 76 |
| cg13029847 | CGGCCTGCTAAGGAACCTCAATTAATAGCTCACTGTAGCCTTCTGATTCT | 77 |

| cg identifier | DNA Sequence | Sequence ID Number |
|---|---|---|
| cg00126959 | GACCCAGAGGCACTGGGGTAGGGCTCGGCAGGGCGGGTCCGGGCGGAGCG | 78 |
| cg24093538 | CTGGGCTCCATGTGTAATTTCAGGGCTCATGGCCACACCCAGCATTGACG | 79 |
| cg16067628 | CGGGTAAGCCCCGCTGTGTTCTCATACCCGAACGGCAGGAACACAGGCAG | 80 |
| cg15258649 | CGCACAGAGCCGCACGGGACTCAGTTTCCACACACACAGAGCCACACGGG | 81 |
| cg03294557 | TAAGAATAACTTTTGCCTCCTACTGTGATTCTGAGGCCTCCCCAATCACG | 82 |
| cg23777956 | GTGGTGACAGAGCCACAGAGGGCTGTGAGCTTGCCCGGCCCCAGGTAACG | 83 |
| cg03636183 | TGGTGGTGGGGCTGCCGGCCAATGGGCTGGCGCTGTGGGTGCTGGCCACG | 84 |
| cg06418475 | CGGGGCGCTGAGCTCAGGCCCAGAACTGGCTGATTTCAGGGATACCCAGG | 85 |
| cg27152890 | CGGCGCAGACACCCCGTTCGGACGATCAGAGAGTGCCCCAACCCTACACC | 86 |
| cg09438320 | GATGACCGGGATACAGTTTGCTGCATTGCAAGGGCTCACCCAGTGTTCCG | 87 |
| cg12157673 | GTCAAAGGACAAGCCCCTGGGACCTGCCCAATGCTGGTTCAGGGGAAGCG | 88 |
| cg22455450 | CGGGGCGGAATATACGATTTCATGCTGATGGCCCACAGAACATAACAGAA | 89 |
| cg08980304 | CGTGTGGACGCGGGAGGAAACCAGAGTGCCCAGAGACAACCCACGCAGAC | 90 |
| cg26881591 | CCATGCAGGGCTCTAGACAGCGGCGCTGCAGAGCTTCCTGAGGTCGGCCG | 91 |
| cg14528537 | CTAGCTTTAGCTGCACATTAGAGTCACCTGCAGCTCTTTTCAAACTGTCG | 92 |
| cg04053045 | GGGGGGTCAATACTCACCTGGTCGTAGACAGGAGCCCTGGAGTCGGAGCG | 93 |
| cg05515143 | CGTGTCTCAGTGACGCTGATTCTGCTTCACAACAAAAGCAAAACAAGAAC | 94 |
| cg06625640 | CGGAAAAGGGAGTGGACAGGAGGGGTACAGAACCAGGCTAATGAGACAAT | 95 |
| cg00102512 | GCTGGCTTTCAGGATCACTATCCAGCAGCAGCTCCGACTTCCAGATTCCG | 96 |
| cg06372850 | CGCTCTCTGGCAAATGTAGAGGCTATTTCTGCTTTTTACAAGAGGTGCTG | 97 |
| cg16978914 | CGGCCCTCCAAAATTGCCCCCACATTGGCTGCCTTATAAATATGTCTGTG | 98 |
| cg07006683 | CGGGTTCAGTCATTGAAAGCCTTCTTGTAATTGCTGAAGGGTATTTGTTT | 99 |
| cg06644428 | CCTCTTTCTGCTCGCCGAAGGCCGCGATGGTGCCATAGGTGAGCTTTTCG | 100 |
| cg21566642 | CGCCTCCTGAGCCTGACTCTGCTCTTCCCGCAGCTTTGGCCCCCCGGGGC | 101 |
| cg21120063 | CGGGCACGATGTCCCAGGAGGACAGAGTTTGCATTGATGAAAACCCTTCC | 102 |
| cg00356999 | CGCTGGGTCTGCTCCCATTGGCTCAGGTCATCTGAGCAGCTGTGCTCTGT | 103 |

34

EP 3 864 177 B1

(continued)

| cg identifier | DNA Sequence | Sequence ID Number |
|---|---|---|
| cg02931526 | GGTGCTCTGGACCGGCGGCAGCCATGGGAATGTCCTAGTCGGCAGCAGTGTCG | 104 |
| cg19393314 | CTCACGTACAGTCTCCTCCCTGTACACGAAGGAGACTCTGCTCTAACACG | 105 |
| cg05303999 | CGCTGGGATCGTGGCTTATGGCGCAGAGACTTCCCCATGACCAATCATTC | 106 |
| cg16416603 | CGGGCACCCTGAGGTTGCCCCCATTCTTTGAACAGCTATGGACGGGGACC | 107 |
| cg05276137 | CGGGCTTCCCGGCAGCAGGTTACTCTTTAACGCTCGACTGCCCACCTCCA | 108 |
| cg16983817 | CGCCCTGATTGGATAGTAGTGCAGGCACCGCGCCCTCAGGCCCTTGCGTGT | 109 |
| cg00744433 | CGCAACTGCAGTTTAGCCTGGTGAGTTGCAATTCTGGCTGCACATGAAAA | 110 |
| cg08706141 | AGTTTTACAAGCCCTCACATAAATCTACACAGCTAACATGCACAAGTCCG | 111 |
| cg03860256 | CGCCTTGCGCGCTCGGCATGAGCGTCACTCCCCACCAGGCACCTCGCCTTGT | 112 |
| cg15876825 | AGGCGGATCCTTGCGCTTGGGCAGACAGCAGCACGTCACGGCTTTTATCTGCCG | 113 |
| cg21770393 | AGTCAGACGTCAGGACCTGCTGTGGGGATGGACCCACAGCTCATCCCCCG | 114 |
| cg01206872 | CCCAGTACTTTGGGACGCCAAGGTGGGTGGGTCACAAGGTCAGGAGATCG | 115 |
| cg02019988 | TACCCCAGCTAGGCCCCTTCCCCGCAGAGGATGTGTTTCTGTGTCCTCG | 116 |
| cg04785972 | GAGGTGGGCGAGGCAGGGCCCACAGACATTGGTGCAGGCTCAGCCGTGCG | 117 |
| cg19859270 | CGACTGGCCACACAATGGCCAGGTAGCGGTCAACACTCATGCAAGTGAGC | 118 |
| cg16665506 | CTTCCCCTCAGCCTTGGTTTCCATATCTGGAAGATGGGTTACCAATAGCG | 119 |
| cg01328703 | ATGTCACTTGCACAAGAGCCAGTCATGCCACAGGGCTAGCCTTAATTTCG | 120 |
| cg17894318 | AAATAACTTGTCCTTTACTTACCCTGCACATCAAATCTTCTCGCCCTGCG | 121 |
| cg17500754 | ATTTTTATTTTATTCCTGCCATTGTTTCTCCTTCGAGTCAATATCTTCCG | 122 |
| cg21924273 | GGGGCGCCAGCCGGCGGAGGAGCGACGGGAGCCTGGGGTGGGTGGCGAGGCG | 123 |
| cg22518157 | CGGCTGTGGTTTCCTGTGCGTCCCAGGTGAGATCTTGTGCTCTTTACAGGGT | 124 |
| cg22659049 | AGGACGACAAATGGCAAGATGTGAGTTGTGGCCAAGGCGCGCGGGCAGCG | 125 |
| cg25806704 | CGGGCTTAGTAAGCCCCTGGAGAAAGGAATCAAAGATGTTTCTGAGGCCT | 126 |
| cg07973162 | CGCTGGCCACCCTCCTAAAGGATCACTGTGGTGCCAGGCAGGAATGAGCT | 127 |
| cg10144483 | CGGTGTGCAAACCTGCAATGAATTGCCCAGTGCCTTCTTGTTTGTAACGT | 128 |
| cg11554391 | ATTATAGAGAATATTAAGATGCTTTTGGATCCTGAGCTTCCCAGGAAGCG | 129 |

35

| cg identifier | DNA Sequence | Sequence ID Number |
|---|---|---|
| cg05575921 | CGGCTGGGTCTCATCTGACACGCAGCCTTCCAGCCTCTCATTGCCCGAGG | 130 |
| cg03604011 | AGGGAAGTTGGGAGTATTGTGCTCCCCTGAGAGCCGTTGCCACGGTTACG | 131 |
| cg12035880 | CCAGCAGCAGCGGCAGCAAGGTTTACAATCCTAAACCATGCAGGCTTCCG | 132 |
| cg19601991 | TAGCCATTTGGCCTCGGCATCACGGTGCGGGAGCGTGGGGCGCAGACTCG | 133 |
| cg26921969 | CAAGCAAGACTCTCCACCCACAAACTGCATATTCTTTAAAGTCACTGTCG | 134 |
| cg04836987 | CGCTTAAGGACCTTATAGCTAGCAAATGGCAGAGACAAGCTTGGGGCAGA | 135 |
| cg15658426 | AGATGTTATCAGCTCAGCGAGACTTTCCATGATCTCCCTAAAATGAATCG | 136 |
| cg21777188 | CGCCCAGAACTGGGACTTCCCAGGTTCTCATAGTGATTGTAGATATCAAT | 137 |
| cg05845376 | GCTAGTGAGCCGCGCGCTGGGCCGGCAGCAGGGGGTTATTTTAGTGCGCG | 138 |
| cg07039560 | GGCTGGAGGCGGCTGGGGGCCCGGCCAGTGCGCCAGGAGCCGCAGGAACG | 139 |
| cg25587069 | GGCAGGCAGGCGGCAGGCAGAGCGCGCTCTCCGGGCAGTCTGAAGGACCG | 140 |
| cg13667243 | CGCTCCGCCGGTGCTCGCTCTATTTGAAAACGCTGACTGTTGGTCCCCCA | 141 |
| cg12363682 | ACAGAACCCCAGAAAAACTGTCCCACAACCCCCAGTGAATGACCAAATCG | 142 |
| cg07412232 | CGGAAGCTGCACCCTAAATTCCCCGGGACCTGTGGCAGGCCTTCCTGGTG | 143 |
| cg15174951 | AGCCACGAAGAAAACAGTTCAACTCAGACCAAAAGATAAAGCTGATCTCG | 144 |
| cg18892128 | GCCTGGTCCCCGAAAGCGCTCAGTCAGAATCCGAGAGCACTGTGGCGGCG | 14.5 |
| cg14924781 | CGCCTCTCACAGACTGCTTCCCCAGCAGGGTGCAGCTTCTTACAGACTGG | 146 |
| cg25955180 | TGATTCAGTCCAGTGATTGGGTTTGTGGCTCCAGGCCTCGCCCACAGACG | 147 |
| cg27067781 | CGTCTCGCCTTGCGAGCAAGCTCGGAATCCAGTTCCTCAGGAACCCCTCC | 148 |
| cg21091547 | CAGCAAAGAAATGACTATAGTTGGAACACAGGACTTTTGCCTCCTGCCCG | 149 |
| cg26470115 | CCCATCCATGACTTCTTATGTGTGCTGTCGGCAGCTGACCAGCGGCCACG | 150 |
| cg04344923 | AGGCAGCAGCCCAGTGGCCTCTTGCTGTGGAGCGCGGGGGCAGCCTCCCG | 151 |
| cg06413272 | CGGGTGTTGTGTCTCTGCCTATCTTCACACATGCACCCTTGTGAACTTGG | 152 |
| cg02817030 | CGGACTCATTGCCATGCATGCCTAGACATTAATATGTGTTCATGGAGGGC | 153 |
| cg26403206 | TGTGCTCAGATTCACAAAGTGAAAGTGCAAAACCCCGTGTTAGCTCATCG | 154 |
| cg15777014 | CGGGCTATAGCGAGACCCCCCATAGTGAGATCTGCTGCGGGACCTAGAGT | 155 |

| cg identifier | DNA Sequence | Sequence ID Number |
|---|---|---|
| cg01704252 | CGGTGGCCTGTTGAGGCCCAACTCATGCCTCTGGCACCCTTTCAAGAGGC | 156 |
| cg15600935 | GCAAGGTGAACCCACCTTGATCCCGGGGCTGCAGAGCCAGTGCGCCTGCG | 157 |
| cg25949550 | CGGTTGGCAAGAGCTTGGCTAGAGAGACTGATGTAGATGGCAGCTTCTTA | 158 |
| cg11207515 | CGAGAGGCATTTCAGCAGAATTTTGAAGGTGGAGTGAAGGAAGTCACTAT | 159 |
| cg09159795 | AAACACTCTCCCGGCTCAGAAACGGTGCAGGTGCATTTACATGTCAATCG | 160 |
| cg07338119 | CGCGGGCTCTATCTGCAGCATCCACCCCCTCAGCAGACGCTGGCATAAGC | 161 |
| cg09608412 | GGGGCCGCAGTGTCCTTGGCTCAGGCCTCCTACGCAGGGAAGCAGGGTCG | 162 |
| cg14700531 | CGGGAAAGTATCACTTGTGACTGTTTGCACTAGGTGCAAAAGACAGATAG | 163 |
| cg03900851 | TCTGTAGAGAAGTCGACCGAGAAGTGAGATGCGGTGGCGACCTCGGAGCG | 164 |
| cg27272363 | CGCTGCCCATGCCCAGACCAAAAATGGGGTGTCTTTTAGTCTAAAAGGA | 165 |
| cg16821867 | CGGCCAGACCCGTCACTCAGATCCCCGCACCAGGCCAGTGAAGAGGAGCC | 166 |
| cg15410835 | CCAGCCCTTCTGGCACAGATGCTGTGCATGTGAGGTGCTTCTCAGAAGCG | 167 |
| cg00844308 | AGGAGATGAGCTATGTCTTCCAACCTCTCCTTTTGGAATAAAACCTTTCG | 168 |
| cg13728106 | CGACGACAAAGAAGATTTGATGAAGTTCAATTAGAGTCAGCGTGCTCA | 169 |
| cg27087885 | GTAAAGTGACACCCATCCCACTCATCCCATTGCTGCCAATGACTTCAACG | 170 |
| cg20448001 | CACCTTCCACTTTAAAGAACACCAAGGTCGGCTGGGTGTGGTGGCTCACG | 171 |
| cg15480287 | CGAGCCCAGGAAATAAGTGCGGGTGACCGAATTCAATCCTGCATCCAACA | 172 |
| **cg19414383** | CGGCCCCACTTAGCCTGAAACGGGGCCCCATGCAGGGTAGAGGCCTGCCA | 173 |
| cg25536676 | CGCACACCGAGTGGCACGACCTGCAGAGGTTACCGCCAGGTTTCCATCCT | 174 |
| cg22820188 | TGGGGTGAGCCACTTTCATTTTCCCAGCGGGGCCAGGCAGTCTTTGCTCG | 175 |
| cg08410533 | CGCAGGTCTGAGAGACATCACAGCATCACAGCGTTTAGTAGTCTTAAATG | 176 |
| cg12483947 | CGACTACCACTGCTGGTGCAAGGACCAATCAGCGACCTATTGAAGTGCCT | 177 |
| cg23371436 | CGGGATGGTGTGACAGGCAAGCCAAAGTTGTATCTGAAAACTCTGCAACA | 178 |
| cg24894584 | GGTCCGCTGGGGCAGAGTGCGGAGTGAAGGGGTGCACTGGGCACTCAGCG | 179 |
| cg26266429 | CTTTAAGATGTCTGACAGTGCCCTAAGGGCACGTGCTCCACAAACAAACG | 180 |
| cg10068417 | CTCAAAGAAAATAAGCCAGGACCAGGTGACCCAGTGGGATCCGCGAAGCG | 181 |

| cg identifier | DNA Sequence | Sequence ID Number |
|---|---|---|
| cg25572904 | TGCGGCCACTGCAAAGCGATGTAAATGTCCCAGAGTGGGGCAGCTGGTCG | 182 |
| cg13543355 | CGTGCCACCTGGCCAACCTCGAAAGGAGGCCCTGCTGGGTTCTTCATCTT | 183 |
| cg26481784 | CGGCGGACCCAGCTGACCAAAGGAACTACAGAAGAGGACGAAGAAAGCGA | 184 |
| cg13898384 | TCGCTTTCCAGCCTTTTGTTTCTGGATCCTCAAAACTCAGAACGTGGCCG | 185 |
| cg19459791 | TGGAGCAAATATAGGTCTAGACACGTAGGGCCTGTGAGGTTTTCCTGACG | 186 |
| cg03172657 | GCGTGTGCTCAGCGTGTGGTTACCGTGTGCACAGCACGAGCTTGAACTCG | 187 |
| cg18181703 | CGTGGCCACTCTTCAGCATCTCTGTCGGAAGACCGTCAACGGCCACCTGG | 188 |
| cg07573872 | CGGGAATGGTGACTCAGCCTTCCAGGAACCTGCGTGGCGTCTGTTTTTTT | 189 |
| cg09051966 | CGGGCGCAGCCCCAGGATGCAACAGCTCTCATACAATTGGCCAGGAAGGC | 190 |
| cg25841091 | CGGGTTTCCCAAGTGCTGGACGCGCAACAGGTTTCCATAAACCCAAAGTT | 191 |
| cg12516875 | CGGCGAGTGAGTGAAAAATTACATTTTCACTTGGGTTCTGAACATTCAGT | 192 |
| cg10753966 | CGGGGAAACTCTGAATTGGTGGTTGCTTAGAAAACACATAGAAGTGTGTA | 193 |
| cg23944298 | TCAGGGTTGAAAACGCAACAGCCAAAATTAACTCAGTGGATGGCTCAACG | 194 |
| cg07553761 | CGCCGGTGGCCGACGGCTTCTGAGGAATTATCTTTTACTTGGCGCCACAC | 195 |
| cg10202557 | CGAGATTAAAGATTCAGAAAGTGAGTAAAGTTAACACTGAGCCCTTGGGC | 196 |
| cg05604079 | AGTTGGTGAGCCAGGGAAGTCTTCCCTCGCCCTGAGGGTGCGGGGAAGCG | 197 |
| cg11444009 | CGGGGCCAAAGAGGAGCGCAGGTTTAAAAAGCAAGTGTATTAGCAAGTTT | 198 |
| cg25149516 | CGCCCACGCGGACCCCTCTCCGTGTGCTTGTCTGTACTTCCCTGATGGGG | 199 |
| cg18584747 | CGGCCGGAGGGCCCTGAACCCTAGGCCAAGACTCATCCACAACCTAGGGG | 200 |
| cg20405584 | CGGGAGATCCTCCCGCGGCCGGAGGGCCCTGAACCCTAGGCCAAGACTCA | 201 |
| cg11299854 | CGTGAGCGGGATCCTGAGTCTGAGTTGCCGCTTAACCCGGCATTTTATAA | 202 |
| cg24708145 | CGGAGGCAAGCAAGCTCCATGTGGTGGGGACAGCGCATTGCGACCTCCAC | 203 |
| cg25663874 | CCATGCTATAAGTGAGGTAAATAAATATGCTGAGCACCATCGCACTTACG | 204 |
| cg25325512 | CGCGGCATTCAGCAGAACTCATGATGAAGGAAACAGTTCCATGGCAGGGT | 205 |
| cg13001142 | CGACAAAATCTGACCAGGCTAGATCTGAATCTGTTAATTTCTGAGACGGT | 206 |
| cg06649410 | CGCAGAGGAGAATGTCAGATGCTCAGCTCGGTCCCCTCCGCCTGACGCTC | 207 |

| cg identifier | DNA Sequence | Sequence ID Number |
|---|---|---|
| cg17501210 | CGCCCGATTCAGACAGCTGGACTCAGAGGGATTCTGCTCCACAGAGAAAC | 208 |
| cg03473532 | CGTATGTGTTTGAGATAGCAGTTGTTTACTATCACTTGAAAATTCTGAAT | 209 |
| cg20468415 | TCATGAACTGGAGCCTGAAGTGGCTCTGAGGCCTCCCCTGGAAAGGAACG | 210 |
| cg11782409 | CGGTGGATTCCTCTCGGGACACATCTGCCACCCGCTGGACTGGCAAAACA | 211 |
| cg07205627 | CGACCATTTGTATGTGTATCTATGTCAGAAAGAATCTTTATTAAAATATT | 212 |
| cg07274490 | TACCATGTAATATACCACTAAACACACTATGTAATACACTACTAAACG | 213 |
| cg07929447 | TTGTCTCAGATTACAGGAACCAAATGCATGTCTGGCCTTGTGCTGCTTCG | 214 |
| **cg00695391** | CGAGCCCGCCCAGCCTCTGAACCTCCGTGTCTTCATCTGTAAATTGAGGG | 215 |
| cg16305333 | GTGCTCTCAGTCCAATTCACACTGAATGAATGAATCAGTCCCAGCCATCG | 216 |
| cg00970752 | CGCTGAGGTCCCGTTTCTGTAGCTGAGTAGCCCGGTGACACTGCCCTCCG | 217 |
| cg04967775 | AGGGCCCCCTGGGCCTGTACCAAGGTCTGCGACAGATGGGTGGGGAGCCG | 218 |
| cg06901711 | CGCCGCAGTCCCAGTCGAGCCGCGACCCTTCCGGCTGCCCCCACCCCACC | 219 |
| cg01431830 | TGTGCCTTGACGGGCTGTCCCAGGCGCCCLIGGAATTGATGCAGTGTCCG | 220 |
| cg10578779 | CGCAGAAGAAAGCTGCTCAAGTGCTGGAGTGGCCAACCCCATGCCGTTCC | 221 |
| cg26359730 | CGCACATCCTCAGCTAGCAGCATGGAGCTGAGTTCAGAGGAGGCACTTAG | 222 |
| cg10585941 | CGGGAGGGAAAGGTGTTAGATGGTGGTTTTAACTCACCAGGGTTTGAATT | 223 |
| cg01939962 | CGGCCTGATTCGAAGACTGGAATAGAGGATTTCTAGAATTCTGTGACTCC | 224 |
| cg06809285 | CATGTCCCAGCTCAGCTGGTCCTGAGAAGCTGCCTGGTAGACAGTCACCG | 225 |
| cg11336382 | TAGAACCAACTGGATACTGCACCCCTCAGTATTGGGGCGTCACAGCACCG | 226 |
| cg08410533 | CGCAGGTCTGAGAGACATCACAGCATCACAGCGTTTAGTAGTCTTAAATG | 227 |
| cg04196119 | CAAGTCCTTTCTGGTTCCCGTGGTTTTTCAGGTACTGCGGTTCTTATTCG | 228 |
| cg12329405 | CTCCCTACGTGGGGGACGTGCAGGATGATGCGGGGTCGGGGGGGATTTCG | 229 |
| cg12379046 | TCCGCAAAAAGCAGGATCCATTCACTTTAGAGAAGTGCCCTCAACAGGCG | 230 |
| cg03950121 | TGTGGTCTCCCCAGACCACCAGGTGCTTCACACACGCACAGGGGCACTCG | 231 |
| cg03096347 | TGCAGCCTCGTAAGCCTCATGGAAGAGCTTGGTTTGTATCCTTTGGAACG | 232 |
| cg22620356 | CAGTGCTGAGCCGGATCCAGAAAATTGAACAGGTCCTGAAGGAGCAGCCG | 233 |

| cg identifier | DNA Sequence | Sequence ID Number |
|---|---|---|
| cg17310258 | CGTCCCCGAAATGAATAATGCAGGCAGGAGCCAAGATTTCTGCATTAGCG | 234 |
| cg18886347 | CGGTGTGGAGGCTTTTTTTGTCAGAAGCTCTGGGGTTTGGCTCTTCTCTG | 235 |
| cg14592365 | CCTCTTTGCTGATCTGGACTCAGCCTGGTATTCTTGTCACAAGGTCATCG | 236 |
| cg07631435 | TTTTTAACCAGTTTGAACATTTTACTACTTCCTAACATCAGAAGCACCCG | 237 |
| cg03112631 | GTGTGGCAAGGCTGAGCTAGTTCGTCTGCCAGGTACCAGAGCAAGCCTCG | 238 |
| cg01635346 | CACTATGTTTGCTGCTTGCTATTCACTTATGGTACATACACAGTCAGTCG | 239 |
| cg25966649 | GGGACGGTGGTAGTGCTTCTCAACATTGGCACTGGCTCCATTGTCATTCG | 240 |
| cg23510415 | TGGGGAGCCAGCTGACTTAACAGGCCTGGATTACATTCTTGCTTCCAACG | 241 |
| cg18159533 | TGCCATTTAATCTAACGCCACCTTCTACGGACCAGAGCAGGTGCTCTGCG | 242 |
| cg16982073 | CGGTTAAATTCGTGAGGCTGTTTGCTGTGTACACAATGCTAAGCAGAAAA | 243 |
| cg25572904 | TGCGGCCACTGCAAAGCGATGTAAATGTCCCAGAGTGGGGCAGCTGGTCG | 244 |
| cg02150207 | TCTGGTCCTGGCCTGGGGGGGTCGTGGTCTCGCTGGTGCAGCCTCCTCCG | 245 |
| cg13649020 | CGCCACCATCGAGTGTGAGCAGCCCCAGCCCGACCTCTACAAGTAAGCGG | 246 |
| cg06903325 | CGCTGTGAAACCTTTGCCTTTGGGTGTCATGGTGGAAGCAAATCTTAGAA | 247 |
| cg05078817 | CGCCAGGGCAAACATTCCTTGGGCGCCTCGGGCTTTTCCCTAGGGAAGCA | 248 |
| cg13898384 | TCGCTTTCCAGCCTTTTGTTTCTGGATCCTCAAAACTCAGAACGTGGCCG | 249 |
| cg13634681 | CGGAGAGACAGCCGCTGTGGTTAGGGGTGTGCATCCTTCTACCATGCCCA | 250 |
| cg01624571 | CGTGGATCTAGCCACAGAAAGGCAACAGCGCTTCAAAGAGGCTCTCCGTA | 251 |
| cg14153919 | CGCTTTTGCAAAACAGGCCGCACCTAGCCCAGATTCTGGAGCTGATTCAG | 252 |
| cg05575505 | CGACTCTGAGTTCTGACAGGGAAATACAATGAGTTTTATGTGGACAGAGT | 253 |
| cg19987111 | CTCTCTCCCAGACCTCAGGTAAAATTAATGTTCTAGGAACGTATGCCACG | 254 |
| cg05638439 | TGGGGTGGCCGTCTGCTCCGTCTTTGGTGGTGAGAACCCGCACCGGTCCG | 255 |
| cg26470314 | CGGTGTCTCCGCTCCTTGCCCGTGGCCAGGTGGTGGTGCTCCCAATGCCC | 256 |
| cg24429836 | CGGGCTTTAAGGTGGCCCTTGCAGATCTTGCAGACATTTTCGTGGGCTCC | 257 |
| cg04120520 | AGACTCCCTCTGAACACCTGTGGAACATGCTGACCTCCCAGTGTCTTTCG | 258 |
| cg09319676 | CGCACATGTGAGGCCACCATTCATACCACACCCTGGGTGGGTCTTGAGTT | 259 |

| cg identifier | DNA Sequence | Sequence ID Number |
|---|---|---|
| cg20433952 | TCTGCTCATTAACACCCTGTTAATAGGAACCCCAGAAACCTGAGCTTCCG | 260 |
| cg02220125 | CGGCCTTGCACACCATCTTAAGTGTGACGCCACACTGCCCTTCACAGCCT | 261 |
| cg18181703 | CGTGGCCACTCTTCAGCATCTCTGTCGGAAGACCGTCAACGGCCACCTGG | 262 |
| cg10701033 | CGCGCCTGGTGCAGCCCTCACCTCACCAGGTGTTGATCATCTTAGTGGTT | 263 |
| cg07529654 | CGCACTTAATATGATTATTTTTTGATGCCAGGTCCACCAAGTGAAGATTG | 264 |
| cg27145096 | CGTGTAAAGGTCAGAGGTCCATTCACCAATGCAATGATCCTCACTGCCCC | 265 |
| cg07573872 | CGGGAATGGTGACTCAGCCTTCCAGGAACCTGCGTGGCGTCTGTTTTTTT | 266 |
| cg14528319 | CAGTATCCTAAAGCGTGCTGTATCTCTCTGTCCGGCTTTTGCACG | 267 |
| cg09920725 | CGGTCTTGTAACAATTGGATGGATGCCTTTGAAGAGCCCCTGTCCCTATT | 268 |
| cg24000528 | TCTCCTCCCCTGCACTGGCTCTGGGTGCTTATCTCTGCAGAAGCTTCTCG | 269 |
| cg24000528 | CGGGGTCTAGGGAAGAGAGATTTCCTAACCTAGACTACAGGCATCAGGAG | 270 |
| cg08007899 | TGATTCCTCCCCCTTTGAAAGAGCAGGCAGCATTTTTCCAAGGCAACCCG | 271 |
| cg15808795 | CGCTGGTCTCGGGGGGGAACCCAACAGACTCACATTTCATGTGAAAGGAA | 272 |
| cg09845806 | AAAGATGTCATTACACAAATACACAAATACTAGCATGCAGACACACAACG | 273 |
| cg03706056 | GCACCTTTGTGAGGTGTTTGTGGGGTTGGGGGAGCTTCAGGCGCTACTG | 274 |
| cg24110177 | CGACAACTCTGTAGTGATTTAGAAGAGAATTGCAAGACATTAACACAGAG | 275 |
| :cg10753966 | CGGGGAAACTCTGAATTGGTGGTTGCTTAGAAAACACATAGAAGTGTGTA | 276 |
| cg00481951 | GTTTCAGCACCTGGGTCAGCGCTTCCCAGGGTCAGCACCAGGGATAGACG | 277 |
| cg04748223 | CGCAGCTGTCGTCTTTGTACCCGGCTTTGCGCACTCCATTGTGGACGCGA | 278 |
| cg22518157 | CGGCTGTGGTTTCCTGTGCGTCCCAGGTGAGATCTTGCTCTTTACAGGGT | 279 |
| cg22714094 | GCACCTTTGTGAGGTGTTTGTGGGGTTGGGGGAGCTTCAGGCGCTACTG | 280 |
| cg10177080 | CGGTACTTCCCTGATGGGGCCCACGCGGACCCCTCTCCAGTGTGCTTGTC | 281 |
| cg15845365 | ATCTTCCCAGTACAGAGCTTAGCATACATTGAATAACTGGCATCTGCTCG | 282 |
| cg27628536 | ACTCTGTCAGATATCCTCGATATTTGGGGATAGTAGTTCAAAAACAGCCG | 283 |
| cg24765579 | AGCTGTTTCCCTCAGGCGGCCTGTTCCACGCCAGGATTAGTTTTGCTTCG | 284 |
| cg15988937 | CGGTTTGCAGGTTCAAAGATGTCTAAAAGAGAATGGCCAGAATGTATATA | 285 |

| cg identifier | DNA Sequence | Sequence ID Number |
|---|---|---|
| cg02081065 | ATCCCTCATCTGGACTTTGAGTGATTGTGTTGCTGGCATGGACCCTTTCG | 286 |
| cg12612118 | CAGAGCCCACTTAGTGCGCGCTAGCTGGGCAGGGATAGGGGTCCTATTCG | 287 |
| cg12298697 | ACAGTATTTTTGTCACTCCAAGTATCCCTTATCTTGCCCTTTTATGGTCG | 288 |
| cg20660989 | CTGAAAGGCGCATTGTCAGCCTGGGGAGGCGACGCGGCGCGAGGACAGCG | 289 |
| cg03546163 | CGGAGGGCTTATTCTATGTAAATAGTTGAAAGGAACTGGATAAGACTGTA | 290 |
| cg25325512 | CGCGGCATTCAGCAGAACTCATGATGAAGGAAACAGTTCCATGGCAGGGT | 291 |
| cg00684824 | CGCCCAGCTGGAGGCTGAGATGTCAGGATTTGTCTTGCAAAGTTACTTCA | 292 |
| cg02713068 | CGCAAAACTTAAGGCTATAGAAGATGGGGAGAGTATCTTTTGATGCAGAC | 293 |
| cg06649410 | CGCAGAGGAGAATGTCAGATGCTCAGCTCGGTCCCCTCCGCCTGACGCTC | 294 |
| cg17501210 | CGCCCGATTCAGACAGCTGGACTCAGAGGGATTCTGCTCCACAGAGAAAC | 295 |
| cg03807873 | CGCTGCACTCAGCCACGTTACGCGGAGTTCATCTGGGGAAGGAGACAAAT | 296 |
| cg21899558 | GAAGGGTCACACATGTCCCAGAATCCCCCAGGAGTGATGCAAATGGAGCG | 297 |
| cg07025011 | AAACCCTAAACACTGAAGTCTCCTCTGGTCAGGTGACCCCTATCAGTCCG | 298 |
| cg16847637 | GCCAGGAGTCCTCGGTCCTGGGAGGGTTTAAAAGCCAGGGGGCCGTCTCG | 299 |
| cg06563451 | GACCCTGGGCTTGGGGGGATTTTTTTGCCTGGAGGGCTCCTCTTCTCCG | 300 |
| cg02716826 | GGCCAGACACCCAGGACCCAAATAACCTAATAATGGCTCATTGCCTCCG | 301 |
| **cg13947317** | CGGCATTGGTCCTTTATTGAACATCCTCCCAAAGCTGGGGCTAGGGTTCA | 302 |
| cg22510139 | CCAGATGTTTTCACTCGATTTTGCAAATCTGCTCCAGGTCTAAGACATCG | 303 |
| cg10116490 | CGGGTCTTCACTCTGGTTGGCTGCCAGATCCTCCAGGGGCGGGGTCTCAC | 304 |
| cg27396830 | TACAGAACCCCTGAAACATTCCTGCCAAATACAGTCGCACGAATTTGTCG | 305 |
| cg19414383 | CGGCCCCACTTAGCCTGAAACGGGGCCCCATGCAGGGTAGAGGCCTGCCA | 306 |
| cg03345668 | CGCACAGCAACTGGGGTGTCCTGACCAGTGAGCTAGAGTTCCACCTTACT | 307 |
| cg17719473 | TTAGGGCCTGGAGATGGGCAGACCCAGGCTCACATCCTAGCTCTGACACG | 308 |
| cg20569940 | CTTTGGCTCGAGCTCCAGGTGCAGATTTACGTTCATTTTCTTTTGTTCCG | 309 |
| cg26156167 | AAAAACTTTTCTGAGCCGGAGCCTCCAGCGCGCTGTGTATTCGTTTTACG | 310 |
| cg10578779 | CGCAGAAGAAAGCTGCTCAAGTGCTGGAGTGGCCAACCCCATGCCGTTCC | 311 |

| cg identifier | DNA Sequence | Sequence ID Number |
|---|---|---|
| cg15631106 | CGCAGATTCCCCATTTGTGAGCAGAGTTAAGCTGAAGGAGCGTGGGCATG | 312 |
| cg22704788 | CGGCCCCAGAAGGCAAGGCGATTGTTAGGAGGAATTAAACAGGCTAGTGC | 313 |
| cg20240347 | CGCCCGGCGACCACTGGTGGTTCTATGCTGCTGGGGTGAAAAGTCTGAAG | 314 |
| cg17803430 | AGGACACTGGCCAGCCTCCAGTTGGTTTCTGAAGCTAGCAGTCTTAGTCG | 315 |
| cg20800892 | CGCCCTGCCCTGGGATTCTCAGACCTTGAAAGCCACAAGTCTTGAAAGTC | 316 |
| cg08410533 | CGCAGGTCTGAGAGACATCACAGCATCACAGCGTTTAGTAGTCTTAAATG | 317 |
| cg12329405 | CTCCCTACGTGGGGGACGTGCAGGATGATGCGGGGTCGGGGGGGGATTTCG | 318 |
| cg14984434 | CTGAATTCTGGTACCTGTTAATGACAGCTTCTGAGAGGTTTCTTTTATCG | 319 |
| cg07690734 | TAACCTTGAACAAAGATCGTCGACGGGCGGCCCTCACTGGAAACCTTTCG | 320 |
| cg17310258 | CGTCCCCGAAATGAATAATGCAGGCAGGAGCCAAGATTTCTGCATTAGCG | 321 |
| cg14955976 | CGAGTTGTAAGTCAACCTGCTGGGTTGTGCTGAAGAGAAATTGGAGACTG | 322 |
| cg06975311 | TTAGGGCCTGGAGATGGGCAGACCCAGGCTCACATCCTAGCTCTGACACG | 323 |
| cg16246188 | CGGCTCTAAGCGGGGCAAGAGCTGCTTTTAATTTGAGACGTACACAGTTT | 324 |
| cg07631435 | TTTTTAACCAGTTTGAACATTTTACTACTTCCTAACATCAGAAGCACCCG | 325 |
| cg12211040 | ACACATACCCCACTAAAGAAGATTCACAAGGTTGGGTGCAGTGGTTCACG | 326 |
| cg11764442 | ACTATGTACCTGGAAATAGCAGGGGCTCAGGGTTTGATCCTATGAGACCG | 327 |
| cg10535933 | CGGCTACGAGCACAGAGCATCATCATCATCAGCCTGCCAGCCGGCCCCGC | 328 |
| cg01624571 | CGTGGATCTAGCCACAGAAAGGCAACAGCGCTTCAAAGAGGCTCTCCGTA | 329 |
| cg24521756 | CCTCTGGACAGTACTTTTGAGACACACTTATTTCTACCACGCACCTTGCG | 330 |
| cg07842062 | CGAGAAGTTCAAGTTCAAGCTGCAGAACACCAGTGTGCAGAAGGAGCACT | 331 |
| cg08072101 | CGCGCAGGAAAAGGGAAACAAACAGCAGCTTCGCAGCCTCCCTGGGCCTG | 332 |
| cg03955530 | CTATGGTCAACTGAGTCAGGGCTGGATTTCTCACCCACCCTGGCCAATCG | 333 |
| cg07839457 | CGCTCCTATTCACCGAACCCCTGGCTGGCTGACCCGGGAAAAACAGTCTA | 334 |
| cg04976151 | TCCACCAGGTCTCTTATCAATGGACAGTACAAACAGTACTACCACGAACG | 335 |
| cg03400403 | CGCGTGTTTCTCTGCGCTGTGGTCGTCATGGACCGGAAGCGTGTGCAGCG | 336 |
| cg04745805 | TTTTCTTCTTTGGCTTTTCCATCACTGCTGGCTACAAGTGTTTGCAACCG | 337 |

EP 3 864 177 B1

43

| cg identifier | DNA Sequence | Sequence ID Number |
|---|---|---|
| cg14545159 | GGGGTTGCCGGCACCTTCTCTTTCTCCCCAGGTCCTTTCATCTGATTCCG | 338 |
| cg18181703 | CGTGGCCACTCTTCAGCATCTCTGTCGGAAGACCGTCAACGGCCACCTGG | 339 |
| cg17100770 | CGCACGGCCTCCAGCAGCAAGCTGATGCTGTCCTACAAGAAGCCAAAAGG | 340 |
| cg27145096 | CGTGTAAAGGTCAGAGGTCCATTCACCAATGCAATGATCCTCACTGCCCC | 341 |
| cg13077366 | GTCCTAAAGCACCAGGATAGATGACGAGCAGGGCCAAGTCCACACAAGCG | 342 |
| cg21656736 | TTTAAGCTATCAATGTGGATGTGCTGGTGCTGAGACCCACCTCCAACACG | 343 |
| cg07573872 | CGGGAATGGTGACTCAGCCTTCCAGGAACCTGCGTGGCGCGTGGCGTCTGTTTTTT | 344 |
| cg21736089 | CTCCTCCGAGTAGCCGGCGCCGTGGATGATGCGCATCTGCTTGATGAACG | 345 |
| cg07543883 | ACTGGACCCTCGGCTCTTCCTTGGACTTCTTGTGTGTTCTGTGAGCTTCG | 346 |
| cg10224107 | TGGGGCTTTAAAAACCACAGCCCTTGGGCAGGAGGGACCTTCGATCCTCG | 347 |
| cg01909777 | CGCTCCCTCTTAGGTCACCTGCCATTTCAGACAGGGAAGAGAACAGTGAA | 348 |
| cg03760035 | AGCAGCTCCACCGGGGATGCGGCTCGTCAGGGGGTGTACGAGAACTTCCG | 349 |
| cg03706056 | GATGTATCTCCCACCCAGGAGTGTTTCTTGGCCAGGTGTGGTGGCTCACG | 350 |
| cg13092901 | CGCCACAGAGGGCTTCCCCAGGTCCAGGCCGGGCCAGCCTTGACCTCTAA | 351 |
| 15849439 | | 352 |
| cg12605080 | AAGGGTAGCTGCTGCCACTGCTACATTCTACAGAAAGCTCAATGAGTCCG | 353 |
| cg01163330 | CGGCTCCCTCACACCCACTGAGAGCATCATGTCCCTGGGCACGCACTCCC | 354 |
| cg10753966 | CGGGGAAACTCTGAATTGGTGGTTGCTTAGAAAACACATAGAAGTGTGTA | 355 |
| cg19501902 | CGCTGCTTCAACTGGAGTTAACCAAGTGAACACAGAGCTTTACAAAGAAC | 356 |
| cg13287247 | CTCGGTCTCGTGTGTCTGTGTGTGCCAGTCCCTTGTGCTGACACAGGACG | 357 |
| cg22930808 | TATGCAGTTGTAATTCTGACAGTATTGCCAAATTGTTCACAGTAAAGACG | 358 |
| cg08122652 | CGGCAGAGAGGCCTGAGATAAGGTI I IGCACATGGCTGGTAAGACTGGA | 359 |
| cg00959259 | CGGTTGTCAGCACCATATTTCTAAAATGCAAATCTGGCAAGTCATTTTCT | 360 |
| cg20576510 | CGCGGCTGTACCACATAGAGAAAGATATGCACTAGTTCAAAGAGAATGCC | 361 |
| cg10202557 | CGAGATTAAAGATTCAGAAAGTGAGTAAAGTTAACACTGAGCCCTTGGGC | 362 |
| cg27134386 | AGGCCCCAAGGTTCCTGACAGAGCTGCTGTTGGGTGTTGGTGTAAAGCCG | 363 |

| cg identifier | DNA Sequence | Sequence ID Number |
|---|---|---|
| cg10584300 | AAAAAGACTGCAGCTCCCATCTTAATTGCTCTCTCATGCTTGTGTGTGCG | 364 |
| cg03274876 | GAATTCTGCTGGCGCTGCAGCTGCAGAATGGTCGGCGGTGGCGGGAAGCG | 365 |
| cg17287155 | AGCTTCAGACACAGTCAACGTGCTGCCAACGTGGGGAGGGCCGGAGGACG | 366 |
| cg04764584 | CGGAGATGAAATTAATGTGAACCACTGAGGCAAAGGCATGAGGCCCCCTC | 367 |
| cg15679283 | CGGCTGCCCCCTCACCAGCACCCTCCGATTCCAGCGACAGCCTCTTGGCT | 368 |
| cg17533522 | GTCCCTGGAGCCAGGCGTTAATCATTGACCTGATTAGCAAGAACGCTCCG | 369 |
| cg14868212 | CGACATGACAAGATTTAAGGGAGGCACATCTCACAGACAAGCATGAAAAC | 370 |
| cg16274199 | CTGGCCCGGCCGCCCCTCAGCTATTTGTTCACGTAATGCGATTGGAAACG | 371 |
| cg13309828 | TAAATGGACTTACTTTGAGGCCATGATCAACATTTAAATCTTGCTCTTCG | 372 |
| cg12612118 | CAGAGCCCACTTAGTGCGCGCTAGCTGGGCAGGGATAGGGGTCCTATTCG | 373 |
| cg03415429 | CGGACACTTCCGGTGGAAGGACTGAGCGGCGCTACACTTCAAGAATTCCG | 374 |
| cg15947697 | TTATTGTTTTCCTGTCTTCTACAGCAGAATTGGATATTCCCAAACAATCG | 375 |
| cg11867651 | CGGGGTCTGGGTTTTCTTGTCCCACTGGGAGTTTCAAGCCCCAGGTAGAA | 376 |
| cg17096289 | TCAAGGGAGGGCGATATTCCAGTGCTGATCCCATTTTCCTCCCCTCCTCG | 377 |
| cg01517384 | TCAGGAAAACTCATGCCATTCTCCATTCAACGGAGGGCGACATTCTAGCG | 378 |
| cg16928551 | CGTCCAGCAGACAAAGACTATGGTTTAATGAAAATAGATGAACCAAGCAC | 379 |
| cg26987613 | ACTGATGATTTCCAGGAACTACCAAAGCCACGGATCAGCTGAGTTACCCG | 380 |
| cg20660989 | CGCATTGTCAGCCTGGGGAGGCGACGCGGCGCGAGGACAGCG | 381 |
| cg21288889 | AACAACAAAAAACTTAAATTTCCCTTGACTTTTAAGTTCACTCTTGTTCG | 382 |
| cg06649410 | CGCAGAGGAGAATGTCAGATGCTCAGCTCGGTCCCCTCCGCCTGACGCTC | 383 |
| cg17501210 | CGCCCGATTCAGACAGCTGGACTCAGAGGGATTCTGCTCCACAGAGAAAC | 384 |
| cg02515033 | TCCCACCCCTTCCATTCTGTTGAGCTGAGACGCGTGTTTTGGTCGTTTCG | 385 |
| cg00907204 | CCTGATTTACACTTGGCTCCACCCATAAAGTTCCCACTGATTATCACACG | 386 |
| cg16847637 | GCCAGGAGTCCTCGGTCCTGGGAGGGTTTAAAAGCCAGGGGGCCGTCTCG | 387 |
| cg0347 3532 | CGTATGTGTTTGAGATAGCAGTTGTTTACTATCACTTGAAAATTCTGAAT | 388 |
| cg07205627 | CGACCATTTGTATGTGTATCTATGTCAGAAAGAATCTTTATTAAAATATT | 389 |

EP 3 864 177 B1

| cg identifier | DNA Sequence | Sequence ID Number |
|---|---|---|
| cg14508358 | CGGTCACGTGATACCCCCCAGTGTACCTTTCCTGCAGCGTGTTACGTGGC | 390 |
| cg04661709 | CTGGGGCTGAGACCCCATTTCTGGGAGGCTCTTCCCAGTGCTGGAGACCG | 391 |
| cg13428009 | TCTTTGAGCCCCAGCTTCCTCATGTGTAAAACAGGATAATAGCAGTGTCG | 392 |
| **cg21789941** | CTGCTGGGGAGCAGTGGACGCCCTCCAGGGTCTAGGGAGCAGATAAGACG | 393 |
| cg22866430 | GCACCCCCCCATCCACTTCATGTTCAGAAAACTCAAAGAGTCAGAAAACG | 394 |
| cg18234973 | AGCTCTGGAATCCAACTGCCTGAGTGGCAAACCTATCTGTAAGACTTACG | 395 |
| cg26110733 | GGGCCTTTAGTGCTGGCTCTTGAGTACCCAGACCCCAGTTAAGAAATACG | 396 |
| cg10636246 | CGACACCCTCAAGGGAGGAGTGCAGGCACTCAAAGATTTGAGTCACAGGC | 397 |
| cg10578779 | CGCAGAAGAAAGCTGCTCAAGTGCTGGAGTGGCCAACCCCATGCCGTTCC | 398 |
| cg17591574 | TGGCCTTAGGTTCTAGTGGCTCTGGTGTGGGCCTGTGCCCAGCCTTTACG | 399 |
| cg13371627 | TTTATAAACCTGCTATTCCTTTCCTTCGGATCCCTGGTTTTCCATCTCCG | 400 |
| cg12458003 | CTGGGCTTGACAGCTCAGAGCAGTGTCCCCAGCTGTGGCCCTAGAAGGCG | 401 |
| cg13180152 | TTTGCCTCTTTTTCAGTCTTGTAATCCCAAACAAAATAGAAGAGCCATCG | 402 |
| cg21459583 | AGAAGCACGTGGCACCTGTCATAGGCCCCTCTGGCTGCTACAAGTTCCCG | 403 |
| cg18278247 | CGGCACCTATCCCAGCAAGATCCCGGGAACAAAATCCTGGAAGACCCTAT | 404 |
| cg11295183 | CGCGTGAGTGTCCTAAGGAGTCCAAGCACAACAGATGGCTGAGTCACACT | 405 |
| cg18388639 | CGAGCCTGTTGTCAACATTAGCCCCGGGTTTCCCAGCACCAACTCCAGCG | 406 |
| cg06752157 | CGGAGCGTGAGCCTAGATTCTTTGGAAGGCTGTACAGTTTAAAATCTAAT | 407 |
| cg18148156 | CGGCAGGTTGGAAATCCAGTTTGTGGCTGATGCAAGCAAACCATGCTGCA | 408 |
| cg19935065 | CGGTTGTGAGGTGCTCACGTGTTTTGGAGATAGCAAAAGTCTCAAATAAT | 409 |
| cg10549018 | TGTCTCCTCATCTCCTGGATCTTTGCCCAGCAAAACCTCCAAAGAGACCG | 410 |
| cg07927953 | CGCACAGCCAGCAGGCGCCTGGGCTCCACTCTCTACCTGGAATCACCAGG | 411 |
| cg01057893 | CGGCAGTCTTCTCCACTGAGGGACTGGGCTGGGAAGTCCTGCGTTTCAGT | 412 |
| cg04431596 | CGGCCCCACCCGGTTCTTGTGTCAAGACAAAAAGAAAACCCAGGTGGCCT | 413 |
| cg06806711 | CGCTGATAGACATCAGGTGACAGGAAATCAGTAGCTTCTGCTACCTTGGG | 414 |
| cg13620770 | CGGCAAGCATCATCGCCAGGCCCCAGGCCTCCTGTGGGACGCCAGTCACC | 415 |

(continued)

| cg identifier | DNA Sequence | Sequence ID Number |
|---|---|---|
| cg03764506 | CACTTCCTGACTAAGGAGCTGCAGCGGATACATCGAAGGGCTCAAGAAGCG | 416 |
| cg07582047 | TCCAGGAAGCAGCAGAGGTCGCGCGCAGCTCACGGTTCTCTGCCTGCGAGACG | 417 |
| cg16649728 | CCAGCTTGAAGCCCGGTGCCAGATGCATAAGCCACTTGCCCGAGAGCACG | 418 |
| cg06854487 | CGTAGCCAGTGCGAGGTTGGCTCTACTTTGGGTTTATGGGTTGGGCATAA | 419 |
| cg25371036 | CGGGCAGCTCTACTGCTTGGAGAAGAGAAAGCTAGGTAACTGTACAGTTG | 420 |
| cg20941739 | CACAGCCAGAGCACTTACAGTGAGCCGAGCCACAGTCCAGCAAGCTGCCG | 421 |
| cg05021075 | CGACACTGTGATAATCATGAAGTTTTGTGTGAGAAAGATAAGGGAGAAGA | 422 |
| cg01975858 | ATAGCTATTCATCCTTAGACCCTGTCCAAACAGAGCAACAGTCATATACG | 423 |
| cg20923047 | TATTTTTGAAGGAGCTGGTCAGCATTTGCCTAGACTCTCGGGACAACACG | 424 |
| cg19783150 | CCCTCTAGTGAGACCCATTAGTGTATAAGCTCTGCCTACACCGAGTTTCG | 425 |
| cg01641177 | CGGGCCATGCCCTGGCCCCAGAAGTCGTTGGTGAAGATGCCCCAGCAGAGC | 426 |
| cg07677157 | CGGTGATCCATAGCAAAAGAGCCTATGAGTCAGGTGATGATCCATTCATC | 427 |
| cg21579274 | ACTACTTTGGGCCTCGGTTTCCCTGCTCCTTGTAGATCAGAGAAGGGACG | 428 |
| cg01687878 | CGGCCATGTCTGGTCACTGCGTATGAATGACATCTGTGTCTCTGCAACGCTT | 429 |
| cg18159533 | TGCCATTTAATCTAACGCCACCTTCTACGGACCCAGCAGGTGCTCTGCG | 430 |
| cg0432 3814 | CGCAGTGACAGGAATAATGCCACTGTCCTGCTGAGTGGGAAAGGAGAGGG | 431 |
| cg04836038 | AGAGGTCTCAGGAAAGTAGCCTTTATTTATGTGGCACCGATCGGAACCCG | 432 |
| cg16188984 | CCGCACAGCACGGAAAGGGAAGGGCAGCAGCCACATGGACCTGGGCCCACGG | 433 |
| cg11605750 | CGGCAACCCCGGCCCAGACAGGGGGTATCCAGAGGGCCCAGGACACCCACC | 434 |
| cg18607491 | AAGAACGACTCTCCGCAATGCGCATGACAGAGAAAAGCGGCTGCTTCTACCG | 435 |
| cg14870958 | TCGAACGCTGGACCCTTGAGACATGTGACGGGACCTGAGGGCAGCAGCCACCG | 436 |
| cg19491388 | CGGAGTTTCTGTTACCTGCAGGGAATGCTCTCTTAACTAATCCAGGGGTG | 437 |
| cg02135821 | CGGAGGCTGGGGGAGATGACATCAGAAAACCTGGTCCAAACTGCTCCAAAA | 438 |
| cg09279803 | CTTTACTCAATGCCAAGATCCACATTAACACAATTCCACTGAATCCTCCG | 439 |
| cg26481784 | CGGCGGACCCAGCTGACCAAGGAACTACAGAAGAGGACGAAGAAAGCGA | 440 |
| cg19459791 | TGGAGCAAATATAGGTCTAGACACGTAGGGCCTGTGAGGTTTTCCTGACG | 441 |

(continued)

| cg identifier | DNA Sequence | Sequence ID Number |
|---|---|---|
| cg16730765 | CTCTTGATGACCTCGTTGTCTGTCTGGCACCATGGGTCTCATCCTGACG | 442 |
| cg19987111 | CTCTCTCCCAGACCTCAGGTAAAATTAATGTTCTAGGAACGTATGCCACG | 443 |
| cg02880119 | AAGTGTCAACGAGATGACTTGCGCTACAGCGGCACACTGGTGAGGAACCG | 444 |
| cg22616007 | CGCATCTGCTTCCCATGAGTCTCTTGGGAAGACAAAGCTCACATTCTTC | 445 |
| cg06946797 | CTCAGGGATCTGTGAGCAGAGAGGCCTGGCTGCAAAGGGTCTGGAGGACG | 446 |
| cg26837962 | TGTGACAGCCCTGGGCTGAAAGACTCCCGGTGGCCGTAGTGTAATAGCCG | 447 |
| cg08659340 | CAACTGCTGGTCTGTTTTCTGCCCCTGTAGTTTTGTATGAATTCTTTACG | 448 |
| cg07984286 | CGCAATGTAAAATTATGTCCAGTTGTAGATGTGTGTGTTGGGGTTTAACT | 449 |
| cg00417823 | CGGGCTGGAATTGAGCTCAGCTAAACAAAGTCCTTCAGCTTTGAAGGGAG | 450 |
| cg05180206 | CGGACAAGATAATTATGATATAACAAAAGCTTGTGTTATTTAAGGTGGGG | 451 |
| cg09899215 | CGTCCCTGGAGCCCACGAAAATGTCTGCAAGATCTGCAAGGGCCACCTTA | 452 |
| cg04379563 | CGTCCCGACTTCACGCGAGGGTTTATACCTGAAACAGACACAGCAGGGAAA | 453 |
| cg09834822 | CGCCCTCTCTGTGTGTAGCTGGAAGAGCCTGACCACTCACGTCTCTCCAGGC | 454 |
| cg18951537 | AACAGCCATGATTCCTAAATATTAAAACATTTTCCTCATCTGACAGGATCG | 455 |
| cg23842572 | ACAGAGACAGAGCCCAAGAATAGAGGCACACGGGAAGTAGACAACATCG | 456 |
| cg25613227 | CGAGGGTAGTTGGTCAGAGTTGTGCGAGCTGGTTGCAGGGAAGGCTACCG | 457 |
| cg07660627 | CAAGCTCTGTGCAAAATAATCTGTAGTGTTGCTATAACGAGCTATAACG | 458 |
| cg22156456 | CGGGGATAGGCAGGTAAGTAGGATCAAGGAGTGCAAACAGCCAAGTGTGA | 459 |
| cg18181703 | CGTGGCCACTCTTCAGCATCTGTGTCGGAAGACCGTCAACGGCCACCTGG | 460 |
| cg25876186 | TCGGGGAAGCTCAGGGTCTCAGGGGCGGGGAGGCAGGACGACG | 461 |
| cg23691771 | CGGGCAGATCCCAAGGTTTCCTGATATTCCCTACTGCTGCAGTCTCTGCT | 462 |
| cg00870265 | GTCTTCATTGTTACTCAGCGGGGACACCCTCCTGGCGCTCCGGGGGCTCG | 463 |
| cg03739265 | CGCTGGAGACCAAAATCTTGGCTATGACCACCCTGGGCAACATAGTGAG | 464 |
| cg03636183 | TGGTGGTGGGGGCTGCCGGCCAATGGGCTGGCGCTGTGGGTGCTGGCCACG | 465 |
| cg02291424 | CGCGGCTTGACTAGTAAAGTGCTCAACTATTCTGACCACCATTGGGTAT | 466 |
| cg26853093 | ATGTGGCGCGGGGGCTTTGGACCCTGCGGCCCAAGCGGCACGTGGGCCTCG | 467 |

(continued)

| cg identifier | DNA Sequence | Sequence ID Number |
|---|---|---|
| cg03110633 | CGGCCGCCGCCCCCACCCGGACCCAACCGCCCGACAAAAGGCCGGAGCCG | 468 |
| cg07127225 | CGGGCTCGGTCCAGACTGACAACAATGCCCTCCTCGTACCTGAAGAACTT | 469 |
| cg06022832 | ACGAAGGACAGTGATCCCTAACAGTTCAGCAGTCCCTACTGTGGCAACCG | 470 |
| cg21566642 | CGCCTCCTGAGCCTGACTCTGCTCTTCCCGCAGCTTTGGCCCCCGGGGGC | 471 |
| cg01940273 | GCACGGGGCCTGGCACGTAGGGGGCCTTCATTGCATCTCTCTTCCCTTCG | 472 |
| cg08110610 | CGGGTGGGGACTAAACAAAAAGGTCAAGGAGAAATGTCAACCTCAAGATG | 473 |
| cg00684178 | CGCTGGGCTCTAGCTCTGTATCTGTGCTCCTGGCAGGCCAGCACCCCATC | 474 |
| cg24580066 | TCAAACTGCCGCGCGGCGTGCGGGTTGACGCCGAGTGTGTGTGTCGGGGCG | 475 |
| cg04588969 | TTCCAGTTCGTGCCATCAAGGAGGAGGCGACCTGAGCACCAAGTACGATGCG | 476 |
| cg21727276 | TGAGATCTCTGTCTCTATCCTATCCTGTCCCTGGCCTTCTGAGGCAAGCG | 477 |
| cg09051966 | CGGGGCGCAGCCCCAGGATGCAACAGCTCTCATACAATTGGCCAGGAAGGC | 478 |
| cg18345924 | TGCATACTGATTTCTGAACATTAAATCTGCATCCAATCGTGTTTCATCCG | 479 |
| cg18638931 | AAAACACCAACAAAAAACGACTCCTAACATCACTCTGGGTGGTGAAAGCG | 480 |
| cg27380813 | CGATCCGGAATTCAGGATGAGCCATGAAGTTATGGATGGAGGTTCGAGAT | 481 |
| cg03827386 | CGTTGTTGTTGTTTTAGTTACGTTTAGCTTTGTCAGCAACACTTTGGCCC | 482 |
| cg15849439 | CGAACCTGATGTGGAGACAAAGTATCCTTCATGGACTTTGAATTTGATAT | 483 |
| cg06349851 | CGGCCAGAGTCTTATCCCCTGGCTCTGCTGATGAGGAACACGTAGAATG | 484 |
| cg25598319 | CGCGGGGGGGTCCCCTGGCACCTCCAAGCCCATTGACGAAGTAAC | 485 |
| cg25845889 | CGAGAGGGCAGCTTTAAGACATTTAGAATTGTTTCTTACCATTGTCTTT | 486 |
| cg15228492 | CAAGTCCAGTTTTCCACCTAAATATCACTTGAGGCCAACATTCTCTTTCG | 487 |
| cg03900798 | CACCTGACAGACAAAGCCCTGGGTCCCAAAATGGCTCATCGGGGTCAGGCG | 488 |
| cg20576510 | CGCGGCTGTACCACACATAGAGAAAGATATGCACTAGTTCAAAGAGAATGCC | 489 |
| cg12988996 | TGTGCCTCCCTGGCAGTCAAAGTTCTAAGGGGGACTCCTGAAGCCAGGCG | 490 |
| cg11569478 | CGGACTCGTTTTATTGACTGACAGTCAGTGCTAGCAGTGACTTCAGCTGC | 491 |
| cg01603456 | GTCATAGAAGGCTGTGTTGCCCCCAGCAAGACAAAGCCACCGCTCCACG | 492 |
| cg18584747 | CGGCCGGAGGGCCCCTAGCCAGCCAAGACTCATCCACAACCTAGGGGG | 493 |

| cg identifier | DNA Sequence | Sequence ID Number |
|---|---|---|
| cg17543884 | GAAAACCCAGCAGAACATAACCGTGCAGGTCTCCGACGTCAATGACAACG | 494 |
| cg22090064 | CGCAAGAGAAGAAAAAAGGTAAGAGTTTAGTTCTCAAAAAGACAAAGAGT | 495 |
| cg11923914 | CGAAGGAAACCTTCGCTGGCTCCCTAGTGCCCTGGAATAACACTGAAACC | 496 |
| cg18394552 | TTTAGGCAGGTGGCCAATTTTGGTCCAGCTGGTGGCAAGGGCAGGTGTCG | 497 |
| cg24801535 | CGGCAGGTTTTGGGACTCATTCCTGCAAGCCCAAGCCAGAACTTGTTCTG | 498 |
| cg12325588 | CGCCATTCCCCAGCACCCGCAGCGGGCACTGTGGCTGCACGCCGCCCTCG | 499 |
| cg23367341 | CTGTCAGTAGATCTCAGCGGGAGAGCAGTGCTGGTATGTGAATGTCCTCG | 500 |
| cg04987004 | CAGTCCCAGCTTTCACTGCCAGGGTCCCAGTCAGATTCCAGGAATTTGCG | 501 |
| cg24859433 | CGCCCCTGGAGAGGCCTCTTGGGGAATGAACTGTCCCTCCCAACCTTGGA | 502 |
| cg10940833 | CGGCGGCAGGAACTATCAGTAGACAGCTGCTGCTTCCATGAAACGGAAAA | 503 |
| cg11539695 | CGGGCCCTGCAGTCCTTAATAAAAAATTGCAGATTTCATTCTAGGTGTCA | 504 |
| cg26987613 | ACTGATGATTTCCAGGAACTACCAAAGCCACGGATCAGCTGAGTTACCCG | 505 |
| cg17598713 | AGATCCTAGGTGAGGGCAGGTTGACTGGCAGGGCTGGTTTTTCGGGATCG | 506 |
| cg26433444 | AAGGCCTGGCTGGCTGCGAGCTCAGGAGGCCGCCTGAGGACTGCACACCG | 507 |
| cg25325512 | CGCGGCATTCAGCAGAACTCATGATGAAGGAAACAGTTCCATGGCAGGGT | 508 |
| cg02272457 | CGGCCCCACAAATTACGTGTGTGCAGGATTAAGGGAAACCAGCAAGGGGT | 509 |
| cg10169261 | CGCGCCTTTTATTGACGCAAACCTCCCGTTCTCGCGGGAACTTGGGAACG | 510 |
| cg03098356 | CGCCATCTTCACACCCCGCTCCGTCGCGTACGCACCTGGGTCACGTGATA | 511 |
| cg06649410 | CGCAGAGGAGAATGTCAGATGCTCAGCTCGGTCCCCTCCGCCTGACGCTC | 512 |
| cg15822010 | CGGCCCTGCACACGTGTCCACTGTGACGCGTCTGTCATGGTCCCCTCGGC | 513 |
| cg15089567 | CGGCTGGGGACCGTAGCAAGAGCAAGAGGGCTCCTCAGCAGTCCCTGTGG | 514 |
| cg00332048 | CGCCAGTATGGAAACACAGTGTGTAAAGCAAGCTTCGAGAGAGGAAAGAG | 515 |
| cg01391867 | CGCCTACAACTCTCTCGGTCAAACAACCGAAAACATCCCGTGCCGGAAGC | 516 |
| cg07113802 | GGTGCGCCCTGGGCAGCTGGGTGGCCTCAAGCCCTACTGGCGAGACTCCG | 517 |
| cg03473532 | CGTATGTGTTTGAGATAGCAGTTGTTTACTATCACTTGAAAATTCTGAAT | 518 |
| cg15573979 | GGATGCACTGCGAGCCTGCCAGGAATTCCAGGCCTCGCAGTGTTCTGGCG | 519 |

| cg identifier | DNA Sequence | Sequence ID Number |
|---|---|---|
| cg08202754 | CGCTTCCACATTTGGACGCCCCCACCCAGCGGCCTCCGTGGCACACACCG | 520 |
| cg14260002 | GGAGGACTTGGGTGTGTGGCAGGCGAGCAGCCAAGCCGGCAGATGACTACG | 521 |
| cg06563451 | GACCCTGGGCTTGGGGGGATTTTTTTGCCTGGAGGGCTCCTCTTCTCCG | 522 |
| cg03460603 | ACACAGAAATGGGGGTTCTCACACTACAGATTAAGGAGTGTCCCAGGCCG | 523 |
| cg25448355 | CGGGACAAGAAACAACATGTGAGCAGCACAGCCATTCAGTTGGGATGCTG | 524 |
| cg24493971 | GGCGGGCCTTGCCTCCAGCAGGGGTGTGAAGAGCCCAGGCTTATTTTCCG | 525 |
| cg02716826 | GGCCAGACACCCAGGACCCAAATAACCTAATAATGGCTCATTTGCCTCCG | 526 |
| cg14355192 | TCCTGCAGGCCTCACCATAGGGCACCTCTTTGGCCCAATTCAGGGCTCCG | 527 |
| cg13649864 | CTCTGAAGCCTGCACCTCTCCATTAAACACAGAACAGCTGCCAGAGGACG | 528 |
| cg23549061 | CGGCTCAGCTTGCTCACCCACCCCAGAAGGAGCGAGCGCATTTATGAAG | 529 |
| **cg13947317** | CGGCATTGGTCCTTTATTGAACATCCTCCCAAAGCTGGGGCTAGGGTTCA | 530 |
| cg21272576 | CGCTGCTGCCTGGGACTCAGTGATTATCCCATTTTGCAGACCAGAAAGAG | 531 |
| cg03522107 | AGCCGGACAAGTGAGACCAGGCGGCCCCTTGGGTGGGCAGGGTCTGTCCG | 532 |
| cg03259703 | CGGCTCAGCTTGCTCACCCACCCCAGAAGGAGCGAG CGCATTTATGAAG | 533 |
| cg05461666 | CGGGCAAGGAAAGGATACAGGTATCTGCCTTTCGCATGGCACTGAGCAAA | 534 |
| cg15860924 | CGGGACAAGCTTGACAAGGTGGGTGTCTTCCTGGACTATGACCAAGGCTT | 535 |
| cg10327168 | CGGTAGAGAATATTTTGACTCCTTGAGTAATGGGAGTTCTTTTGTGGTCT | 536 |
| cg24393673 | CGCGCTGCAGTCACGCCTCCCGCTGCCAGCCCGGCACCGGGATCTTAATC | 537 |
| cg23923856 | GGAGGAGGATCCCCGAGAGGGGCGCAAGTTGCGATGTCCGGAAGCCGACG | 538 |
| cg14476101 | CGCCAGCAGATACAAAGGCAGACAAAGTGAGCGAGGCAGTTTCCAGGACC | 539 |
| cg20569940 | CTTTGGCTCGAGCTCCAGGTGCAGATTTACGTTCATTTTCTTTTGTTCCG | 540 |
| cg10578779 | CGCAGAAGAAAGCTGCTCAAGTGCTGGAGTGGCCAACCCCATGCCGTTCC | 541 |
| cg05996213 | CGGGTGGAAGGAGAATGAGGAGGCAACCAAAATAAGATCAAAAAGTCAGG | 542 |
| cg03424844 | CGATGAGCAAAATCACACATCCTCATGTCCATCTGGTGCTCTGTATTGTG | 543 |
| cg19570545 | GCGGAGCCAGCGCTCTTGGGGGGCAGGGCTGCGAGTCCCCTGTGTGGGACG | 544 |
| cg20248954 | CGTGACCTGTCTGTACCCTAGCGCGGGGCTTCTGTACGCTGCTAGGAAGC | 545 |

| cg identifier | DNA Sequence | Sequence ID Number |
|---|---|---|
| cg08138571 | GAAGCAGTTAAGACAACAATACCCAGGACTAGCCAGATCCCGAATTACCG | 546 |
| cg23814988 | CGTCGGGCTCACAGAAGGCTCAGGTTTCCTCCAGTATAGTTCAGGGGATG | 547 |
| cg20801751 | CGCCTCATACAAGAACACAGAACAGGGAGCTTGTCTCCTTGGGGATTAGA | 548 |
| cg11333189 | CGCTGCGGCAAACCTAGGAGGGACCCAAAAGCAGAAAATCCTAGAAAAGA | 549 |
| cg08055490 | CGGAAACTGATCTAGTCAGGGAGGTCAGAGAATATCTCCCTGAGGAAAAG | 550 |
| cg02613108 | CGCACTGACTGGTGAGCGCTTTGCACCAATTAGTAGCAGGTGCTACAGCC | 551 |
| cg12329405 | CTCCCTACGTGGGGGACGTGCAGGATGATGCGGGGTCGGGGGGGATTTCG | 552 |
| cg20245361 | CGGGGTGCATTAGCTGTGACTTTTGCTGCATCCAGTCATGCACATGTGCC | 553 |
| cg07793207 | GCTTTCTGCTTTCACCGGGAGGCCCTTTAATTGTTACCAAAAATAGGACG | 554 |
| cg07733920 | AGGTACTGGATGCACGGCAGGCCCTGCCACTGCTTCTCAGAACCCTCTCG | 555 |
| cg02415341 | AATTCTTTCACAGTTCTGGAGTCAAGAAGTCCAAAATGAAGGTGTTCACG | 556 |
| cg12893697 | CGGCCCTACACGGGCACCGGGCCTCAGTCCTGCCAGGCCCACGCCATCCT | 557 |
| cg18886347 | CGGTGTGGAGGCTTTTTTTGTCAGAAGCTCTGGGGTTTGGCTCTTCTCTG | 558 |
| cg19083871 | GAAGCTGGCGACGAACCCGTGTCTCAAGAATTTCTACGGCTTGTTTCCCG | 559 |
| cg13875901 | CGGCTGCAGGCCCCAGAGTCCACGAGGGTGGGTACAAATCCCCTGGGCAC | 560 |
| cg02970861 | TGATTGGCCCAAAGCATCGTGAATATTCATGACAGCAGCCACAAAGGCCG | 561 |
| cg26967875 | TTCTCGGGTCTTCGTGAGCATTAAGTCAATGAACCCAGGGCACCAAAGCG | 562 |
| cg00574958 | AGGCCAGCGAACATCCAGCTGTCAGTTGGTCTGGGGACTATCAGCATTCG | 563 |
| cg22355889 | GAAGATGGTGAACCTGAGATGACCTGTCAATGGGATTCCCAGACAGTCCG | 564 |
| cg05839709 | TGGATAAAGGGGTTTCTAATACCCAGCTCACATGGCTGTGGTGACAAACG | 565 |
| cg19061798 | GTGAACACCTGCCGAAGCCTTTGCCTTGGGAGAGGCTCCAGCAAAATCCG | 566 |
| cg11932091 | CGGGCACTGACTGGGGACCCAGGTGAGTCTAGCAAATGACAAGGCCCAAC | 567 |
| cg26527903 | CGGCAAGGTTTATAACGAGAATAAATGAAATAAATACAAAATCCAGAATA | 568 |
| cg24420742 | CGGGGACTTGATATGGAAAGAATTAATGTACTGGCTTTTTTGTATAGATG | 569 |
| cg10948795 | CGGGTAGGGTTAGGCTCAGTTCAGGCAGAACCTGCCATTCGCTCATTCAC | 570 |
| cg17117459 | ATTCATCTCTAGTCTCTGTAAAATGCCAGCCCTCTTCTGGGTTAACAGCG | 571 |

| cg identifier | DNA Sequence | Sequence ID Number |
|---|---|---|
| cg18331061 | CGCACCGTCTCACCTGAGTTTTCTGCCACTGGATCATGTGGGTCTGGTTG | 572 |
| cg21879240 | GAGTGTTCCCCATGCTGACTTCTGCCAGGCTGCAGGGAATAAAGCTAACG | 573 |
| cg20656525 | CGGAAAGGTTCATTGGCTAATTTGCCACAGTTGGTTTTCAATATGTCCAT | 574 |
| cg14543641 | CTGTGCAGACGCCATTTGCCCCCAGCTTAATTGAGCATATCTCTCCCTCG | 575 |
| cg04634427 | CGGTGGGAACGGGAAGCTTCCCCAATCAGACTGCATCCTGAGGGACAGGA | 576 |
| cg01624571 | CGTGGATCTAGCCACAGAAAGGCAACAGCGCTTCAAAGAGGCTCTCCGTA | 577 |
| cg05575505 | CGACTCTGAGTTCTGACAGGGAAATACAATGAGTTTTATGTGGACAGAGT | 578 |
| cg06051159 | CGGCCTTTGTGGATGCCAACCTTTGTCACAGCCAGGAGGGGGCCTCCCTC | 579 |
| cg00994306 | CGGCCTCAGGCCAAGCATGAGAACAACGTCAGACAAATCCCCATTGATTG | 580 |
| cg02295856 | CGCCGACCCACAGGGCACAATGGTCTACAAAGTTGGAAATGAAGGTCTGC | 581 |
| cg12991522 | GCACGGGCAGCTGAGCTGGGGGTACAGGGCACCCCCGTGTCCCAGGGTCG | 582 |
| cg00036119 | CAGGAAACTCCTACCTCATCACGGCAGCTGCATGTGTGGGTAAGCCCACG | 583 |
|  | CGGCAGTTCTTAGTGAGGGCAATTCATTCAATCCAGAGCCAGAAACAGGA | 584 |
| cg06722193 | CGGCCACCTCAGACTTCTGTATAGGAGGAGGCCTGACTGGGAGATCCCTG | 585 |
| cg05782444 | TGTCCCAGGATGACATCAAGGGCATTCAGGAGCTCTATGGTAAACCTCCG | 586 |
| cg26943759 | TATGGACAGTCTTTTGCCTGGCGTGGGGGCCAAGTGGACAATACCACACG | 587 |
| cg05737638 | CTCCCTCCACGGGACCCAGCCTGCAGAAGGGTCCTGCAGGAAGTGGCACG | 588 |
| cg21649005 | GAGTTTCTACTCTGCCTACAGGTCTCGGGTGAATTCACACAAACTCTTCG | 589 |
| cg06419850 | CAAGATCCAGGGACCTAGAGGCCTCGGCGATGACACTGCGCTGAACGACG | 590 |
| cg19761014 | CGCCAATGGTTCCAGCAAAGCCCCAGGACTGCTCTGCTCCTGGTCTTGTA | 591 |
| cg05174890 | TCAGAGCTGTTTTCGTGGGTTTTTAACTTCCAGTTGCTCAAGGGTCCACG | 592 |
| cg10431713 | CGGCTCCACACTTCCCCAGCGTGGCCTGCATTTCTATTGGGCAGCTGGCG | 593 |
| cg02483931 | TATGAGAGGCCAGAATATTGCTTCCGTTCCCTTTTCATCTCTGCACACCG | 594 |
| cg07477034 | CGCCCTGATAGCACCGCAGAGGTTCTGATCTAATTGATCTGGGATGCGGC | 595 |
| cg15815084 | ATATACAGTGGCCATGAAGCAGGCACCACTTAGAAACCTCTGTATTAACG | 596 |
| cg12054453 | CGGCAGAAGCTTCACCACAAAAGCGAAATGGGCACACCACAGGTAAGACT | 597 |

| cg identifier | DNA Sequence | Sequence ID Number |
|---|---|---|
| cg03568017 | CGGCTAGAGACTCACTGACTCATGCGTGTGTGGTAGGAATCTTCCAGGAA | 598 |
| cg02633924 | CGGGACGGGGACTTCCTGGAAGATTCCTACCACACACGCATGAGTCAGTG | 599 |
| cg18181703 | CGTGGCCACTCTTCAGCATCTCTGTCGGAAGACCGTCAACGGCCACCTGG | 600 |
| cg27637521 | GGAAACTTGCTGTGGGTGACCATGGCGCACGGAGCCAGCGTGGATCTGCG | 601 |
| cg05420896 | GGGTGGTACAAGGGGCGCCCCTTCGCTGTTTTAAAATCTGATGCTTCACG | 602 |
| cg19283806 | GATTTCTCCTTGAACAATCCCCGCAAAGATAGCAGCCAAAAAAGGATGCG | 603 |
| cg27569863 | GAAAGCACTAAATTATCCTGCAAACTCCCGGCTGATGCCTAAGGAATTCG | 604 |
| cg13611456 | CGGAAGCACAGTTTGCAACAGTTTGGGGTTGTCTTATTTTCACTGCTGGC | 605 |
| cg10677351 | CGCACGGATGGGACCTGCTTCTCACTCCTGCACAGCTGAACCTCCAGGAC | 606 |
| cg15011409 | CCAGGAGTGCGCTGCTCTCGGGAAGGCATCCCATGGCCTGAGCAGCAGCG | 607 |
| cg17676631 | GCTGGGTCCCCGTGTAGCACTTAGCTAAAGATGATCAGCAGCTGAGAACG | 608 |
| cg11594160 | ACAGTATACAGTCGGTGCTCAGGATGCATTTGTTTTCCTGTCCCCCAGCG | 609 |
| cg26950531 | TGCCGGCCACGTGGTTGCTGGGCAGGTGTGGGGTCAGGCTGTGGATCCCG | 610 |
| cg22538557 | CGGCTCAGCCAACCCCCTAGATAATAGCGGTTTCTCAAACTGTGGTCCCG | 611 |
| cg09920725 | CGGTCTTGTAACAATTGGATGGATGCCTTTGAAGAGCCCCTGTCCCTATT | 612 |
| cg10354495 | CCTATTCTTCCAGAACCTTCCTGGAGTATTCCGTTTTCTCTAGGACAACG | 613 |
| cg04659537 | CGGGGGAATTCATTGATAAGGAGAGCAAGCATCCCAAGAACATCCCCATC | 614 |
| cg14088844 | CGGACACAGCACCAGGTCCAGATTGGCAAGAAAGGACCCTGGGCTTGCTC | 615 |
| cg07258 300 | ATCTTCAAGTCTCACTTTGGTCCTCAGTTTGTAGTATCTATTGCAGACCG | 616 |
| cg26191447 | CGGTACCAGTAACTGCCAGGAAAAGGAAGGGAATATGTCCAGAAAAAGCA | 617 |
| cg02831419 | TGGTTATTCCCCAGGAGCCGCGCGAAGCATGAGCTAATTTTCAGTGAGCG | 618 |
| cg20550050 | CGCACATGAGGCACAGAGAAAGGACAAAACGCAGCCACACCTGAGCTGCG | 619 |
| cg06266189 | GTGCCTCCCTTCTGCACTGTGGGTGTGTGGAGCTGTCTTTCTTGGAGACG | 620 |
| cg12791136 | TGAGCAGGAGCGAGACAGAGCACCACGGGGCACGTGAGGCCAGGGCACCG | 621 |
| cg23278885 | TCCCACAGTGAACCCCTCTAGGTGCAATTACAGGATGATTTTGTGTCTCG | 622 |
| cg08074820 | GCCCCAGGACCCCGGGATGTGCAGCCTAGGGGTGGGGTTGAGGTTCTGCG | 623 |

(continued)

| cg identifier | DNA Sequence | Sequence ID Number |
|---|---|---|
| cg11086127 | ACCTCTTGTGGTATTCCTCTATCAGGCAATCTGGGTGGGCAGAGACTTCG | 624 |
| cg00412842 | AGTCTTAGCTTAAGGAAATGTGCCACCTGCGTGCAGGGATGAGGTCATCG | 625 |
| cg27294156 | CCATGCTGCGGATCCTGTGCCTGGCACTCTGCAGCCTGCTGACTGGCACG | 626 |
| cg22163406 | TTCATCGCAATGGGCTGTATAGCTTGGCCCGTCTCATTGTAACAAATTCG | 627 |
| cg08334432 | TGGGCTAGCCCAGCGCCACCTTCCTGGAGACTGTCTGGTGGAAGACACCG | 628 |
| cg06133392 | CGCTGCTGAATGCACAGCACTGTGAATGCACTTCCATCACTGAATATACA | 629 |
| cg23573129 | CGGGGCTGAGTACTTGGCTGACCACCCTAGGGCCACACCAGCCCAGGGGA | 630 |
| cg20585830 | CGCACAATACGTGGAAGGCCATGGAAGGCATCTTCATCAAGCCTAGTGTG | 631 |
| cg19253577 | AAGCACTTGATGTTTGCCCTCACACTGTTCCTCTCCCATTCCAGTTGTCG | 632 |
| cg09219182 | GGCCTTACACCAGTGCAGAGGGCAGGCTTGAGGCTGATCTGGTACACACG | 633 |
| cg01708387 | TAGAATAGTGACTGGCACATAATAGGTGTTCAATAAATGTCAACCCACCG | 634 |
| cg12918464 | CGCCTCAGCTGGTGCTCCAAGACCTGCTGGCCAGACTGGTGAGGCCAGGT | 635 |
| cg14508777 | CGGGGACAGGGCTCCTAATGTGTTTGTCAAGTAAATAATGAGTAATAATC | 636 |
| cg05993589 | CGGTGAGGCCAGTGCACTCAAGTTTGGGAACTACCAATATAGAGTAAAAG | 637 |
| cg23431721 | ACATACGTCACATTGGCAGTGTTTGTAGGATATCGGGCTGTAGGGATCCG | 638 |
| cg07105440 | GCTAGCTGCCGGGCATTGGGAGGGCTTGACGGGCCAGAGGTGACTGAGCG | 639 |
| cg03929796 | CGGCACCTCGCCTCGACCTTGCCATTTTATACTCAATTGGGGCGTAGGGT | 640 |
| cg23511118 | GCAGGTCATTACTATGGGTAACTGGGGCTCCATCTCTCTGAAATACCACG | 641 |
| cg13155421 | CCCTGGCTGGTGTTTCATGTCCACCAGCTCTGCGTTCAGCACAGCACTCG | 642 |
| cg03840504 | CGTCTGTCCCAAAGGGCGACACAATCCTGAGGCAGCTGTTCGCAAACTCG | 643 |
| cg19389001 | CGGCGCACCTCTCCAGTGGGACCCGCAGGACGCCGTCTCTCAGCCCCACG | 644 |
| cg15709989 | TAAGGCCCTTCAGAAATAGGATCGACAGGGCTGGAGTGGAGGGGCCCCCG | 645 |
| cg13296371 | CTAGCATGGTGCCTAACAGCTCCTACCAAATAGTAGATGCTCAGTAAACG | 646 |
| cg21761813 | CGGTCAGTCTGCCTACAGAGTGCTTGCCAGGCAATGAAGGAAGAGCATGC | 647 |
| cg00398048 | CGTGCGAACATGAACTAATAATCCCI I IGGGTGTCTGGAATGCCTGCCCA | 648 |
| cg25149516 | CGCCCACGCGGACCCCTCTCCGTGTGCTTGTCTGTACTTCCCTGATGGGG | 649 |

| cg identifier | DNA Sequence | Sequence ID Number |
|---|---|---|
| cg10177080 | CGGTACTTCCCTGATGGGGCCCACGCGGACCCCTCTCCAGTGTGCTTGTC | 650 |
| cg09578605 | CAACACTGTGAAACCCTGTCTCTACTAAAATACAAAATATTAGTCAGTCG | 651 |
| cg01817885 | CGGTAGGGTTTGGAGGTATTATTCAGTGTAGAGGAAACAGTATGCATAAA | 652 |
| cg12980795 | CGCTGGCAAGATGAATCACAATAACAACTCTGAAGGCAAGAGGATTGGTG | 653 |
| cg01256539 | CGGCAGAATTGTGCTTAATAGTTGGTGAGGI I ICTATGGGCGATGTGGAA | 654 |
| cg19076659 | CTTCCTTCAGGGAGTCGTATATATGAGAGGGCTTTAGCTTGCTCTCCTCG | 655 |
| cg05182912 | CGGGACACGTACTATCTGCCTCAGCCTAGTGCTAAGCACTTTACACATTT | 656 |
| cg00543335 | CGGGGCTCATGGCⲄAAAGTTAAGGTACTGATCAAAGTTTTGGATGTAAAT | 657 |
| cg21584251 | CGCTCCAGGCAGCGCTCCACTTCCGCGGCTTCGCCCTTGACAGCGCCCTG | 658 |
| cg00558975 | GGAGGGCATCTTATTTTACCAGGTAAACAAGGGTCACTAAGAGGCATGCG | 659 |
| cg01187498 | TTCACACTTAGGGCTCAGGACGCCCCTGTAGGCAGGGTGCTGGTGGGACG | 660 |
| cg22983827 | CGCAAAAGCAAGATTCGCTCCAGGAGTTTCATGCCACAGGATGGTCGACG | 661 |
| cg02782857 | GCTCATCACCTGGGGGCAGCTTTTGTACCTGCACCCTGTATGAGGCTCCG | 662 |
| cg17286012 | GGTCTCTGGCATCTGTCCCGTCTCCAGCACAAACAACATCTGGGCAATCG | 663 |
| cg02066343 | GCTGAGGGTGCCAGGTTGTCCTCCAAGGCCATCAGAAAGAAGGCACCACG | 664 |
| cg25325512 | CGCGGCATTCAGCAGAACTCATGATGAAGGAAACAGTTCCATGGCAGGGT | 665 |
| cg08373528 | CAGCCTCATGAACTCCATGGGTGTCGTCACGCTCCTCATTTGGCTCTTCG | 666 |
| cg15290848 | CGTTCAGTGCCACTCAGGCCTGGCTGCGAAGATACAGATGGTATATGTGA | 667 |
| cg08877853 | GGCAGTGTTGACTGCGTTCCATACCGGGACATCCAACACAACATTTGTCG | 668 |
| cg01883195 | TGATTCTGGACCTCAGCTCTTCAACAAAGCTGAGGTCCAGAATCAAACCG | 669 |
| cg00936722 | AGCTTTCTGGGTCTCCTTAATGAGCCTGAGGCCTGCTGCCTGCAAGTGCG | 670 |
| cg00554421 | CCTGAGGCTCTGGGAGCAGATGGGAGCACAGGCTCTGTTTTGGCCCTGCG | 671 |
| cg11287647 | TGCAATGATAAGTTTGCTGAACTGCATCTCTGCCCAATGCCACTTCCTCG | 672 |
| cg13917964 | CCCATGCAGGAGAAACCCGTTTGGTGTTTACTACTAAGTCATGATCTCCG | 673 |
| cg00277397 | CGCTGTGTGTGTTGAGACACACGCAACATTCGTGCACATAGAACTCATGA | 674 |
| cg20352798 | CGGTGCCCCACAGGCCACTGTGCAGGATCTCCACTCGGCCCTGACACAGG | 675 |

EP 3 864 177 B1

56

| cg identifier | DNA Sequence | Sequence ID Number |
|---|---|---|
| cg10119082 | CGGGTCCCCAGTCCTCAAGGTCCCAGCACAATGCCCCAGGAGGAAGACTC | 676 |
| cg17981101 | CGCCCCAAGTCATGAGGGAAGCTTCCAGACTGACTCAGGCTCAGGCTGGC | 677 |
| cg03494277 | CGGTGACTGCAGGCTCCTGGATCCCATGGAGCAGAAACCTGAACTTGTCT | 678 |
| cg09315887 | TGGCTACCGTGCTGTGGGGTGCCTCGAAAGTGTGAATCACCAAATATTCG | 679 |
| cg04599158 | TGTTCAATGTAATAAACAACTGGCTCTTTGCTGCTTCAGATCTGTGCTCG | 680 |
| cg25149516 | TTATCAAAGACTTTATATTTTCTGTAAAATACTACACAGTAAATCTTCCG | 681 |
| cg01026009 | CTACCCTACTGGAAAATAGTGTTAATTATTATTCAGTTCTATTTCCATCG | 682 |
| cg03276920 | TCTTCTTTCCACAGGTTCTCAGGCAAGAGCCACCTGCTATTGCCGAACCG | 683 |
| cg13398470 | CGTGGCTATGCTGTGGTCTCCCTGAGCCTACTCTAGCTGGAAAGGTAATT | 684 |
| cg07205627 | CGACCATTTGTATGTGTATCTATGTCAGAAAGAATCTTTATTAAAATATT | 685 |
| cg05065162 | CGACACTGCACATCACCGAGACCAGGCACAGTGCCGGTTATGCCTAAGCA | 686 |
| cg12396523 | CTCCCGAGTTCGCCCAACTTACTACTCTGCATGCTGGTGCCTAGTCTGCG | 687 |
| cg12831076 | CAACGGAACGAAGCTGCATCTAAGTCTTATTCTAGAATTCTTTGTGACCG | 688 |
| cg22902266 | CGGACAATTGGGGCCAAACGAGGAAGGACACAGACCCAAAAGCCAGACCC | 689 |
| cg04661709 | CTGGGGCTGAGACCCCATTTCTGGGAGGCTCTTCCCAGTGCTGGAGACCG | 690 |
| cg01355108 | AACAATTATGGGCTTAAAGAATTGATCATTACAGCCCCTGGGATTTAGCG | 691 |
| cg26581729 | CGCCATAAGTGCCCCCAGAACTTCAGCGCCCACCATGGCGCACAAGGCCG | 692 |
| cg18476633 | CGGTGAGTCATGCTTCAGGTTTGCCAGGGCCTGTGTCCCGCAAAAAGGCG | 693 |
| cg26341773 | CGCTGGGAGGCACCACTTGACATGACCTGGGACAGGTGGCTCAGTCTCTC | 694 |
| cg25034591 | CTCTGCATCCTCCCCAAGCAAAACCTCACCAAGGTGGTGAGATCCTGACG | 695 |
| cg03102848 | CGGGGGCGCCGCCTAGAAACACACAATGGCCCCTCCTGGCCAACCCCCCG | 696 |
| cg18154457 | CGCGTCATAAAGGACAAGCAGACCCAACTGAACCGCGGCTTTGCCTTCAT | 697 |
| cg11924796 | CAACCTCAGGTTCCTTTTAATGGAGTAGTCACTTATTCATTCAACATACG | 698 |
| cg08871010 | ACAGTCATACTGTCCTGAGTAAGGACAGTCAACAAAGGATCATTTCTCCG | 699 |
| cg06780601 | CGCGTAACACTGTGCCTGGCTTTGCAAATATATTTTACAGATAGTGTAGT | 700 |
| cg27395754 | CTGACCAAGGCATCACAATAAATACAGTTAGCAGCACACAGAAGCCTGCG | 701 |

(continued)

| cg identifier | DNA Sequence | Sequence ID Number |
|---|---|---|
| cg02587153 | CGACATTAAAAGACCAATGGAGAATGTATATTAGCAATCTGTAAGGCAGT | 702 |
| cg15192905 | CGCCGCCATCTCAGGTCTCTTGGCTTTGCCAGGGCCCACCGGAGAAAACT | 703 |
| cg08352336 | GTCAGTTTTCTCCGGTGGGCCCTGGCAAAGCCAAGAGACCTGAGATGGCG | 704 |
| cg01997410 | GAGAGGCGCAGGCGAGCTGGAGGGGAGCGGCGCGCGGCGGCCAGTGGGCG | 705 |
| cg16730484 | GAGAGTTCAAAGTGAGCTTACTTCCAGTCTCACGTACAGATGATTCTACG | 706 |
| cg22686892 | CGGGATGGAAGTTAGCGAATTTGGGCAACTTGCCTCTACTGCCCAGTCGA | 707 |
| cg14073590 | CGTGGGCGAGTTGGTGGCAAAATCTGAACTGGATACTCACGGAGGGCCCG | 708 |
| cg07989867 | CTTTGGTGCAGAACCACCGCATCCACTTTCTCAGGCCCAGGGGAGGGGCG | 709 |
| cg21080294 | AAGCAGGCATGGTTGATTTCCCGCTAGCGGGAACGATCTGGCCTCAGACG | 710 |
| cg14680768 | CGGCAGAGAGTTGCCACTGCAACGCCTGGCTCCCAGCCCTCCCACCCAGC | 711 |
| cg10142252 | CGCCATCTCAGCCCTGGTGAGCCCCAAATCTGAGAGGGAGGGAAGGTCCT | 712 |
| cg18717447 | CGCCCCCAGCTGCCAGTCCCCAGCAGCTCAGTCCTGCAGTGAGAGTCTTG | 713 |
| cg03960747 | CGGAGGCCAGGCCACGCAGAACCTCACAGACCAAAGCTGCTTCGCAGTCG | 71.4 |
| cg16832801 | CCTGCGGGACTACCAGAAGGTGCTAGTGCAGCTGGAGAGCCTGGAGACCG | 715 |
| **cg11781622** | CGGCCGGGACTCTCCATGTCCTATGGACTCTCGCTGAAGCCACTCGGCCC | 716 |
| cg13947317 | CGGCATTGGTCCTTTATTGAACATCCTCCCAAAGCTGGGGCTAGGGTTCA | 717 |
| cg24145109 | GCGGTGTGCACTGTGACCCGCCTTCCCGGGAAAGTCAGAGCACAGAAGCG | 718 |
| cg19694465 | CGCCCCTGCCTGCTCGCTGCACTCCAGGTCACAGTTGGCACAGCGCACCG | 719 |
| CG15211499 | GAGGAGTGGGTGGGAGACACGCGTGTCCATGCCGCGAGGGTGTGTCGACG | 720 |
| cg10511249 | CGCCCTCGGAGCCCCTGCAGTCCCCTCACCGGGCGCTCCCAAACAGCCCG | 721 |
| cg16383389 | GCCGAATTTCTGAGGTATCCGGGCTCTGGTGGACTGAGGGGGGCGACGCG | 722 |
| cg04607246 | CGGGGAGGGGGTAGTTGTACTCTGCTTGTACAGTCCTTGAGCCCAGTTTA | 723 |
| cg08926056 | TCCCACTTTTGTGAACCTTGAGTTTTTGAAACTTTCACTGAAAAAAACCG | 724 |
| cg14652203 | CGCAGGAGGAGTTGGGAGGACAAAAGGAGAAAATGAACTAGTTGGGTTAC | 725 |
| cg20248954 | CGTGACCTGTCTGTACCCTAGCGCGGGGCTTCTGTACGCTGCTAGGAAGC | 726 |
| ·cg23814988 | CGTCGGGCTCACAGAAGGCTCAGGTTTCCTCCAGTATAGTTCAGGGGATG | 727 |

| cg identifier | DNA Sequence | Sequence ID Number |
|---|---|---|
| cg14626196 | TCTTTTAACCCAGCTAACACTTCTGTTAATAGTGTCTTCAGCCAGATGCG | 728 |
| cg16704703 | GGGGCAGGACATCTCCCAGACAGCTGGGGACACCCTTTCCAGCGGGAGCG | 729 |
| cg19328485 | CGGTGGGGACACAGAACCAAACCATATCGTTTGGTATTCCAGAATTTCTG | 730 |
| cg04254609 | TCTTTGCAACGTCCCTCAAGCACTTAAGGAGAGGCGGCAGGGCCCAAGCG | 731 |
| cg02974659 | AGCAGGACAGGCACCCGCTGGCCGGGAGGTCATTGACTGGCAGAACGACG | 732 |
| cg19795482 | GGAGGAGGACTTGAATACACGCGTCGCTGACTCAAAGATGGTCCCCTCCG | 733 |
| cg17133967 | CCCGGAGCAGTGGGTTCCTCTGCCGGGCCAGGAGGGTGATGGAGGGACCG | 734 |
| cg17639894 | CGCGCCGGCTGACGTCACCCTGTGTCGACCAGCCCCCTGAGCCCGGAAAG | 735 |
| cg13657511 | GAAGCACAAAGGCGACGTGACTCCTAGAATCTGTCCAGAACCCATGGTCG | 736 |
| cg26411299 | GTGACAGCTGAGCTGAGACCTCAAGACACAGTCACCTGGGCCACCTTCCG | 737 |
| cg05425050 | CTTATGCAAATCACTTAGGTACATGCAAAAGTATCCCTTCTCCCGGAGCG | 738 |
| cg11058730 | CGCGCCGCATCTCCGGGAACAACAGTCTCCCTCCCGAGCATCACAGCCAG | 739 |
| cg05590616 | CGCTCCTCCTAGCTAGCGGCCGCCGCCCGCCGCCGCCTGCGCCTCCAGCT | 740 |
| cg07268726 | CGGCCCCAGCCTGCCCAGGGCCCAGCTGCTGGAGACCCGCAGCTCGTCCC | 741 |
| cg02780017 | CGGAGGGCTCAGGTTACACCCCATAGGAGGGTTCAGGTGTCCCCACATCT | 742 |
| cg05710301 | CTCCAGCTGGCGCACTACAAGGTAAGAACTGACCGCCTAGGGTTTTGTCG | 743 |
| cg00574958 | AGGCCAGCGAACATCCAGCTGTCAGTTGGTCTGGGGACTATCAGCATTCG | 744 |
| cg02580722 | CGCGCAGTTAAAAAAGAACTCTTACTTTGGTTGGTTGGAAAAGAGCTGAT | 745 |
| cg11507178 | GTCAGAGTTCGGGTGTCTGTGGGTCTCTGAGCCTCTGCTGGCAGCACCCG | 746 |
| cg08832227 | CGCAGCTCAAGACCCTGGCGCAGTTCCCCAACACGCTGCTGGGCAACCCT | 747 |
| cg22047262 | CGGCTGGCAGGGGCCCTGTGCCCCCAGGTACCCCGTACCCCTGCCAAAGT | 748 |
| cg11043909 | CGCAAGACTAGGCAACCTCCAGCCAGTCCCTGGGTCGGGCGGATCCTCCC | 749 |
| cg06705834 | AACATGAGACAACTGAAGTGCCCTGGTTTATGTGCCCTGCTCCTCCTCCG | 750 |
| cg17177219 | ATTTCACAGATAAGGAAACTGAGGCCCAAGATGGCACAGTGGCCTCGACG | 751 |
| cg24805559 | GAGACATGGCGACGGCGAAGGTGGCAGCCTGGAGCCTTGGGCTGAGGTCG | 752 |
| cg10323552 | CGCCCAGCCCAGATTTTGGCAGTGCCGAAGCTCCACACTTTCCTAAGCAG | 753 |

| cg identifier | DNA Sequence | Sequence ID Number |
|---|---|---|
| cg13649020 | CGCCACCATCGAGTGTGAGCAGCCCCAGCCCGACCTCTACAAGTAAGCGG | 754 |
| cg01936220 | ATTGTCTCCCAATTTCTTCGTCTGTAAAATGGGGCTGGCAGCAGTGCCCG | 755 |
| cg09225388 | CAGCCACTACTCCAGACTTTGTTCCTACAACTGACGATTGCCAGAATCCG | 756 |
| cg05241355 | CGGATCCGTTTGAAACCTGTCCGGTCCCTGCTTTCTATGCCAGGCAAAAA | 757 |
| cg21112391 | CGTGGGGTAGGGACTTTCATCAGGACATAGAGTGGCAGTCATGTGCTTCG | 758 |
| cg12155356 | AGTGTCCGAGTAAGCCTAATGACACACATTCACATAACTAAGAAGACACG | 759 |
| cg08140104 | AGTCCACGTGTCAGAGGTGTTTGAACCAGAGCAACCCTATCTTAAATACG | 760 |
| cg01823956 | ACTTTCTCCTTCTTGTTTTTATTCGGCATGGCTGGAGCGGGCCGCCGCCG | 761 |
| cg10439456 | CGCAGAGAGATGTACCAACACTGTGCTCCTGAGTGACTTATAAAGAGCCA | 762 |
| cg21791657 | TTTAAGTTAAAATTGCCCAGACGTGGTATTCTGAATCTGGGTGATTGGCG | 763 |
| cg19510604 | CGCTCCTGGTTGTCACATTCTGGCTCTTTTATGGGGTGGGAAATCTTTCA | 764 |
| cg07120314 | CGCTGCCAGGAGGAGCCGAGGCGAGTGGGCGTGGCTCTGGCGAGTGCCCG | 765 |
| cg10530344 | CGCCAGCCCCAAGCAAGCCAGGCAGTGTCCTGGTGTGGCAGCTCACAGGC | 766 |
| cg21658515 | TCATCCAGGCTGCAGTGCAATGGAGCGATCTTGGCTTACCATAACTTCCG | 767 |
| cg16708495 | GTGGTGGAGATGCCTGACATGGCTTGGGGACCTGCTGCCGGGGGAGCTCG | 768 |
| cg03997502 | GTGCAGGGGGAGCTGGCGCAGCTCAAGGCCTGGGTGAGGAAGCTGCAGCG | 769 |
| cg08405844 | CGGGGCTCTCCCAGATGCCCACGCTGCCTGCTCCCACTGGAGACGGGGAC | 770 |
| cg05144928 | AATTGGAATGTGGCAGGGAAACGCTGGGAGACCCCTCCCCATCCTTCTCG | 771 |
| cg09351263 | CGGCAACATCACTCCAGAGAGCCCAGCAGCTTCCTGGGAGAGGCAGGCTG | 772 |
| cg22510362 | GAGGGTCAGGTCTGTGCTGGGGGTGAGGTCCATGCTGGGGGTCAGGTCCG | 773 |
| cg03072681 | GTCTGGTCAAGAGAGATCCAGACAAAATCAACCTTCTGCGTCAACAGCCG | 774 |
| cg06959205 | CGCAGGGCACTCGGAGCAGGTCTCTGGCTGCCTGGCACAGGAAGGGCCTC | 775 |
| cg00892703 | CGCTGATACTGACATTTACAGATGAAACCGTCTGAGACCCGCTTCCCAGC | 776 |
| cg06912355 | AGGGTGTCGGCAGCAAGATGGCTCCGGGGGTTTAGACACTGCTGGCTTCG | 777 |
| cg13606988 | CGCACCCCAGCCAGAGAGGGAATACTGCACCTCCCAGAATGTGGCCTGGG | 778 |
| cg16569347 | AAGTACCCCTGCCGCTATTGTGAGAAAGTGTTTGCTCTGGCGGAGTACCG | 779 |

60

| cg identifier | DNA Sequence | Sequence ID Number |
|---|---|---|
| cg12165215 | TCAGGAGAAATTTCTAACTGTAACCAAAGGGAGCAGGCAGGACCCAGACG | 780 |
| cg08667899 | CGCGGCTCAACCAAGCCTAGAAGTGGGGGCGGGGAATCTTCCGCCCTCCT | 781 |
| cg01130991 | CGGTATTGTCATGTTGCATCAAGGTTCCTCATTGCAGACAGAAATCTGCC | 782 |
| cg12054453 | CGGCAGAAGCTTCACCACAAAAGCGAAATGGGCACACCACAGGTAAGACT | 783 |
| cg18181703 | CGTGGCCACTCTTCAGCATCTCTGTCGGAAGACCGTCAACGGCCACCTGG | 784 |
| cg10508317 | AGCTGAAGGTCTTGAGGCGCAGGCTGGTGTCCAGGGGGCGGCTCATCCCG | 785 |
| cg27637521 | GGAAACTTGCTGTGGGTGACCATGGCGCACGGAGCCAGCGTGGATCTGCG | 786 |
| cg04243822 | CGCACCCAGCCCTTTACAAGCAGCACAAACGTATTTGGTTGTCTGGGGCC | 787 |
| cg12486944 | CAGGAGGCATGTGGGGGCCTCTGCCAGGCACTGCACAGGAGGGCGCGTCG | 788 |
| cg24391240 | GCCTCGCGGGATTAGGGGCCGGTAGAGGTGGGCGGGTCGGGCGGAAGTCG | 789 |
| cg19617213 | CGCCGAGGCCCGCTGCCCGCCCCCGCCCGCCCCCGCCCGCCCGCCTCACC | 790 |
| cg12846567 | CGCCCGGCTGGCTAGACCATTGCTAATGGCTCAAGAGTTCAGTTCTAGCA | 791 |
| cg03358468 | CGCCTGCAATTAAAGGCCAGATTGGAGGCATCTGATCCCCAGCCCTGATT | 792 |
| cg18569704 | TGTTGGACCTCTAAACGGTAATTACGGGCGACAACGCAGATCCAGGATCG | 793 |
| cg15001547 | GAGCAGCAGCAGCATGTACCTGGCCATGCTGGACGGAGATGCTTTGAACG | 794 |
| cg02991799 | GCAGCCGCAGAGACCCTTGGGAAGTGTGGTCTTCGGGTGCCGGCGGTTCG | 795 |
| cg25450266 | CGGAAGGGATGTTGTTTTGCTTCCGCAGACACCTGACCTCCAACTTCCTA | 796 |
| cg22030032 | CGGGAATGGTGCTGTCTGCCCAGCCCCACCCGGCCTTGTAATGAACACTT | 797 |
| cg09915601 | TGCCTGCAAACTAAGGCAGCATTATTCAGACCTGGGCTACCCTGGTCCCG | 798 |
| cg05971102 | CGGTCTGGTTAGAAAATATCCAGCCCCACAGCAAGGGGAAAGAGTAACAT | 799 |
| cg07520608 | CGGATTTCTGAAAATGTGGTTATTCCAGAGAATTCCTTCACGAGATTTAA | 800 |
| cg01412762 | CGGGTGCTTCCAACCAGTCTGCTGCTCCTGCCCCACCCGTGCACGACAGC | 801 |
| cg09920725 | CGGTCTTGTAACAATTGGATGGATGCCTTTGAAGAGCCCCTGTCCCTATT | 802 |
| cg12977946 | GTGGGCGTAGGACCGTCTGAGCCATGTGTGGGATATAATCTCCTGGTGCG | 803 |
| cg20150163 | CGCCGGAGGCCGCCACAACGCAGGCGCATTCAGCTAAGGACCACTCCCTC | 804 |
| cg04946715 | CTTGTGGCCATCTCTGTAAAGTTTCTGGGGAGGTGGGCACGGGGTAAACG | 805 |

| cg identifier | DNA Sequence | Sequence ID Number |
|---|---|---|
| cg08645207 | GCATGGCAAGAAGCACCCCCAACACCCAGTTCTCCTGGAGACACTGATCG | 806 |
| cg14716323 | CAATACAAATTAATAACAACCAGCCAATTCCATTGTCCTTGTATGCATCG | 807 |
| cg01956624 | CGCGGGCAGGTGCTGCTGGTGGGCGAGCTGTGGGAGCGCGAACAGAGCCG | 808 |
| cg00497251 | CGGGACAGCATGTCACTCCGGGAAGCCTCCCGTTCAGGTGTCTGGTCCCT | 809 |
| cg27114661 | AGCCAGGAAGCAGTTGGCAAAGGGGCGGGGTGACGCGGTGACGCAATCCG | 810 |
| cg03443360 | CGGAACATGTTTGGGTATAGGGAGCCATCAGACTGCAGTGCACACCTGAC | 811 |
| cg17758363 | CGGCCCCTGGATCCTAATTCAGACCAGCCTGCCTCTGGCAATAGGAAAGT | 812 |
| cg14928764 | TCTAGTGTCTCTAGGGCCACCTCACTTTCAGCAGTCTGTCCCAGGTGTCG | 813 |
| cg11850311 | TACATCTGCTCCCCTGGGCTTTCTCAATTCCTAAATAATGTTGCTGTTCG | 814 |
| cg14234805 | GGAATGGGAGGAACGCAATGCGTCCCGCGCGGCGATCTGGGATAGCTGCG | 815 |
| cg20747455 | CGGGAGCTGTCAATCACGAACACCAGATCCAGGGGCCCAGTGTGACACCT | 816 |
| cg12112556 | CGCTCTCCTAGAGAGGGCTTCTGATTTGGAGCTGTTGTTTTGGTCTGAGA | 817 |
| cg17262492 | CGGGCCCCTGGGGCCTCAGAGGGACCCCGGCTGCCACTGACATATGAAGA | 818 |
| cg07871532 | AGTGGCTGCAGCATGGGTCAAGGTCAGAGAATCCGGGAGCCCCACAGCCG | 819 |
| cg08548559 | AAGCGTACGCTCTGTGCCAGTCCTTAGGTAAAATGTTTTACTTTGTGCCG | 820 |
| cg27115863 | CGCCAGCCCGCGGAGCCCCAGTGACTCAGAGCCGTGGCTCCCCTGGCCCG | 821 |
| cg22650271 | CGGCTCAGGACCAGCAGGCATGCCTCCTTGGTCTCATGTTGACCTTAGAG | 822 |
| cg19778944 | CGCGACTCACCCACGGTGGCCGGGCAGAGCCTGCCTCGAACCCGGCTTCT | 823 |
| cg17025841 | GGAGGAGGCTTTCTGCTGGCCTAGTACTTTTCCAACTCTGACAGTGGCACG | 824 |
| cg06841024 | GAAGCACTTGGACAGGCCATCGGGCCAGAAGCACCTCGCAAGGGTGTCCG | 825 |
| cg27326062 | TACTTGCTCAGGGCTTTCTTCCCGCGTGTGCTTGGCGCTGCTCACAAACG | 826 |
| cg10747042 | AGATGCCTAATTCAGCCGGGAGCCGCGGGCCGGAGCTGGCTGAAGGTCCG | 827 |
| cg18098839 | CATAAACCAGATGTTTCTTAAAATAGCCCAGTTAAATCCACCCTTCCTCG | 828 |
| cg13457961 | CGGGCGGGGAAAGCGAGGTTTAACTAACTCCGCAGCTATATTCTTCCGCC | 829 |
| cg10407935 | CGCCACCCCACACTCCCTGCCTGGGGAAGTCGGACGCCGCCCCACACCCC | 830 |
| cg01096266 | CAGAGAGCAAATCCTTCTTCCTGCCCTAGGCTGCAGTGCTCCTAACCTCG | 831 |

| cg identifier | DNA Sequence | Sequence ID Number |
|---|---|---|
| cg05613002 | TAAACCGAGTTAATCATCGGTCCATTTGAATAATCAGTCACGTGGTGGCG | 832 |
| cg05890887 | CAAGCAGCCAAGCTGCTCCACGATTGGCTGCTTTAGCCTTACGTGCGTCG | 833 |
| cg06440348 | GCAAACCGTGAATGTGGGGCAGTGTGGGGCCAAGCGGTGCGCTTGGTACG | 834 |
| cg21833476 | TTTCTCGAGGCAGGGGGCACGGTAGCACAGGGAGCTTCTCTTTGTGGGCG | 835 |
| cg26036479 | CGACAGCCACCTTATGTGGAAAATACTCGCGCCGGCCGTCATTCATAATC | 836 |
| cg25629712 | CGGCTCAGAGCACAGGCTCAGAAATCAGACTGCCCACGTTTGAAAGCTGG | 837 |
| cg12716346 | GGCGGCGCTGGGCATTCTCTGCCATCCTTAGGGGTCCTCCACAGCATGCG | 838 |
| cg02514143 | CGCACGAACCCTTCATCCTGCTCCAAACAGAATGAGAATGTCATGTCGCA | 839 |
| cg15016740 | CGCTGCCCAAGTCTCAGATATTCGATGCCTCACATTGCCAATATGGAGTC | 840 |
| cg01256539 | CGGCAGAATTGTGCTTAATAGTTGGTGAGGTTTCTATGGGCGATGTGGAA | 841 |
| cg08260658 | CATGGATTTAATCAGTGTACTTTTACCTTCAGGCTGTAAATTAGAAACG | 842 |
| cg25024993 | CGAGATTTTTAACCTGAAACTGAAGAATCTGGTACTGTAAGTGTAAAGAA | 843 |
| cg22157525 | CGCCGAGGGCTCCAGGCCTCGCTTCCAGCCATATAGGTAGTAGGCCGAGA | 844 |
| cg21227060 | CGCCTGCTCTGTGCCCCACTACGCTTCATGCAGGGGTCCATGTGCAGCTC | 845 |
| cg17255947 | CAGTAACTCCGGCCGAATTTGTTCGGGTGTGAGGTTTAAGGCCCTAATCG | 846 |
| cg26372792 | GTGACTGGCCAAAAAAAACCGCAATTTTGGGGTCTAATTCGATTGTGACG | 847 |
| cg26867866 | GGGCGGTCAGGCGGGGTCAAGGCCAGGCAGCGGGGCGCGTCTGCGTTGCG | 848 |
| cg09144073 | CTATTGGTTGAGTTAACCAGCATTCAAAAGCAGTCAGCGAACCAACTACG | 849 |
| cg11074353 | CGGACAGGTTTTAAACAACCTACCAAGAGCCAGCGTGGGTGTCCACACCC | 850 |
| cg10000096 | CGGGGTGAACGTCTGGGTCCTCGGAAATCCTCAGTCTCTGGAACTGAATT | 851 |
| cg17501210 | CGCCCGATTCAGACAGCTGGACTCAGAGGGATTCTGCTCCACAGAGAAAC | 852 |
| cg26960221 | GGGAGGCCTTGGTTGGAGTGTCTGAGGCCTCAGCTGGGCCCCTGCCTCCG | 853 |
| cg24172278 | CGCAGCAGCCAGCTCATGGGCCCCGCATCCAGGTGTGTCCTGCCCTCTTC | 854 |
| cg01753176 | CGCTGGGCACTGCCCTGGGGGCTGAGCAGCTGTCTCCATTGGTCATTCAG | 855 |
| cg21843627 | CGGAACTCGCTGGAAGCTGTGATAGTCCCAGCTATGGTTTATCACAGGGA | 856 |
| cg19080320 | GAGCTGCAGCTGGGGACTGGTGCGCCATGCAATTGACATAGCGCTGCTCG | 857 |

| cg identifier | DNA Sequence | Sequence ID Number |
|---|---|---|
| cg20915897 | TACTTCAGGGTAAAGAAGTTCAGGTCTGCCATCTCCAGCTCTGTCTGGCG | 858 |
| cg26116499 | CGGCGGCTTCCCCAGGTGCTGCTCCGCCCAGTGCCCCCCCAGATCTCAAC | 859 |
| cg07665217 | AGCATGACCAGAATGGTTCCTCAGAATCAGGAGGTGAAACCTAACATTCG | 860 |
| cg02505126 | AGAGTGGCGCGAGCCTGCGTTTTCCGGCCAGAGGACATGGTGCGTTTTCG | 861 |
| cg13565994 | GGATCCCAAATAACAGATCTGGGAGAATCCTGCCTCAGTACTTGGATGCG | 862 |
| cg17561365 | CGCCCACAGGGCCCAGTGGGCTCTGAGTAAGGTGCACGGCACACACCAGC | 863 |
| cg09194449 | GCCTGGGATCATCCCCAAGTGGGTCTAGGGTGGGCCTGTGAGGAGGAGCG | 864 |
| cg26206456 | CGACTGCAGGCCTACGGCTTCCTTTGAGGGCTCACGTTTACAAGATAACA | 865 |
| cg21462914 | CGGCCGCCAACCGACCACTGACTGTCTGCCAGGCATCCGGGCTCCCTCCA | 866 |
| cg17560136 | GGCCTCCAGAGCTGGGTAAGTGGAGGGCTCTGTGTATGTTGCTGTACACG | 867 |
| cg19033875 | CGCCCATGCCCGCGCTAGTGGGAGGAGGCTGGAAAGCGCCTTCCCATTTT | 868 |
| cg18995788 | CGGGCAGATACGAGCAGATTGACTCGCCAGGACTGTCATTGGGCCACCGC | 869 |
| cg14387545 | CGGCGCTGACGCCGCAAGGCCACTTCTATCGCTCCCGACCACGGTCTGTT | 870 |
| cg23083672 | CGCTTGTCAGAGTCACCATCTACTACCATGTATTTCAGGCATGGGGCAAA | 871 |
| cg02758183 | CGAGGTGCTGGTGTTCATACAGATAAGGCTCTCTTCATTGCAACAGCCAC | 872 |
| cg20308511 | TCATTTGCAAATGTTACTGGGGGACACACCGGCTCCCAGTAGGGTTTCCG | 873 |
| cg21412053 | CGCAAGAGAAGAGGGAGCACGCCCAGAAGCTGATAATGCTGCAGAACCTG | 874 |
| cg26071410 | TGGCAGCCCAAACGTCAGAGGCACCTCACTTTGCCATGGCCTGGC | 875 |
| cg09625066 | CGGCCCAGTGAACCTGAGCTTTGGAGTTTGGACGAATAGCTAGTTTACCT | 876 |
| cg11235848 | CAGGGACTGACGCCCCTCTAGCAAAGCATCTGTGGTACATCCCAGACCCG | 877 |
| g02311013 | GATCTCACACTTTGGAGACATACCCCTAAGTAGACCAGAGGCATCACACG | 878 |
| cg21664443 | CGCCTGGAGAAGCAGTGCCAAGTCTATTCCCTAATAAAGTACTTGTTGTG | 879 |
| cg05127178 | CGGAGCTGCAGAAAGAAAGGGAAGAAACTAACGTTCTTTCTGCGTCTGGG | 880 |
| cg19110434 | CGCTAAAACTCACAGCCTGTGGACAAAGCTTTTTGGTTTTCAAATCGGC | 881 |
| cg25697726 | CGGTCAAAATTAAAAACTTCTGCACTGAAAAGAAAAAAGGGAGAGAGAGA | 882 |
| cg03204600 | CGCCCCCAAGAAGCTCCCCACCCCCAAACCAGAACTGGTGGCTGGTGACT | 883 |

| cg identifier | DNA Sequence | Sequence ID Number |
|---|---|---|
| cg11049305 | ATTGAATGCCAAAGTTACTGGTCCCCAGGCAGCAGCTAGCACATTCAACG | 884 |
| cg13501581 | CGAGTGGCTACTGTATTGAACAACACAGATCTAGAAACAGAGTGTGGACC | 885 |
| cg01564693 | CGGGAACCTCCACAGGCTAAGGTGAGTGCCAGCACTGGCACCAGCCCTGG | 886 |
| cg10509982 | CAAAGGCAGACATCGCTTCAGGTAGGTTGCAGCCAAAATGGCTTCCAACG | 887 |
| cg20557017 | TTCTTTGTGGCCAAGAAATTCCCACCTTACCACTTACAGGGAGGCCATCG | 888 |
| cg05373692 | CGCAGTGTGAAAGATGCGAGCCATAGCATCTCGTAACATGAAGGCCATCC | 889 |
| cg05470074 | TATTTTGTATTTGGCTTGCTCTGGTΓTCTGCTGGAGCTTTTAAAAAATCG | 890 |
| cg08262933 | CGGGATTACCTAGTGCCTTCACAATCGGTCAGAGCTGGATTCAGATTCCT | 891 |
| cg15062055 | CGGGTTACTACTGTACCTACCAGCTGATTCCAGCAGCCTTTTCTCCAGCG | 892 |
| cg12889449 | ACCGTTTTTTCCCCCGAATAAAGTGTGCCCGTGTAGCTGTACAGATTTCG | 893 |
| cg23955970 | TCTTTCTTCACGTCTTGTTGGTTACTGCTACCTGGCTTCCAACTGTTGCG | 894 |
| cg05530348 | AGCTGGCCAGACTGCTGCAGTTTTCAGTCCCAGGAACTGAGAACGTGGCG | 895 |
| cg11595135 | GGATTTGGTCTAGGCTTTCTGGCTTGGCCCTGGAACTCTCAGCAAGGACG | 896 |
| cg06298190 | CGCAGGTTACTTCCTATAAATGGAAGAACCTGAATCATTAAAAGATAAAA | 897 |
| cg20686207 | TAAGGCCTCGGCCATGACTGTGGGTGGGTTTAACTCAGCTAGCAGCTGCG | 898 |
| cg14686949 | CGAGACTAAGAAGTGACCCAACCTAGGTGACCCTTCAGCTAGGTCACTCT | 899 |
| cg18003791 | CGCCCATAAGGATATAAGGGAGACAGAGGGAAAAAGGAGGAAGCTCACTC | 900 |
| cg05295197 | GCAGGCGGGCCTCAGGGGCTGTCTTTCCTGCACGGCTGTTGTGTGCTTCG | 901 |
| cg23106779 | CGGCACCACACAGCTAGTGCCGGAACTTTTTGTGCAGAGAAAGAAGACCC | 902 |
| **cg10143960** | GGTAGGGTGGGGCCAGGCTGAGCCTGCTCTGCGGGTCCCGGACCCCAGCG | 903 |
| cg07284273 | CGTGCAGGCCACAGTAATGGTGGTGCAGGTGGCAGGAGTAGGTGCCCTCG | 904 |
| cg17747265 | GGACCTGAAGATCTGCATGTATGACCGGCTCTACCAGGACTCTGTGCTCG | 905 |
| cg09971184 | TGACACTGTCTTCGGGGCACATATGGTCATCTATGAGTGCTGTGAGCACG | 906 |
| cg00935435 | GAGGGGCTGGCCCCCTTCACCCTGTGATCGACAGGACAGAGCTGGGCACG | 907 |
| cg15651727 | ATGCATGTTTCACACCTTCTAGAACTCAGAGTAAACCTAGCTCTTCAACG | 908 |
| cg13990487 | GTTAACAACTGTCAACAGCCTATAGAAGATCAAGGCTAAATTCCCTAACG | 909 |

65

(continued)

| cg identifier | DNA Sequence | Sequence ID Number |
|---|---|---|
| cg00456299 | CGAGTTTAGCTTTTAAAGAATCCTTTGAAGAAGGATTGCAACGTGACTAA | 910 |
| cg04974804 | TAGAGAAATACATTGTAACAATTTGGTTTCTTTCCTGATGGCCTTTATCG | 911 |
| cg13574848 | ATTACAAGCATGAGCCAGGGCACCCAGCCAGAAATGATATTTAAACTACG | 912 |
| cg25616777 | CCATGTATAAGTCCCCAGGACTCACAAATCTCCTCTTTCAGGATATCTCG | 913 |
| cg12386614 | CGTTAGGTCCCACGGCTTGCCCTGCTCAGCACCTAATAAGGCCATTAGCT | 914 |
| cg15355146 | CGGGGGCTAAGCCAGGCGCCGTCTGCCTCTCCACCTAGACCAGACCCCTTC | 915 |
| cg19471466 | CGGGGGGTGCCAAAAAGCCCCTCCTTCTGACGGCGCCCTCCTGGAGCCCAACA | 916 |
| cg14709691 | CGCTTTACGGAAAGAACGCCCCGCGAATAAATCCGGAGCGGATTTGCATC | 917 |
| cg21280384 | CGGAGCCACAGAGAACACTTTTGAGACGCAGACAACCACAGATGTGCG | 918 |
| cg14476101 | CGCCAGCAGATACAAAGGCAGACAAAGTGAGCGAGGCAGTTTCCAGGACC | 919 |
| cg19693031 | TTCCTATCACAATTTACAAAAAGCCTCCAAAAAACCTTGAAAAGCTTACG | 920 |
| cg16700924 | CAAGGGAAGCTCCCGCCTCAGGGTCTCTGTCTTCCTGTGGTTAAGAGACG | 921 |
| cg20246707 | CGCTCCGGCTGGTAATTTCCAAAACACAGAGGGAAGAGCATGGTGGAGCAA | 922 |
| cg01365152 | CGGAACTGTCTCCTGACCCGTGGGTACAGTATTTTCTCGTATGCCACCAAG | 923 |
| cg20327105 | TTGGTGCAGTTTTGAGCGTCCGTGTATAAGGAGTGGCTCCGTAGTCACCG | 924 |
| cg25741192 | CCATCAGTAGAGCAGGAGTCGTTGGCTCAATGCAGTCGTGTCTGCAACCG | 925 |
| cg13678787 | CAGTGTTCGGCAGAGGACAATCTGTTCGAGAATAATTAGATTCCCCCTCG | 926 |
| cg08614871 | CGATGCTTAATTACAAAAGGTTTGCCCCTGTAGTGACCGGGCAGCAAT | 927 |
| cg22660341 | CAGCTCCCACCTGTGCAGAGTGGCGGCCTGGGAGCTGAGCGTGGCCCTCG | 928 |
| cg19647685 | CGCGCGAGCTTCCTTACAGGAGGCTTTGAATGCTGCCTGGAGTATATTTGA | 929 |
| cg03153178 | CGCTTAAACAATGAGCGGGGCTATATGTCTTGCGCTTGTCTCTGAAGGAAGG | 930 |
| cg10531748 | CGCCGGGAGCTGCTCGCCCCAGTGTCAGCAGAGCCTGGCCCCAGGCCATGAC | 931 |
| cg01508796 | CGGGTGAGTGTTTAACTTTCAGCCCAGCTTAAGATCTCCGAAGACACCCA | 932 |
| cg06439736 | GCAGCGTGCCTACGCTTTGTTGCAGGAGGCAGGTTGGAGGGACGCTGGACG | 933 |
| cg18853287 | AGGATGTTCAGTGACTGCAGCCAGGCCTACCTGGAGAGCTTTTGGAGCG | 934 |
| cg13446658 | CGGCTTCTGAACTTTGTACTTTGCGAGTGTACTTTGTACTTTGGTCCTGT | 935 |

| cg identifier | DNA Sequence | Sequence ID Number |
|---|---|---|
| cg03322338 | CGGCCTCTTATCTCGACAATCGGTTGCTCTTGGCCAGCCCGTCCTGCTGC | 936 |
| cg09566331 | CGCTTATGCCCAGCTGCTCCAGCTGGAAAAGACAGGACTGCAAGTGGGTT | 937 |
| cg21992507 | CGGACTTTCTGCGCAGAGAAAACACTATCTCCTTTGGAGGCTGTGCACTT | 938 |
| cg01438090 | CGCTGATTGCACTGTCAAGAAGGAAAATACTGTTTGAAAAGACAACAGGA | 939 |
| cg01562537 | AACAAACCATTACGTGTTCCAATTAGAGCGGCAGCGGACCTGGCTGGCCG | 940 |
| cg17044529 | CGGCAGTGAGTCACTTACATAGATGTGTGTAACATGCCCCAGAGACCTCA | 941 |
| cg00574958 | AGGCCAGCGAACATCCAGCTGTCAGTTGGTCTGGGGACTATCAGCATTCG | 942 |
| cg17058475 | AGCGACACTTATGTCCTCAATTTATTGCTACGTTCACTCGGTCAGAGTCG | 943 |
| cg21960184 | TTCATGCCGGACAAATTTCCCTATGAACAATGCAGCCAAGGGCTGCATCG | 944 |
| cg02395454 | CGTGGGCAGAGTGGTTGGCTGCCCCACTGTCAGCCTGTTCTGAAAGTAGG | 945 |
| cg24675983 | TGCAGTTCTTATAATAAACACTTAAACAGGGCCAGGCACTGCAGTTCACG | 946 |
| cg21574681 | AGGACTAGATGTTCACCTTAAATCCAGCCACTGCTCCAGACTGCAAATCG | 947 |
| cg17850642 | CGCCCTTTTCCGGTATCCTGGGLCTCAACAGGGATAAAAGGAGGCAGCACT | 948 |
| cg01031441 | GGTGGGGACTGGCATTAGGGGTGGGCGGGAGAGACCCTAAAAATCCACCG | 949 |
| cg01638369 | CGTGTGTTTGTAATTGTGTATGGTCCTGCTAATGTTTGTGTTTATCACTG | 950 |
| cg07539443 | CGGTTCACAGCATGGGAGCATCTGTGCTATACCAAGATTACTCATAGGAT | 951 |
| cg15997315 | CGGGGCTGTCAAGTGTCTTTAAAAGGTCCCTTCAGGGTGGATGGAAACCA | 952 |
| cg13842421 | GCCCCCAGCTCTCAAGGCCGGTGCAAGGATGGGCTGTGCTCAGCGATGCG | 953 |
| cg02925268 | TCCTCAGACTGTTGGGATGATGAGCGGACGGTGAACTCCCACCACCTGCG | 954 |
| cg26975476 | AGCGGGAGTCACTTCGGAGTGGGGTGTTGACAGCGTGAAGCCCCTGAGCG | 955 |
| cg13589330 | CGCTTTCAGGGGTCAAAGACCTGGCAGAAATGACTTCCCAACCCCAGATG | 956 |
| cg02853801 | TAATTGCCCTCCTGTTTCCTAGAAAGCTTATAGGGCAGCAGTAGGAAACG | 957 |
| cg06147196 | CGCAGAACAGCACAGTGTCACATGGTCTTCACGGCCCTGAGGGCCCAACT | 958 |
| cg24126612 | CGGGGACCACCAGCTGCTTTGCCCATGGATTGGAACGCATTGGGTGGAGT | 959 |
| cg26407029 | GCTGTTCCTCATCACTCAGTACCCAGTTTGCTGACGTCTACAATATTCCG | 960 |
| cg01802397 | CGTGATTGCACGTATCAAGATGTGTGCTCTTAGATTTGTTTTCAATCCAG | 961 |

| cg identifier | DNA Sequence | Sequence ID Number |
|---|---|---|
| cg01885071 | CGGCGCTCAGCAAATTTAGATGAATGCAACGCTTCAACGTGGGCTCTCGA | 962 |
| cg01808343 | GTCTTGATGTGACCAGAGAACACCATGTACAGGCAAAGCCCAGCCCCTCG | 963 |
| cg15471661 | GCTGTCGCTGCAGGTGGGAACCGAGAGCCAGGAAGCCCAGGGAGCTGTCG | 964 |
| cg16558846 | ACAAGGGGCTCCTCCTTTGTCCTGGGGGAGTTGCATGGATCCTGTCTTCG | 965 |
| cg17382302 | GTTCCACATAGAGAGCCTGGCTGCATGATCTTGAGGTCACCTAACTCCCG | 966 |
| cg25291250 | CGCGCCTGGCCTATACAATATTTTAAATAATTTTGTGTGTGATACAATGA | 967 |
| cg01021485 | CGCCCGCCTCATTTGCGCCTTGCAGCACTGCTGGACCAGGTATCTGCCCG | 968 |
| cg27505538 | CGGGTCAGACCGTCATTGGCCATCTCACAGTCCCGGCGCTCCAGAGCCCC | 969 |
| cg24924577 | CAGCCATTGGCTGATTTGAAAGAATACACAGGGTGTGCCAGCTGGTGTCG | 970 |
| cg00564555 | CGGTCTCAGCCGTCCCCATGGTCTCAGCCGTCCCCAGGGTCTCAGGAGTC | 971 |
| cg02193806 | CGGTTATATCAACATGATGCCCAAACACCAGGTCTGCTTAGGGTAAAACT | 972 |
| cg05732130 | GTACAAATGGGTCCAGGAGCTTCAACGAGCGTTCCAGCTTCTTCGTAACG | 973 |
| cg02189786 | CGCGCACCGCCCCGCTCCACGCCCCTATCAGGATTCGGGTCCTCGTGAGG | 974 |
| cg00534468 | CCTCGACTGCTGGCTGTGATGTTCTCCAACACTGCTATTCACAGCGAGCG | 975 |
| cg03699074 | GTCCCGTTCACACTGATCGTTCCATTTCCTCTTGTGTCTGAGTGGGAGCG | 976 |
| cg01829657 | CGGGGGGTTGGTCAGCACCACACACTGTGCACAGGGAAGATGGAGCCTCGC | 977 |
| cg00892703 | CGCTGATACTGACATTTACAGATGAAACCGTCTGAGACCCGCTTCCCAGC | 978 |
| cg27113347 | TCGATGCTGGTGGTCTGCGTGGTGGTTCCCAACAGCAGTTCAGGGGCTCG | 979 |
| cg09826306 | GGCGGCTCTAGCCTCTCGGGGGACGTTCGGCTACTCCAGTGGGGGGGTTCG | 980 |
| cg25567048 | GAACAGCCACGCATGGTCCGTGAAATGGGATCATGTGCAGAACAGCCACG | 981 |
| cg06513864 | CGCCCCACCAGCCCGAGAGCTAGAATTGAATCTGGGGGACCCCTGCGGCG | 982 |
| cg11020638 | AGCACTGTAGGGAGTGGCCGGGATCATTCGAGCCCACAGATAGTGCTCCG | 983 |
| cg05815247 | CGGACAGCTAATCGTTTTAGTGACAGGATGAGAGAGCCCTTCGTGTTCTG | 984 |
| cg21348173 | CGGCCTGACACTCTCAGAGATGGGATTCCCAATCCATCCACTCTGAAATG | 985 |
| cg22850234 | CCGGAGGCTGATGCCTTATCCATTAGGCCACTGGGTCACCACAGGATCCG | 986 |
| cg10508317 | AGCTGAAGGTCTTGAGGCGCAGGCTGGTGTCCAGGGGGCGGCTCATCCCG | 987 |

| cg identifier | DNA Sequence | Sequence ID Number |
|---|---|---|
| cg27637521 | GGAAACTTGCTGTGGGTGACCATGGCGCACGGAGCCAGCGTGGATCTGCG | 988 |
| cg16252995 | CGGCAGAGAGGACAGGATCCTGCCACCCAGCCTCATCCCCATGCTTCCCC | 989 |
| cg03524223 | TCCCACCTTGTGAAACTTTGCCAGGTGTCGCTCACAGGGGACTGGGTCCG | 990 |
| cg05495454 | AGAACATCCACTCTCTTCCAGCCAGAATACCATTAATACGTGTGTCAGCG | 991 |
| cg07573872 | CGGGAATGGTGACTCAGCCTTCCAGGAACCTGCGTGGCGTCTGTTTTTTT | 992 |
| cg22519313 | CTTCCTATGTGATTTTCTTCCAACATTCTCCCATGAAAAAATGTCAGACG | 993 |
| cg05313771 | TGGGTGATGGAAACTGAGGCACAGTCATCCCCCCAGCAGGTCCTTGGACG | 994 |
| cg09522532 | CGGGCCCCCCAGAATGGAACACGAGGGGGTGGTCTCTTGCTGGGCTCCAG | 995 |
| cg15783941 | CGCCAGCCCAGGGCTGGATGTTTTATTTTGGCATGCCCCAGGTGCTCTTC | 996 |
| cg24115922 | CGCACATGCGCATCCAACCCGATTCCACGGGTTCCCAGGAGGCGGATTCC | 997 |
| cg18593194 | CGGGAAGTCTGCCAGTTGGCAGTCTCTGACGTAAAAGATAGCAGATACTG | 998 |
| cg25654926 | AGCCCCACTAATAGATATTCTGATTCTGTTGGTCTGGAATGGGAACCGCG | 999 |
| cg10853231 | GGAGGGCAGGTCCGGGGGCTCTGTGTCCCCACTGGGCCCCCAGCACTGCG | 1000 |
| cg19717326 | CATTAAAGCTCAGACTCGGGTTTTCCGAGCAGGTGTTGCAAGCCTAGCCG | 1001 |
| cg13650654 | CGGGTCTCTAAGATGTCTTATGAATGCAGGTCAGAGGGTCACATGTTAAC | 1002 |
| cg02875404 | CAGTGGATAGTGACCAACTCTGTGTTGGTCGCCTCATGTTTCCCAGCACG | 1003 |
| cg14889658 | AGCATTCGGGCATCAGAGGTTTGTGGAGAGGGCAGCCCAGTATACAACCG | 1004 |
| cg02591356 | CGTCTAAATTACTTGGAGTTGATCTTTCTAAACAGGATTGCTCAGACACA | 1005 |
| cg26471058 | GCCTGAGGTCAGGAGTAAGCAGACACCAGCACTGCTCTTTCTCCAAGACG | 1006 |
| cg25939861 | CGGAAATCAGCTTGGGGGCCTTCTAGCCCTGCAGCTCAGAAAAGTGTCAG | 1007 |
| cg03913456 | ACCGCGCTCAGCCCGTGGAATTTACTTTATAATGGGGAGGCAGATGATCG | 1008 |
| cg24506130 | CGGTTGTCAGCTGCCGGAAGAAGAGCGTGGAATTGAGAACACAGACCCCT | 1009 |
| cg26009035 | TGGGTGCCAGGGGGATTATCAGCGTCTGCGCAAGGCCGATGGTGGCAGCG | 1010 |
| cg05308744 | TGATTGGATGCAGGATTGGCGGGAAGGCAGCGGGGTCATCAAAGGCTGCG | 1011 |
| cg22663545 | CGATGAGCTCAGCCCCCTCAGTCCAGGCCGAGAGCCAGATGGGGGTCACG | 1012 |
| cg02767242 | GTCCCCCTCGTTTCCTTTTAGAAGTCATTGGCCGATCGTTTTAAAAGTCG | 1013 |

(continued)

| cg identifier | DNA Sequence | Sequence ID Number |
|---|---|---|
| cg13603203 | TGGGATCAGGATCATGGTGAGATGTATGCCTTTCCCAGGAATGCTAGCCG | 1014 |
| cg20951650 | ACTACTCTCCTGGAGTGGTTACAGTTAACCAAGGCAAAACCCCTTCTTCG | 1015 |
| cg12683410 | CGGCTGTTGTCCTGCTTACAGGACTCCAGAGCAGCAGGAAGGAGCTATGT | 1016 |
| cg16887334 | CGCACTCGGCCTGACCCACGGCGACCCTCTGTGACCAATCATACTACCAA | 1017 |
| cg26126740 | TATTCCAATTTGGGCCTGCGTGTTCGGAGCTCCTGGTGGCGGTCTCCACG | 1018 |
| cg10120040 | CGGGCTTTGGTGACCAGGTCTTCTGTCAGTGAGCACACATAGCCCGGAGG | 1019 |
| cg25761955 | CGCCTGTGTGGACGCTTGTGAGCTGCACCTTCATGCTATTATAATGGCGA | 1020 |
| cg22871721 | GAAAACACACAGATGTCAAACCAGAGGAGCTGCCAAGCTGGGTACCGACG | 1021 |
| cg22778797 | CGCCGGCCCCAGGTAGTATATTTTATTGGCACAACTGCCGGTATTTGCC | 1022 |
| cg27243685 | CGGAGGATGTGTTTCCAGAGAGCTGGTCTATTTCAGACTGACAGGCCACT | 1023 |
| cg06500161 | CGGCGGGACTAGTTCCTTTGGGCATAATTTAGGTGTTTTGTGAAATGTTA | 1024 |
| cg17215151 | TGCTTAGTAGCTAAAGGCCACCTTCTATAACACGAAATAGTCTACAAGCG | 1025 |
| cg01127300 | ATGCCTTCTTGGGGCCTGGCTTGCTGCTGACGGGAGCACTCAGGAATGCG | 1026 |
| cg12511214 | CGACAACAGGGACTCGGTGGACCTGGCCGAGCTGGTGCCCGCGGTGGGCG | 1027 |
| cg07031797 | ACAGTCGAAGCGCACTGCTCTGGTGGGGGATGTTGGCGGTGCAGGAGGCG | 1028 |
| cg18405719 | CGAGGCTAAAATGTGGGGCCAGAAGATAAATCTAATGAGAGCTGTTGCTA | 1029 |
| cg21614211 | CGGGGGTGTGTTTCTGTTTGTGTAACTGGGTGTGTGTGTATCTAGCCATC | 1030 |
| cg06813250 | TAGAAACTGATGTTATTCATAGATTCCACACATTGTATAGAAGAACATCG | 1031 |
| cg15705999 | CACCCCCAGCCTCGCCACGGCTCGCTCCCCCCGGGGTCAGGCCGTTACCG | 1032 |
| cg09000779 | CGGGACTGTGGCCAAGGCCCTCAGAGGCCATGGGGAGACACGACAAGACC | 1033 |
| cg12473849 | CGCAAGCACATAACAAAAGCCAAGTTAAGAAGGTTAGCGATTTAGTTGTC | 1034 |
| cg27336439 | TCACCATTGCCACTTAATGGTCTGAGGGGGGCTTTTCCTGTTACTGCACG | 1035 |
| cg00480331 | TGGATCGCCCAGGGGCGGGCAGTCCCTGGAAGACAGGTACGGAGTGGTCG | 1036 |
| cg13724379 | GGAAACGAATCTGGGAACGAGTTTGAAGCGCGGTCAAGTTTGACTCATCG | 1037 |
| cg15012625 | CGAGGGTCCCAGTGTTCCACATCCTTGCCAACGCCTGTTATTGTCTGTTG | 1038 |
| cg01658265 | CGGGGTAACCCCAGGTCCCAGTGATTTCCATAGACAAAAGAGATGCTGGT | 1039 |

70

(continued)

| cg identifier | DNA Sequence | Sequence ID Number |
|---|---|---|
| cg00252095 | AGTCTTGCCTGGTTACAGGCCTGTGCTCTCCAGGCAGAACAAGCGCAGCG | 1040 |
| cg26805150 | GACAGTTCTTGGGAAGCCGGCAAACTGCAAAAGCCCCATATTTCCTTCCG | 1041 |
| cg02373784 | CGGGCTCCTCAGGTCTGAGGCAGCTCTACCACTGACTTGGTGAATTTTAG | 1042 |
| cg07951602 | CGCCTCTCAAGAGCACGATGTAAGGGCTCCAAGATGAGTTTGGGCTTCCC | 1043 |
| cg19719391 | GGCACTTTTCAGCTCTAATGCTCTTGCTTTCCAAAAGCAAAACAGAACCG | 1044 |
| cg08284371 | CGGGGCAGCACAGACACCCGGGCAAATTCATGATCTCTACCGAGTAAATA | 1045 |
| cg26816491 | GTGTCCTTTAAGAACTGTAACACTCACCGCCAGGGTCCACGGCTTCATT | 1046 |
| cg06690548 | CGTGAACTACCCAGATGCAGCATCCGCTGTTGGTGTGATCTGGAACACCC | 1047 |
| cg18376497 | CGGAAGGCTGAAGGGGGAGATAATGAGCTAAAGAACAACTTTCACCACCC | 1048 |
| cg06176987 | TTTAGTTTCCTTCTTTTATTTTCCTGGCAATAGCCCACCATTGTATATCG | 1049 |
| cg22744079 | CGGACACTGAGGCCTTGTAATGCTGTTGGCTGAAGAGAGGTGGTTGGTCA | 1050 |
| cg16400903 | TCCGCGGGGCCTGAGCAGAGTCGGCCTTGTAAGGCAGAGGTCTCGGTGCG | 1051 |
| cg09884076 | CGGCCTCTTGTCCACCTTGCCCCGAAGGCAAGTCTGGATATGAAAAGAAA | 1052 |
| cg12599971 | AGCTTCTTTGCTTTGACTGTTTCCTCTTCGTCTCTTTGGTCTTCG | 1053 |
| cg26403843 | CGGATTGCTGCTTCTAAGCCTGAGAAGGTCATTTAATGAACTCATTGAGG | 1054 |
| cg07017437 | CGCGCCAGCCCATGTATGCCATGGAAATATGGCTGTTGGAATTCTGGTGT | 1055 |
| cg21036336 | CGTCTTGGGGACGCTATGGCTCCAGTTCTGACACTCAAGAAACGATGGAT | 1056 |
| cg03714531 | GGGCGGCTTCTGATCTCTTGCGGACAGCTCAGATTAGTGGCTCGAGGCCG | 1057 |
| cg23036136 | CTTGGGTGCCCAGGTCTCTTCCTTCCTGAAGAATGCGGCACTTGCTTCCG | 1058 |
| cg10189661 | CGGGCAGACTGCGATGAAACCCCAGTTTGTTGGGATCTTGCTCAGCTCCC | 1059 |
| cg05218245 | GGTTTCCAGGTAGCTTGATCTGTTCCAGGCCTGGCTGGGGTCTGCCCTCG | 1060 |
| cg04018738 | ACCCACCTGCCCGCCGGATCTGGAATAATGTGACTCGCTGGTTTGTCACG | 1061 |
| cg02115397 | TGCTGCGGTTGGAGCGCTCCACCAGGATGTCCAGATGGGCTTTGATTGCG | 1062 |
| cg12927358 | GGGGTTTAAGGATGATTCCCCACTCCACAGCCTGACGCATCGACTCTGCG | 1063 |
| cg05466385 | CTACGAGTCTTATTTCCTGTTAACTTTTTTCCTTTTGCTTGATCCTATCG | 1064 |
| cg01702009 | CGGCTGGGGGAGGAGGCACCCACAAGACTGCCTTCTAGGCCACAGGCCCA | 1065 |

| cg identifier | DNA Sequence | Sequence ID Number |
|---|---|---|
| cg06560379 | CGGAGGGAGGGAGGAACTGCTCTGGAAATCCCCTAGGCCAGGGCCTGCTC | 1066 |
| cg21710255 | TGTTCCTGTTACTCTTCGTGAATTTTCTCACCATAAAACTGTTGCCAACG | 1067 |
| cg20459712 | GTACTTTGATGCTCTACTTGGCAGAATGTATTGCTTCTGGTACCCACACG | 1068 |
| cg18718984 | GTGCTACCTAGAGAGGCCAGCATGTGGGCTCCACATTTCTGCTGAAGACG | 1069 |
| cg03707967 | CGGTGCTTATAATACATTCCTGTGTGTGTGTTCACTTTGATTTACGGAAA | 1070 |
| cg02027561 | CGTCAGGTTTATATCTTTGGGATTTGAATGTAAAGATGTTGGATTTTTTTT | 1071 |
| cg00695799 | CGCTCCATGTGACAGAAACATGAGCGTTTCCAAAATGAAAACACTGGGGG | 1072 |
| cg18116267 | TTCCCTTGCTTTTCCCAGCTTCCGGTAGCCCAGGCATTCCTTGTCTTGCG | 1073 |
| cg17501210 | CGCCCGATTCAGACAGCTGGACTCAGAGGGATTCTGCTCCACAGAGAAAC | 1074 |
| cg26905845 | CGGCTAGAGGATGACCCATCTTACGCCTATGGAGAACACTGCGGAAATCA | 1075 |
| cg03730314 | TTGCCTGTGTGTGATTTATGCATGTTCCCACCAGCAGGTGTGGATTCTCG | 1076 |
| cg03394401 | CGGGCTCCGATCTCCGGTGTCACAGCAAAGATCCGCGTCTTTCCGATGAA | 1077 |
| cg19196326 | CGCCTCCCCACGCACTTCCCTGTATGGGTTACAAAGCAGGTGCACATCCA | 1078 |
| cg13550401 | CGTGGCACACAGAGCCAGGTTTCCCCGAAGTAGTCTAACGCTGTTGCTAA | 1079 |
| cg08549335 | CATTGGCTCTTCACTTGTTGCTTGGTGTTTTCCTTAGTCTTCTGAGATCG | 1080 |
| cg17220237 | CGGGACATGAAGTCTAAGGGGATTATTTTGGAGCTTTAAAACGTGATGAC | 1081 |
| cg22322818 | CGGGAATTTGGTGAGAGTCCCCCACAGTTCTGTCTGCCAGGCACTGGCGA | 1082 |
| cg08695830 | CGCATCCTCCAGCCATGCTATGCTTGCAAAGATGTCTCTGGTCATTGGGA | 1083 |
| cg17981101 | CGCCCCAAGTCATGAGGGAAGLITCCAGACTGACTCAGGCTCAGGCTGGC | 1084 |
| cg01337813 | GGAAGTCCTCTGTGCATGCGTGTCTGTGCCATAGGCGTGGGAACACCTCG | 1085 |
| cg25977769 | CGCCTAGGTATGGGAAATGTAAGTATCCTCAGCAGAGACCACAGTTCTCT | 1086 |
| cg07592681 | CGGTCAGCACTGGGTGAATGGGTTGAGGGAACCCACATAAAATCCCCAAG | 1087 |
| cg09584650 | CGATCTTCCCCATGGAAGACATGCACATGGGTTAGGAGAATACAACACTC | 1088 |
| cg01070197 | CGCAACAACTTCTCAGCTACAGTAAATCAGGATTTGGGGTGATAGGAAGG | 1089 |
| cg17744279 | CAATGTATACTATAGCAAGTGGTACCAGTGCTGCGGGTACCAGTGCTGCG | 1090 |
| cg13518625 | CACAGATCAGACTGCTAAGTCTTTACACATCCGAAAGTTAGACTTTGACG | 1091 |

| cg identifier | DNA Sequence | Sequence ID Number |
|---|---|---|
| cg03393241 | GCCACGGGAGATTTCATCAGAAGAATATCAGTCTATTGATATGGGTGCCG | 1092 |
| cg22660904 | CCTCCCTACAACTGTAGGTTCCCCTGCTGCTGGGCTCAGCTGGGAGCTCG | 1093 |
| cg26074100 | CGGGACAGCCCCGATGCCGATGCCTGTGTTCTGTGTCCCCAGGTCTAAAG | 1094 |
| cg01029867 | GAGCCCAGCGGGGTTAGGGTTCAGGTGAGGGTTTAGGGTCGGGGCGAGCG | 1095 |
| cg14268714 | CGTACCCCGGGATCTGGCATTTAGCATGGCAATCAAATATGTGGTGAAACG | 1096 |
| cg13673536 | GTTTAAAATTCTCTCTTCTGAGTAATAGTGTTCATGTGCTCCCATTCTCG | 1097 |
| cg14457452 | ACTAGGTAAACACCACAGTAATAACTGTTGCAGGTAAGAATCATCAGGCG | 1098 |
| cg21208062 | CGGGAAATGACCAAGCGTCTCCACAATTTAGGGGCAGGTCAGGGTACCTT | 1099 |
| cg12020464 | CGCCACAAACCCACCAAGGTGCTCTCCCCCGAGTACTTGTGGGACCAGCA | 1100 |
| cg13873209 | CGGCACCACTCATGTTCATAGGCCAGCTGAGGCTCAGGGGGCTGACTTCC | 1101 |
| cg13499318 | CGCTGGGCAACCGTAAGTATTTAGGGAGACAGAGGTGCCTCTTCTGCAGA | 1102 |
| cg13915886 | CGACGCCCTTGCTCACAGGCTCCATTGAGAGGCTGCCCCGGTGCTCATCT | 1103 |
| cg22249566 | CGCAGCCCTGCGGGACCTGAAAAGGCGAGACTAAGTCACCAGCCAAGCTG | 1104 |
| cg17511128 | CGGTCACTGTTTGTCTTGAAAAAAAGAGAGAAAATGTTTGTCATTCCAGG | 1105 |
| cg02495445 | CGTCTTCTAGCAGGTATGAGGGAGACAACTACAAACGCCAGAGGCTCAGC | 1106 |
| cg16088894 | AGCTCTCGCCGCTGAAGGGGCTCAGCCTGGTCGACAAGGTGAACAGGCCG | 1107 |
| cg13573587 | GCAGGGCAGCCAGCTACGAATTTTGAGCTCTGGGGCGCGCTCACCTGGCG | 1108 |
| cg03788610 | CGGGTCAAGAGCAGTGACCTAGTGGCCCTCAGCCCTGGATGGAGGACAGA | 1109 |
| cg03990195 | CGCCATTCTGTTGGTACTAAGGTGTTGTGGTTGGTAGAAGCTTAAGTGAG | 1110 |
| cg08147391 | CGGGTCGTGGTGACTACTAATGGGTATAAGATTTCTTTTTGAGATGATGG | 1111 |
| cg05548952 | TGTTGACCTCAGGTCCAGTTTGTTTGCAGCCTCCCTCTTCTGACAGATCG | 1112 |
| cg07929447 | TTGTCTCAGATTACAGGAACCAAATGCATGTCTGGCCTTGTGCTGCTTCG | 1113 |

**EXAMPLE 2 REFERENCES**

1. Dawber, T.R., Meadors, G.F. & Moore, F.E., Jr. Epidemiological approaches to heart disease: the Framingham Study. Am J Public Health Nations Health 41, 279-81 (1951).

2. Kannel, W.B., Feinleib, M., McNamara, P.M., Garrison, R.J. & Castelli, W.P. An investigation of coronary heart disease in families. The Framingham offspring study. Am J Epidemiol 110, 281-90 (1979).

3. Aryee, M.J. et al. Minfi: a flexible and comprehensive Bioconductor package for the analysis of Infinium DNA methylation microarrays. Bioinformatics 30, 1363-9 (2014).

4. Anonymous, A. Design of the Women's Health Initiative clinical trial and observational study. The Women's Health Initiative Study Group. Control Clin Trials 19, 61-109 (1998).

5. Anderson, G. et al. Implementation of the women's health initiative study design. Ann Epidemiol 13, S5-17 (2003).

6. Whitsel, E. Epigenetic Mechanisms of PM-Mediated CVD Risk (WHI-EMPC; R01-ES02836). in National Institutes of Health. U.S. Department of Health and Human Services. Research Portfolio Online Reporting Tools (2016).

7. Teschendorff, A.E. et al. A beta-mixture quantile normalization method for correcting probe design bias in Illumina Infinium 450 k DNA methylation data. Bioinformatics 29, 189-196 (2013).

8. Johnson, W.E., Rabinovic, A. & Li, C. Adjusting batch effects in microarray expression data using Empirical Bayes methods. Biostatistics 8, 118-127 (2007).

9. Patterson, R.E. et al. Measurement characteristics of the Women's Health Initiative food frequency questionnaire. Annals of epidemiology 9, 178-187 (1999).

10. Taylor, H.A., Jr. et al. Toward resolution of cardiovascular health disparities in African Americans: design and methods of the Jackson Heart Study. Ethn Dis 15, S6-4-17 (2005).

11. Moore, A.Z. et al. Change in Epigenome-Wide DNA Methylation Over 9 Years and Subsequent Mortality: Results From the InCHIANTI Study. J Gerontol A Biol Sci Med Sci (2015).

12. Wong, H.K., Cheung, T.T. & Cheung, B.M. Adrenomedullin and cardiovascular diseases. JRSM Cardiovasc Dis 1(2012).

13. Larrayoz, I.M. et al. Adrenomedullin Contributes to Age-Related Memory Loss in Mice and Is Elevated in Aging Human Brains. Front Mol Neurosci 10, 384 (2017).

14. Liabeuf, S. et al. Plasma beta-2 microglobulin is associated with cardiovascular disease in uremic patients. Kidney Int 82, 1297-303 (2012).

15. Smith, L.K. et al. beta2-microglobulin is a systemic pro-aging factor that impairs cognitive function and neurogenesis. Nat Med 21, 932-7 (2015).

16. Ferguson, T.W., Komenda, P. & Tangri, N. Cystatin C as a biomarker for estimating glomerular filtration rate. Curr Opin Nephrol Hypertens 24., 295-300 (2015).

17. van der Laan, S.W. et al. Cystatin C and Cardiovascular Disease: A Mendelian Randomization Study. J Am Coll Cardiol 68, 934-45 (2016).

18. Paterson, R.W. et al. Cerebrospinal fluid markers including trefoil factor 3 are associated with neurodegeneration in amyloid-positive individuals. Transl Psychiatry 4, e419 (2014).

19. Fujita, Y., Taniguchi, Y., Shinkai, S., Tanaka, M. & Ito, M. Secreted growth differentiation factor 15 as a potential biomarker for mitochondrial dysfunctions in aging and age-related disorders. Geriatr Gerontol Int 16 Suppl 1, 17-29 (2016).

20. Maioli, S. et al. Alterations in brain leptin signalling in spite of unchanged CSF leptin levels in Alzheimer's disease. Aging Cell 14, 122-9 (2015).

21. Cesari, M., Pahor, M. & Incalzi, R.A. Plasminogen activator inhibitor-1 (PAI-1): a key factor linking fibrinolysis and age-related subclinical and clinical conditions. Cardiovasc Ther 28, e72-91 (2010).

22. Ashutosh, Chao, C., Borgmann, K., Brew, K. & Ghorpade, A. Tissue inhibitor of metalloproteinases-1 protects human neurons from staurosporine and HIV-1-induced apoptosis: mechanisms and relevance to HIV-1-associated dementia. Cell Death Dis 3, e332 (2012).

23. Houseman, E. et al. DNA methylation arrays as surrogate measures of cell mixture distribution. BMC Bioinformatics 13, 86 (2012).

24. Horvath, S. DNA methylation age of human tissues and cell types. Genome Biol 14, R115 (2013).

25. Horvath, S. & Levine, A.J. HIV-1 Infection Accelerates Age According to the Epigenetic Clock. J Infect Dis 212, 1563-73 (2015).

26. Horvath, S. et al. An epigenetic clock analysis of race/ethnicity, sex, and coronary heart disease. Genome Biol 17, 171 (2016).

27. Hannum, G. et al. Genome-wide methylation profiles reveal quantitative views of human aging rates. Mol Cell 49, 359-67 (2013).

28. Levine, M.E. et al. An epigenetic biomarker of aging for lifespan and healthspan. Aging (Albany NY) 10, 573-591 (2018).

29. Zhang, Y. et al. DNA methylation signatures in peripheral blood strongly predict all-cause mortality. Nat Commun 8, 14617 (2017).

30. Horvath, S. & Levine, A.J. HIV-1 infection accelerates age according to the epigenetic clock. J Infect Dis (2015).

31. Langfelder, P. & Horvath, S. WGCNA: an R package for weighted correlation network analysis. BMC Bioinformatics 9, 559 (2008).

**Claims**

1. A method for predicting lifespan of an individual comprising obtaining information on physiological factors associated with lifespan of an individual comprising **(i)** information on the numbers of cigarettes smoked by an individual in their lifetime, and *in vivo* plasma levels of: **(ii)** tissue inhibitor metalloproteinases 1 (TIMP1); **(iii)** cystatin-C; **(iv)** beta-2-microglobulin (B2M); **(v)** growth differentiation factor 15 (GDF15); **(vi)** adrenomedullin (ADM); **(vii)** leptin; and **(viii)** plasminogen activator inhibitor 1 (PAI1);

   the method comprising obtaining genomic DNA from the individual; and

   i) observing methylation of the genomic DNA in 172 methylation markers from the group of methylation markers in SEQ ID NO: 1-SEQ ID NO: 172;
   correlating observed methylation in the 172 methylation markers with the number of years in which an individual has smoked in their lifetime, such that information on predicted lifespan of an individual is obtained;
   ii) observing methylation of the genomic DNA in 42 methylation markers from the group of methylation markers in SEQ ID NO: 173-SEQ ID NO: 214;
   correlating observed methylation in the 42 methylation markers with plasma protein levels of tissue inhibitor metalloproteinase 1 in an individual, such that information on plasma protein levels of tissue inhibitor metalloproteinase 1 in an individual is obtained;
   iii) observing methylation of the genomic DNA in 87 methylation markers from the group of methylation markers in SEQ ID NO: 215-SEQ ID NO: 301;
   correlating observed methylation in the 87 methylation markers with plasma protein levels of Cystatin-C in an individual, such that information on plasma protein levels of Cystatin-C in an individual is obtained;
   iv) observing methylation of the genomic DNA in 91 methylation markers from the group of methylation markers in SEQ ID NO: 302-SEQ ID NO: 392;
   correlating observed methylation in the 91 methylation markers with plasma protein levels of Beta-2-microglobulin in an individual, such that information on plasma protein levels of Beta-2-microglobulin in an individual is obtained;
   v) observing methylation of the genomic DNA in 137 methylation markers from the group of methylation markers in SEQ ID NO: 393-SEQ ID NO: 529;
   correlating observed methylation in the 137 methylation markers with plasma protein levels of growth differentiation factor 15 in an individual, such that information on plasma protein levels of growth differentiation factor 15 in an individual is obtained;
   vi) observing methylation of the genomic DNA in 186 methylation markers from the group of methylation markers in SEQ ID NO: 530-SEQ ID NO: 715;
   correlating observed methylation in the 186 methylation markers with plasma protein levels of adrenomedullin in an individual, such that information on plasma protein levels of adrenomedullin in an individual is obtained;
   vii) observing methylation of the genomic DNA in 187 methylation markers from the group of methylation markers in SEQ ID NO: 716-SEQ ID NO: 902;
   correlating observed methylation in the 187 methylation markers with Leptin in an individual, such that information on plasma protein levels of Leptin in an individual is obtained; and
   viii) observing methylation of the genomic DNA in 211 methylation markers from the group of methylation markers in SEQ ID NO: 903-SEQ ID NO: 1113;
   correlating observed methylation in the 211 methylation markers with plasminogen activator inhibitor 1 in an individual, such that information on plasma protein levels of plasminogen activator inhibitor 1 in an individual is obtained.

2. The method of claim 1 for the prediction of time to cardiovascular disease.

3. The method of claims 1 or 2, wherein genomic DNA is obtained from human fibroblasts, keratinocytes, buccal cells, endothelial cells, lymphoblastoid cells, and/or cells obtained from blood, skin, dermis, epidermis or saliva.

4. The method of any of claims 1 or 2, wherein methylation is observed by a process comprising treatment of genomic DNA from the population of cells from the individual with bisulfite to transform unmethylated cytosines of CpG dinucleotides in the genomic DNA to uracil.

5. The method of any of claims 1 to 3, wherein genomic DNA is hybridized to a complimentary sequence disposed on a microarray.

6. The method of any one of claims 1-5, wherein correlating observed methylation in the methylation markers comprises a regression analysis.

7. The method of any one of claims 1 to 5, wherein genomic DNA is amplified by a polymerase chain reaction process.

8. A method of observing the effects of a test agent on genomic methylation associated epigenetic aging of human cells, the method comprising:

    (a) combining the test agent with human cells;
    (b) observing methylation status in 172 methylation markers of SEQ ID NO: 1-SEQ ID NO: 172 in genomic DNA from the human cells;
    (c) comparing the observations from (b) with observations of the methylation status in 172 of methylation markers of SEQ ID NO: 1-SEQ ID NO: 172 in genomic DNA from control human cells not exposed to the test agent such that effects of the test agent on genomic methylation associated epigenetic aging in the human cells is observed.

9. The method of claim 8, wherein the method further comprises:

    observing methylation of the genomic DNA in 42 methylation markers from the group of methylation markers in SEQ ID NO: 173-SEQ ID NO: 214;
    observing methylation of the genomic DNA in 87 methylation markers from the group of methylation markers in SEQ ID NO: 215-SEQ ID NO: 301;
    observing methylation of the genomic DNA in 91 methylation markers from the group of methylation markers in SEQ ID NO: 302-SEQ ID NO: 392;
    observing methylation of the genomic DNA in 137 methylation markers from the group of methylation markers in SEQ ID NO: 393-SEQ ID NO: 529;
    observing methylation of the genomic DNA in 186 methylation markers from the group of methylation markers in SEQ ID NO: 530-SEQ ID NO: 715;
    observing methylation of the genomic DNA in 187 methylation markers from the group of methylation markers in SEQ ID NO: 716-SEQ ID NO: 902;
    observing methylation of the genomic DNA in 211 methylation markers from the group of methylation markers in SEQ ID NO: 903-SEQ ID NO: 1113; and/or
    observing methylation of the genomic DNA in 1113 methylation markers of SEQ ID NO: 1-SEQ ID NO: 1113.

10. The method of claim 8, wherein a plurality of test agents are combined with the human cells.

11. The method of claim 8, wherein the cells are primary keratinocytes from multiple donors.

12. The method of claim 8, wherein the method observes human cells *in vitro.*

13. The method of claim 8, wherein the test agent is a compound having a molecular weight less than 3,000, 2,000, 1,000 or 500 g/mol.

**Patentansprüche**

1. Ein Verfahren zum Vorhersagen der Lebensspanne eines Individuums, beinhaltend das Erhalten von Informationen über physiologische Faktoren, die mit der Lebensspanne eines Individuums assoziiert sind, beinhaltend (i) Informationen über die Anzahl von von einem Individuum in seiner Lebenszeit gerauchten Zigaretten und *In-vivo-*Plasmaspiegel von: **(ii)** Gewebeinhibitor-Metalloproteinasen 1 (Tissue Inhibitor Metalloproteinases 1, TIMP1); **(iii)** Cystatin-C; **(iv)** Beta-2-Mikroglobulin (B2M); **(v)** Wachstumsdifferenzierungsfaktor 15 (GDF15); **(vi)** Adrenomedullin (ADM); **(vii)** Leptin; und **(viii)** Plasminogen-Aktivator-Inhibitor 1 (PAI1);
wobei das Verfahren Folgendes beinhaltet: Erhalten von genomischer DNA von dem Individuum; und

**i)** Beobachten der Methylierung der genomischen DNA an 172 Methylierungsmarkern aus der Gruppe von Methylierungsmarkern in SEQ ID NO: 1 bis SEQ ID NO: 172;
Korrelieren der beobachteten Methylierung an den 172 Methylierungsmarkern mit der Anzahl von Jahren, in denen ein Individuum in seiner Lebenszeit geraucht hat, sodass Informationen über die vorhergesagte Lebensspanne eines Individuums erhalten werden;
**ii)** Beobachten der Methylierung der genomischen DNA an 42 Methylierungsmarkern aus der Gruppe von Methylierungsmarkern in SEQ ID NO: 173 bis SEQ ID NO: 214;
Korrelieren der beobachteten Methylierung an den 42 Methylierungsmarkern mit Plasmaproteinspiegeln von Gewebeinhibitor-Metalloproteinase 1 in einem Individuum, sodass Informationen über Plasmaproteinspiegel von Gewebeinhibitor-Metalloproteinase 1 in einem Individuum erhalten werden;
**iii)** Beobachten der Methylierung der genomischen DNA an 87 Methylierungsmarkern aus der Gruppe von Methylierungsmarkern in SEQ ID NO: 215 bis SEQ ID NO: 301;
Korrelieren der beobachteten Methylierung an den 87 Methylierungsmarkern mit Plasmaproteinspiegeln von Cystatin-C in einem Individuum, sodass Informationen über Plasmaproteinspiegel von Cystatin-C in einem Individuum erhalten werden;
**iv)** Beobachten der Methylierung der genomischen DNA an 91 Methylierungsmarkern aus der Gruppe von Methylierungsmarkern in SEQ ID NO: 302 bis SEQ ID NO: 392;
Korrelieren der beobachteten Methylierung an den 91 Methylierungsmarkern mit Plasmaproteinspiegeln von Beta-2-Mikroglobulin in einem Individuum, sodass Informationen über Plasmaproteinspiegel von Beta-2-Mikroglobulin in einem Individuum erhalten werden;
**v)** Beobachten der Methylierung der genomischen DNA an 137 Methylierungsmarkern aus der Gruppe von Methylierungsmarkern in SEQ ID NO: 393 bis SEQ ID NO: 529;
Korrelieren der beobachteten Methylierung an den 137 Methylierungsmarkern mit Plasmaproteinspiegeln von Wachstumsdifferenzierungsfaktor 15 in einem Individuum, sodass Informationen über Plasmaproteinspiegel von Wachstumsdifferenzierungsfaktor 15 in einem Individuum erhalten werden;
**vi)** Beobachten der Methylierung der genomischen DNA an 186 Methylierungsmarkern aus der Gruppe von Methylierungsmarkern in SEQ ID NO: 530 bis SEQ ID NO: 715;
Korrelieren der beobachteten Methylierung an den 186 Methylierungsmarkern mit Plasmaproteinspiegeln von Adrenomedullin in einem Individuum, sodass Informationen über Plasmaproteinspiegel von Adrenomedullin in einem Individuum erhalten werden;
**vii)** Beobachten der Methylierung der genomischen DNA an 187 Methylierungsmarkern aus der Gruppe von Methylierungsmarkern in SEQ ID NO: 716 bis SEQ ID NO: 902;
Korrelieren der beobachteten Methylierung an den 187 Methylierungsmarkern mit Leptin in einem Individuum, sodass Informationen über Plasmaproteinspiegel von Leptin in einem Individuum erhalten werden; und
**viii)** Beobachten der Methylierung der genomischen DNA an 211 Methylierungsmarkern aus der Gruppe von Methylierungsmarkern in SEQ ID NO: 903 bis SEQ ID NO: 1113;
Korrelieren der beobachteten Methylierung an den 211 Methylierungsmarkern mit Plasminogen-Aktivator-Inhibitor 1 in einem Individuum, sodass Informationen über Plasmaproteinspiegel von Plasminogen-Aktivator-Inhibitor 1 in einem Individuum erhalten werden.

2. Verfahren gemäß Anspruch 1 zur Vorhersage der Zeit bis zu einer Herz-Kreislauf-Erkrankung.

3. Verfahren gemäß Anspruch 1 oder 2, wobei genomische DNA aus menschlichen Fibroblasten, Keratinozyten, bukkalen Zellen, Endothelzellen, lymphoblastoiden Zellen und/oder aus aus Blut, Haut, Dermis, Epidermis oder Speichel erhaltenen Zellen erhalten wird.

4. Verfahren gemäß einem der Ansprüche 1 oder 2, wobei die Methylierung durch einen Prozess beobachtet wird, der die Behandlung von genomischer DNA aus der Population von Zellen von dem Individuum mit Bisulfit beinhaltet, um unmethylierte Cytosine von CpG-Dinukleotiden in der genomischen DNA in Uracil umzuwandeln.

5. Verfahren gemäß einem der Ansprüche 1 bis 3, wobei genomische DNA an eine komplementäre Sequenz hybridisiert wird, die auf einem Mikroarray angeordnet ist.

6. Verfahren gemäß einem der Ansprüche 1-5, wobei das Korrelieren der beobachteten Methylierung an den Methylierungsmarkern eine Regressionsanalyse beinhaltet.

7. Verfahren gemäß einem der Ansprüche 1 bis 5, wobei genomische DNA durch einen Polymerasekettenreaktionsprozess amplifiziert wird.

8. Ein Verfahren zum Beobachten der Wirkungen eines Testmittels auf die genomische Methylierung, die mit epigenetischer Alterung von menschlichen Zellen assoziiert ist, wobei das Verfahren Folgendes beinhaltet:

(a) Kombinieren des Testmittels mit menschlichen Zellen;
(b) Beobachten des Methylierungsstatus an 172 Methylierungsmarkern von SEQ ID NO: 1 bis SEQ ID NO: 172 in genomischer DNA von den menschlichen Zellen;
(c) Vergleichen der Beobachtungen aus (b) mit Beobachtungen des Methylierungsstatus an 172 Methylierungsmarkern von SEQ ID NO: 1 bis SEQ ID NO: 172 in genomischer DNA von menschlichen Kontrollzellen, die nicht dem Testmittel ausgesetzt waren, sodass Wirkungen des Testmittels auf die genomische Methylierung, die mit epigenetischer Alterung in den menschlichen Zellen assoziiert ist, beobachtet werden.

9. Verfahren gemäß Anspruch 8, wobei das Verfahren ferner Folgendes beinhaltet:

Beobachten der Methylierung der genomischen DNA an 42 Methylierungsmarkern aus der Gruppe von Methylierungsmarkern in SEQ ID NO: 173 bis SEQ ID NO: 214;
Beobachten der Methylierung der genomischen DNA an 87 Methylierungsmarkern aus der Gruppe von Methylierungsmarkern in SEQ ID NO: 215 bis SEQ ID NO: 301;
Beobachten der Methylierung der genomischen DNA an 91 Methylierungsmarkern aus der Gruppe von Methylierungsmarkern in SEQ ID NO: 302 bis SEQ ID NO: 392;
Beobachten der Methylierung der genomischen DNA an 137 Methylierungsmarkern aus der Gruppe von Methylierungsmarkern in SEQ ID NO: 393 bis SEQ ID NO: 529;
Beobachten der Methylierung der genomischen DNA an 186 Methylierungsmarkern aus der Gruppe von Methylierungsmarkern in SEQ ID NO: 530 bis SEQ ID NO: 715;
Beobachten der Methylierung der genomischen DNA an 187 Methylierungsmarkern aus der Gruppe von Methylierungsmarkern in SEQ ID NO: 716 bis SEQ ID NO: 902;
Beobachten der Methylierung der genomischen DNA an 211 Methylierungsmarkern aus der Gruppe von Methylierungsmarkern in SEQ ID NO: 903 bis SEQ ID NO: 1113; und/oder
Beobachten der Methylierung der genomischen DNA an 1113 Methylierungsmarkern von SEQ ID NO: 1 bis SEQ ID NO: 1113.

10. Verfahren gemäß Anspruch 8, wobei eine Vielzahl von Testmitteln mit den menschlichen Zellen kombiniert wird.

11. Verfahren gemäß Anspruch 8, wobei die Zellen primäre Keratinozyten von mehreren Spendern sind.

12. Verfahren gemäß Anspruch 8, wobei das Verfahren menschliche Zellen *in vitro* beobachtet.

13. Verfahren gemäß Anspruch 8, wobei das Testmittel eine Verbindung mit einem Molekulargewicht von weniger als 3000, 2000, 1000 oder 500 g/mol ist.

**Revendications**

1. Un procédé de prédiction de la durée de vie d'un individu comprenant l'obtention d'informations sur des facteurs physiologiques associés à la durée de vie d'un individu comprenant **(i)** d'informations sur le nombre de cigarettes fumées par un individu dans leur durée de vie, et les niveaux plasmatiques in vivo de : **(ii)** métalloprotéinases d'inhibiteur tissulaire 1 (Tissue Inhibitor Metalloproteinases 1, TIMP1) ; **(iii)** cystatine-C ; **(iv)** bêta-2-microglobuline (B2M) ; **(v)** facteur de croissance et de différenciation 15 (GDF15) ; **(vi)** adrénomédulline (ADM) ; **(vii)** leptine ; et **(viii)** inhibiteur de l'activateur du plasminogène 1 (PAI1) ;
le procédé comprenant l'obtention d'ADN génomique à partir de l'individu ; et

**i)** l'observation de la méthylation de l'ADN génomique dans 172 marqueurs de méthylation provenant du groupe de marqueurs de méthylation dans SEQ ID NO : 1-SEQ ID NO : 172 ;
la corrélation de la méthylation observée dans les 172 marqueurs de méthylation avec le nombre d'années pendant lesquelles un individu a fumé dans sa durée de vie, de sorte que des informations sur la durée de vie prédite d'un individu soient obtenues ;

**ii)** l'observation de la méthylation de l'ADN génomique dans 42 marqueurs de méthylation provenant du groupe de marqueurs de méthylation dans SEQ ID NO : 173-SEQ ID NO : 214 ;

la corrélation de la méthylation observée dans les 42 marqueurs de méthylation avec les niveaux de protéine plasmatique de la métalloprotéinase d'inhibiteur tissulaire 1 chez un individu, de sorte que des informations sur les niveaux de protéine plasmatique de la métalloprotéinase d'inhibiteur tissulaire 1 chez un individu soient obtenues ;

**iii)** l'observation de la méthylation de l'ADN génomique dans 87 marqueurs de méthylation provenant du groupe de marqueurs de méthylation dans SEQ ID NO : 215-SEQ ID NO : 301 ;

la corrélation de la méthylation observée dans les 87 marqueurs de méthylation avec les niveaux de protéine plasmatique de la cystatine-C chez un individu, de sorte que des informations sur les niveaux de protéine plasmatique de la cystatine-C chez un individu soient obtenues ;

**iv)** l'observation de la méthylation de l'ADN génomique dans 91 marqueurs de méthylation provenant du groupe de marqueurs de méthylation dans SEQ ID NO : 302-SEQ ID NO : 392 ;

la corrélation de la méthylation observée dans les 91 marqueurs de méthylation avec les niveaux de protéine plasmatique de la bêta-2-microglobuline chez un individu, de sorte que des informations sur les niveaux de protéine plasmatique de la bêta-2-microglobuline chez un individu soient obtenues ;

**v)** l'observation de la méthylation de l'ADN génomique dans 137 marqueurs de méthylation provenant du groupe de marqueurs de méthylation dans SEQ ID NO : 393-SEQ ID NO : 529 ;

la corrélation de la méthylation observée dans les 137 marqueurs de méthylation avec les niveaux de protéine plasmatique du facteur de croissance et de différenciation 15 chez un individu, de sorte que des informations sur les niveaux de protéine plasmatique du facteur de croissance et de différenciation 15 chez un individu soient obtenues ;

**vi)** l'observation de la méthylation de l'ADN génomique dans 186 marqueurs de méthylation provenant du groupe de marqueurs de méthylation dans SEQ ID NO : 530-SEQ ID NO : 715 ;

la corrélation de la méthylation observée dans les 186 marqueurs de méthylation avec les niveaux de protéine plasmatique de l'adrénomédulline chez un individu, de sorte que des informations sur les niveaux de protéine plasmatique de l'adrénomédulline chez un individu soient obtenues ;

**vii)** l'observation de la méthylation de l'ADN génomique dans 187 marqueurs de méthylation provenant du groupe de marqueurs de méthylation dans SEQ ID NO : 716-SEQ ID NO : 902 ;

la corrélation de la méthylation observée dans les 187 marqueurs de méthylation avec la Leptine chez un individu, de sorte que des informations sur les niveaux de protéine plasmatique de la Leptine chez un individu soient obtenues ; et

**viii)** l'observation de la méthylation de l'ADN génomique dans 211 marqueurs de méthylation provenant du groupe de marqueurs de méthylation dans SEQ ID NO : 903-SEQ ID NO : 1113 ;

la corrélation de la méthylation observée dans les 211 marqueurs de méthylation avec l'inhibiteur de l'activateur du plasminogène 1 chez un individu, de sorte que des informations sur les niveaux de protéine plasmatique de l'inhibiteur de l'activateur du plasminogène 1 chez un individu soient obtenues.

**2.** Le procédé de la revendication 1 pour la prédiction du temps jusqu'à une maladie cardiovasculaire.

**3.** Le procédé des revendications 1 ou 2, dans lequel l'ADN génomique est obtenu à partir de fibroblastes humains, de kératinocytes, de cellules buccales, de cellules endothéliales, de cellules lymphoblastoïdes, et/ou de cellules obtenues à partir de sang, de peau, de derme, d'épiderme ou de salive.

**4.** Le procédé de l'une quelconque des revendications 1 ou 2, dans lequel la méthylation est observé par un processus comprenant le traitement de l'ADN génomique de la population de cellules de l'individu avec du bisulfite pour transformer des cytosines non méthylées de dinucléotides CpG dans l'ADN génomique en uracile.

**5.** Le procédé de l'une quelconque des revendications 1 à 3, dans lequel l'ADN génomique est hybridé à une séquence complémentaire disposée sur une biopuce.

**6.** Le procédé de l'une quelconque des revendications 1 à 5, dans lequel la corrélation de la méthylation observée dans les marqueurs de méthylation comprend une analyse de régression.

**7.** Le procédé de l'une quelconque des revendications 1 à 5, dans lequel l'ADN génomique est amplifié par un processus de réaction en chaîne par polymérase.

8. Un procédé d'observation des effets d'un agent de test sur le vieillissement épigénétique de cellules humaines associé à la méthylation génomique, le procédé comprenant :

(a) la combinaison de l'agent de test avec des cellules humaines ;
(b) l'observation de l'état de méthylation dans 172 marqueurs de méthylation de SEQ ID NO : 1-SEQ ID NO : 172 dans l'ADN génomique provenant des cellules humaines ;
(c) la comparaison des observations de (b) avec des observations de l'état de méthylation dans 172 des marqueurs de méthylation de SEQ ID NO : 1-SEQ ID NO : 172 dans l'ADN génomique provenant de cellules humaines témoins non exposées à l'agent de test de sorte que les effets de l'agent de test sur le vieillissement épigénétique dans les cellules humaines associé à la méthylation génomique soient observés.

9. Le procédé de la revendication 8, dans lequel le procédé comprend en outre ;

l'observation de la méthylation de l'ADN génomique dans 42 marqueurs de méthylation provenant du groupe de marqueurs de méthylation dans SEQ ID NO : 173-SEQ ID NO : 214 ;
l'observation de la méthylation de l'ADN génomique dans 87 marqueurs de méthylation provenant du groupe de marqueurs de méthylation dans SEQ ID NO : 215-SEQ ID NO : 301 ;
l'observation de la méthylation de l'ADN génomique dans 91 marqueurs de méthylation provenant du groupe de marqueurs de méthylation dans SEQ ID NO : 302-SEQ ID NO : 392 ;
l'observation de la méthylation de l'ADN génomique dans 137 marqueurs de méthylation provenant du groupe de marqueurs de méthylation dans SEQ ID NO : 393-SEQ ID NO : 529 ;
l'observation de la méthylation de l'ADN génomique dans 186 marqueurs de méthylation provenant du groupe de marqueurs de méthylation dans SEQ ID NO : 530-SEQ ID NO : 715 ;
l'observation de la méthylation de l'ADN génomique dans 187 marqueurs de méthylation provenant du groupe de marqueurs de méthylation dans SEQ ID NO : 716-SEQ ID NO : 902 ;
l'observation de la méthylation de l'ADN génomique dans 211 marqueurs de méthylation provenant du groupe de marqueurs de méthylation dans SEQ ID NO : 903-SEQ ID NO : 1113 ; et/ou
l'observation de la méthylation de l'ADN génomique dans 1113 marqueurs de méthylation de SEQ ID NO : 1-SEQ ID NO : 1113.

10. Le procédé de la revendication 8, dans lequel une pluralité d'agents de test sont combinés avec les cellules humaines.

11. Le procédé de la revendication 8, dans lequel les cellules sont des kératinocytes primaires provenant de donneurs multiples.

12. Le procédé de la revendication 8, dans lequel le procédé observe des cellules humaines in vitro.

13. Le procédé de la revendication 8, dans lequel l'agent de test est un composé ayant un poids moléculaire inférieur à 3 000, 2 000, 1 000 ou 500 g/mol.

FIGURE 1

FIGURE 2

FIGURE 3

FIGURE 4

FIGURE 5

| | | n | AgeAccelGrim bicor | p | adj.DNAm GDF-15 bicor | p | adj.DNAm B2M bicor | p | adj.DNAm Cystatin C bicor | p | adj.DNAm TIMP-1 bicor | p | adj.DNAm ADM bicor | p | adj.DNAm PAI-1 bicor | p | adj.DNAm Leptin bicor | p | adj.DNAm PACKYRS bicor | p |
|---|---|---|---|---|---|---|---|---|---|---|---|---|---|---|---|---|---|---|---|---|
| Diet | log2(Total energy) | 3700 | -0.02 | 0.15 | -0.02 | 0.27 | -0.03 | 0.06 | -0.04 | 0.02 | -0.02 | 0.35 | 0.01 | 0.69 | 0.03 | 0.05 | 0.01 | 0.55 | -0.05 | 3E-3 |
| | Carbohydrate | 3700 | -0.13 | 4E-13 | -0.03 | 0.07 | -0.01 | 0.38 | 0.02 | 0.26 | -0.03 | 0.12 | -0.06 | 5E-4 | -0.15 | 2E-20 | -0.08 | 5E-8 | -0.08 | 2E-6 |
| | Protein | 3700 | -0.01 | 0.39 | 0.00 | 0.96 | 0.00 | 0.91 | 0.00 | 0.85 | 0.01 | 0.63 | 0.02 | 0.22 | 0.02 | 0.34 | 0.02 | 0.30 | -0.02 | 0.21 |
| | Fat | 3700 | 0.09 | 2E-8 | 0.02 | 0.18 | 0.03 | 0.05 | -0.02 | 0.16 | 0.03 | 0.08 | 0.03 | 0.08 | 0.13 | 1E-14 | 0.09 | 2E-8 | 0.05 | 0.01 |
| | log2(1+Red meat) | 3700 | 0.06 | 4E-4 | 0.01 | 0.46 | 0.02 | 0.28 | -0.02 | 0.20 | 0.03 | 0.07 | -0.05 | 4E-3 | 0.12 | 4E-13 | 0.07 | 4E-4 | 0.00 | 0.92 |
| | log2(1+Poultry) | 3700 | 0.03 | 0.08 | 0.04 | 0.02 | -0.03 | 0.09 | 0.00 | 0.79 | 0.01 | 0.48 | -0.01 | 0.41 | -0.01 | 0.54 | 0.00 | 0.89 | 0.05 | 3E-3 |
| | log2(1+Fish) | 3700 | 0.00 | 0.87 | -0.02 | 0.13 | -0.03 | 0.05 | -0.05 | 4E-3 | 0.01 | 0.64 | -0.03 | 0.11 | 0.01 | 0.47 | 0.01 | 0.69 | 0.01 | 0.42 |
| | log2(1+Dairy) | 3700 | -0.09 | 1E-7 | -0.06 | 2E-4 | -0.04 | 0.02 | -0.06 | 3E-4 | -0.05 | 2E-3 | 0.02 | 0.34 | -0.01 | 0.46 | 0.00 | 0.86 | -0.11 | 6E-11 |
| | log2(1+Whole grains) | 3700 | -0.07 | 2E-5 | -0.03 | 0.12 | -0.03 | 0.07 | -0.03 | 0.05 | -0.03 | 0.10 | -0.03 | 0.05 | -0.04 | 0.03 | -0.01 | 0.54 | -0.08 | 5E-4 |
| | log2(1+Nuts) | 3700 | -0.02 | 0.15 | 0.05 | 3E-3 | 0.03 | 0.07 | 0.09 | 2E-8 | -0.02 | 0.19 | 0.01 | 0.38 | 0.01 | 0.75 | 0.02 | 0.29 | 0.02 | 0.13 |
| | log2(Fruits) | 3700 | -0.10 | 3E-8 | -0.03 | 0.05 | -0.04 | 0.02 | -0.01 | 0.51 | -0.03 | 0.04 | -0.03 | 0.06 | -0.04 | 0.01 | 0.04 | 0.01 | -0.09 | 2E-8 |
| | log2(Vegetables) | 3700 | -0.08 | 7E-7 | -0.03 | 0.04 | -0.04 | 0.02 | -0.07 | 4E-5 | -0.04 | 0.03 | -0.02 | 0.14 | -0.03 | 0.10 | 0.00 | 0.84 | -0.06 | 5E-4 |
| Dietary biomarkers | Retinol | 2267 | -0.01 | 0.49 | -0.02 | 0.37 | 0.00 | 0.95 | -0.05 | 0.01 | -0.03 | 0.17 | 0.04 | 0.04 | 0.11 | 5E-7 | 0.00 | 0.94 | -0.02 | 0.27 |
| | Mean carotenoids | 2266 | -0.22 | 3E-26 | -0.13 | 3E-10 | -0.16 | 3E-15 | -0.16 | 9E-16 | -0.17 | 4E-16 | -0.12 | 3E-9 | -0.14 | 2E-11 | -0.15 | 3E-13 | -0.13 | 4E-10 |
| | Lycopene | 2267 | -0.07 | 6E-4 | 0.06 | 0.01 | -0.11 | 3E-8 | -0.04 | 0.07 | -0.13 | 1E-10 | -0.06 | 2E-3 | -0.08 | 2E-4 | -0.04 | 0.03 | -0.03 | 0.21 |
| | log2(alpha-Carotene) | 2267 | -0.22 | 4E-26 | -0.13 | 3E-10 | -0.12 | 6E-10 | -0.14 | 4E-12 | -0.16 | 8E-16 | -0.15 | 3E-13 | -0.14 | 4E-11 | -0.13 | 3E-11 | -0.08 | 2E-5 |
| | log2(beta-Carotene) | 2266 | -0.22 | 3E-26 | -0.16 | 5E-14 | -0.12 | 8E-10 | -0.14 | 3E-12 | -0.17 | 8E-16 | -0.16 | 1E-15 | -0.15 | 4E-13 | -0.13 | 2E-12 | -0.09 | 4E-5 |
| | log2(Lutein+Zeaxanthin) | 2267 | -0.14 | 3E-12 | -0.10 | 2E-7 | -0.14 | 4E-14 | -0.13 | 3E-10 | -0.17 | 1E-16 | -0.16 | 3E-15 | -0.16 | 4E-14 | -0.11 | 3E-7 | -0.02 | 0.30 |
| | log2(beta-Cryptoxanthin) | 2267 | -0.15 | 2E-13 | -0.08 | 2E-5 | -0.12 | 3E-8 | -0.08 | 1E-5 | -0.13 | 4E-11 | -0.12 | 3E-9 | -0.11 | 1E-8 | -0.11 | 3E-8 | -0.11 | 6E-8 |
| | log2(alpha-Tocopherol) | 2267 | 0.06 | 3E-3 | -0.01 | 0.65 | 0.01 | 0.53 | 0.07 | 1E-3 | 0.01 | 0.76 | 0.03 | 0.21 | 0.10 | 9E-7 | -0.02 | 0.30 | -0.03 | 0.11 |
| | log2(gamma-Tocopherol) | 2267 | 0.14 | 2E-11 | 0.02 | 0.25 | 0.00 | 0.98 | 0.08 | 4E-5 | 0.02 | 0.25 | 0.04 | 0.03 | 0.11 | 4E-7 | 0.08 | 1E-4 | 0.05 | 0.02 |
| Measurements | log2(C-reactive protein) | 2809 | 0.14 | 3E-22 | 0.11 | 2E-8 | 0.14 | 1E-13 | 0.17 | 3E-20 | 0.11 | 3E-10 | 0.14 | 2E-9 | 0.14 | 1E-14 | 0.12 | 2E-12 | 0.14 | 5E-4 |
| | log2(Insulin) | 4042 | 0.16 | 3E-25 | 0.07 | 2E-5 | 0.13 | 6E-16 | 0.10 | 7E-10 | 0.14 | 3E-20 | 0.13 | 4E-16 | 0.16 | 1E-24 | 0.15 | 7E-22 | 0.04 | 0.01 |
| | log2(Glucose) | 4144 | 0.12 | 6E-14 | 0.02 | 0.12 | 0.03 | 0.02 | 0.07 | 4E-5 | 0.06 | 2E-3 | 0.07 | 4E-5 | 0.14 | 2E-18 | 0.09 | 1E-8 | 0.08 | 5E-8 |
| | log2(Triglyceride) | 4148 | 0.11 | 5E-12 | 0.02 | 0.10 | 0.07 | 8E-6 | 0.02 | 0.10 | 0.06 | 3E-4 | 0.12 | 4E-16 | 0.13 | 1E-20 | 0.11 | 2E-13 | -0.03 | 0.07 |
| | Total cholesterol | 4148 | 0.01 | 0.65 | 0.01 | 0.69 | 0.10 | 7E-11 | 0.06 | 1E-4 | 0.10 | 3E-11 | -0.02 | 0.15 | -0.01 | 0.56 | -0.05 | 2E-3 | -0.07 | 2E-5 |
| | LDL cholesterol | 4084 | 0.00 | 0.83 | 0.03 | 0.03 | 0.12 | 4E-14 | 0.13 | 1E-16 | 0.13 | 4E-18 | -0.06 | 7E-5 | 0.10 | 1E-11 | -0.08 | 2E-6 | -0.12 | 1E-14 |
| | HDL cholesterol | 4145 | -0.10 | 3E-10 | -0.06 | 5E-3 | -0.05 | 3E-3 | -0.07 | 3E-6 | -0.01 | 0.43 | -0.08 | 1E-6 | -0.11 | 3E-11 | -0.03 | 0.07 | -0.08 | 3E-6 |
| | log2(Creatinine) | 2748 | 0.03 | 0.07 | 0.04 | 0.04 | 0.03 | 0.10 | 0.03 | 0.15 | 0.04 | 0.02 | -0.01 | 0.64 | -0.06 | 3E-3 | 0.02 | 0.32 | 0.04 | 0.02 |
| | Systolic blood pressure | 4177 | 0.07 | 3E-5 | 0.01 | 0.51 | 0.03 | 0.03 | 0.09 | 1E-8 | 0.06 | 2E-4 | 0.05 | 1E-3 | 0.07 | 3E-5 | 0.02 | 0.17 | 0.05 | 1E-5 |
| | Diastolic blood pressure | 4178 | -0.01 | 0.36 | -0.04 | 0.01 | -0.02 | 0.14 | 0.07 | 3E-5 | -0.03 | 0.10 | 0.03 | 0.03 | -0.01 | 0.55 | -0.04 | 0.02 | 0.00 | 0.88 |
| | BMI | 4145 | 0.14 | 3E-20 | 0.05 | 7E-4 | 0.06 | 5E-4 | 0.11 | 3E-11 | 0.17 | 1E-23 | 0.12 | 1E-14 | 0.12 | 2E-15 | 0.15 | 3E-18 | 0.04 | 0.01 |
| | log2(Waist/hip ratio) | 4037 | 0.19 | 3E-34 | 0.10 | 2E-11 | 0.09 | 2E-9 | 0.11 | 1E-12 | 0.12 | 3E-16 | 0.12 | 3E-13 | 0.15 | 2E-23 | 0.12 | 8E-14 | 0.12 | 1E-14 |
| Life style | Education | 4143 | -0.09 | 2E-8 | -0.09 | 1E-7 | -0.06 | 8E-4 | -0.11 | 1E-12 | -0.03 | 0.02 | -0.05 | 1E-3 | -0.03 | 0.10 | -0.01 | 0.42 | -0.07 | 1E-5 |
| | Income | 4054 | -0.07 | 2E-4 | -0.03 | 0.07 | -0.04 | 0.02 | -0.04 | 0.02 | -0.04 | 0.02 | -0.04 | 0.02 | -0.02 | 0.31 | -0.02 | 0.22 | -0.07 | 2E-5 |
| | log2(1+Exercise) | 3814 | -0.10 | 3E-10 | -0.05 | 6E-4 | -0.03 | 0.06 | -0.06 | 3E-3 | -0.06 | 5E-4 | -0.05 | 7E-4 | -0.08 | 1E-7 | -0.06 | 4E-4 | -0.05 | 3E-3 |
| | Current smoker | 2321 | 0.23 | 3E-13 | 0.19 | 1E-21 | -0.01 | 0.67 | 0.12 | 2E-9 | 0.01 | 0.59 | 0.04 | 0.05 | 0.01 | 0.59 | 0.00 | 0.87 | 0.23 | 1E-106 |
| | log2(1+Alcohol) | 3700 | -0.04 | 0.02 | -0.04 | 0.02 | 0.06 | 8E-4 | -0.04 | 0.01 | -0.03 | 0.08 | -0.02 | 0.35 | -0.01 | 0.49 | -0.01 | 0.44 | -0.02 | 0.26 |

FIGURE 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019034829 W
- US 20150259742 A
- US 025185 A
- US 119145 A
- US 6214556 B
- US 5786146 B
- US 6017704 A
- US 6265171 B
- US 6200756 B
- US 6251594 B
- US 5912147 A
- US 6331393 B
- US 6605432 B
- US 6300071 B

- US 20030148327
- US 20030148326 A
- US 20030143606 A
- US 20030082609 A
- US 20050009059 A
- US 058566 A
- US 4683202 A
- US 4683195 A
- US 4800159 A
- US 4965188 A
- US 5333675 A
- US 6300070 B
- WO 04096825 A1

**Non-patent literature cited in the description**

- **HORVATH S.** DNA methylation age of human tissues and cell types.. *Genome Biol.*, 2013, vol. 14 (R115)
- **XU GAO et al.** DNA methylation changes of whole blood cells in response to active smoking exposure in adults: a systematic review of DNA methylation studies. *CLINICAL EPIGENETICS*, 16 October 2015, vol. 7 (1), 113
- **LU et al.** *Aging (Albany NY).*, 21 January 2019, vol. 11 (2), 303-327
- **HANNUM et al.** Genome-Wide Methylation Profiles Reveal Quantitative Views Of Human Aging Rates. *Molecular Cell.*, 2013, vol. 49 (2), 359-367
- Technical Note: Epigenetics. CpG Loci Identification ILLUMINA Inc., 2010
- **OAKELEY, E. J.** *Pharmacology & Therapeutics*, 1999, vol. 84, 389-400
- PCR Technology: Principles and Applications for DNA Amplification. Freeman Press, 1992
- PCR Protocols: A Guide to Methods and Applications. Academic Press, 1990
- **MATTILA et al.** Nucleic Acids Res.. 1991, vol. 19, 4967
- **ECKERT et al.** *PCR Methods and Applications*, 1991, vol. 1, 17
- PCR. IRL Press
- **OLEK et al.** *Nuc. Acids Res.*, 1994, vol. 24, 5064-6
- **SYVANEN**. *Nature Rev. Gen.*, 2001, vol. 2, 930-942

- **HORVATH S**. DNA methylation age of human tissues and cell types.. *Genome Biol*, 2013, 14
- **HANNUM G** ; **GUINNEY J** ; **ZHAO L** ; **ZHANG L** ; **HUGHES G** ; **SADDA S** ; **KLOTZLE B** ; **BIBIKOVA M** ; **FAN JB** ; **GAO Y et al.** Genome-wide methylation profiles reveal quantitative views of human aging rates.. *Mol Cell*, 2013, vol. 49, 359-367
- **LEVINE ME** ; **LU AT** ; **QUACH A** ; **CHEN BH** ; **ASSIMES TL** ; **BANDINELLI S** ; **HOU L** ; **BACCARELLI AA** ; **STEWART JD** ; **LI Y et al.** An epigenetic biomarker of aging for lifespan and healthspan.. *Aging (Albany NY)*, 2018
- **ZHANG Y** ; **WILSON R** ; **HEISS J** ; **BREITLING LP** ; **SAUM KU** ; **SCHOTTKER B** ; **HOLLECZEK B** ; **WALDENBERGER M** ; **PETERS A** ; **BRENNER H**. DNA methylation signatures in peripheral blood strongly predict all-cause mortality.. *Nat Commun*, 2017, vol. 8, 14617
- **DURSO DF** ; **BACALINI MG** ; **SALA C** ; **PIRAZZINI C** ; **MARASCO E** ; **BONAFÉ M** ; **DO VALLE ÍF** ; **GENTILINI D** ; **CASTELLANI G** ; **FARIA AMC et al.** Acceleration of leukocytes' epigenetic age as an early tumor and sex-specific marker of breast and colorectal cancer.. *Oncotarget*, 2017, vol. 8, 23237-23245
- **LIN Q** ; **WEIDNER CI** ; **COSTA IG** ; **MARIONI RE** ; **FERREIRA MR** ; **DEARY IJ** ; **WAGNER W**. DNA methylation levels at individual age-associated CpG sites can be indicative for life expectancy.. *Aging (Albany NY)*, 2016, vol. 8, 394-401

- **MARIONI R** ; **SHAH S** ; **MCRAE A** ; **CHEN B** ; **COLICINO E** ; **HARRIS S** ; **GIBSON J** ; **HENDERS A** ; **REDMOND P** ; **COX S et al.** DNA methylation age of blood predicts all-cause mortality in later life.. *Genome Biol*, 2015, vol. 16, 25
- **PERNA L** ; **ZHANG Y** ; **MONS U** ; **HOLLECZEK B** ; **SAUM KU** ; **BRENNER H.** Epigenetic age acceleration predicts cancer, cardiovascular, and all-cause mortality in a German case cohort.. *Clin Epigenetics*, 2016, vol. 8, 64
- **CHEN BH** ; **MARIONI RE** ; **COLICINO E** ; **PETERS MJ** ; **WARD-CAVINESS CK** ; **TSAI PC** ; **ROETKER NS** ; **JUST AC** ; **DEMERATH EW** ; **GUAN W et al.** DNA methylation-based measures of biological age: meta-analysis predicting time to death.. *Aging (Albany NY)*, 2016, vol. 8, 1844-1865
- **HORVATH S** ; **RAJ K.** DNA methylation-based biomarkers and the epigenetic clock theory of ageing.. *Nat Rev Genet*, 2018, vol. 19, 371-384
- **ZHENG SC** ; **WIDSCHWENDTER M** ; **TESCHEN-DORFF AE.** Epigenetic drift, epigenetic clocks and cancer risk.. *Epigenomics*, 2016, vol. 8, 705-719
- **JUNG M** ; **PFEIFER GP.** Aging and DNA methylation.. *BMC Biology*, 2015, vol. 13, 1-8
- **NWANAJI-ENWEREM JC** ; **WEISSKOPF MG** ; **BACCARELLI AA.** Multi-tissue DNA methylation age: Molecular relationships and perspectives for advancing biomarker utility.. *Ageing Res Rev*, 2018, vol. 45, 15-23
- **IGNJATOVIC V** ; **LAI C** ; **SUMMERHAYES R** ; **MATHESIUS U** ; **TAWFILIS S** ; **PERUGINI MA** ; **MONAGLE P.** Age-related differences in plasma proteins: how plasma proteins change from neonates to adults.. *PLoS One*, 2011, vol. 6, e17213
- **RIDKER PM** ; **BURING JE** ; **COOK NR** ; **RIFAI N.** C-reactive protein, the metabolic syndrome, and risk of incident cardiovascular events an 8-year follow-up of 14 719 initially healthy American women.. *Circulation*, 2003, vol. 107, 391-397
- **DAWBER TR** ; **MEADORS GF** ; **MOORE FE, JR.** Epidemiological approaches to heart disease: the Framingham Study.. *Am J Public Health Nations Health*, 1951, vol. 41, 279-281
- **CONSIDINE RV** ; **SINHA MK** ; **HEIMAN ML** ; **KRIAUCIUNAS A** ; **STEPHENS TW** ; **NYCE MR** ; **OHANNESIAN JP** ; **MARCO CC** ; **MCKEE LJ** ; **BAUER TL et al.** Serum immunoreactive-leptin concentrations in normal-weight and obese humans.. *N Engl J Med*, 1996, vol. 334, 292-295
- **ROSENBAUM M** ; **NICOLSON M** ; **HIRSCH J** ; **HEYMSFIELD SB** ; **GALLAGHER D** ; **CHU F** ; **LEIBEL RL.** Effects of gender, body composition, and menopause on plasma concentrations of leptin.. *J Clin Endocrinol Metab*, 1996, vol. 81, 3424-3427
- **GAO X** ; **JIA M** ; **ZHANG Y** ; **BREITLING LP** ; **BRENNER H.** DNA methylation changes of whole blood cells in response to active smoking exposure in adults: a systematic review of DNA methylation studies.. *Clin Epigenetics*, 2015, vol. 7, 113
- **HORVATH S** ; **GURVEN M** ; **LEVINE ME** ; **TRUMBLE BC** ; **KAPLAN H** ; **ALLAYEE H** ; **RITZ BR** ; **CHEN B** ; **LU AT** ; **RICKABAUGH TM et al.** An epigenetic clock analysis of race/ethnicity, sex, and coronary heart disease.. *Genome Biol*, 2016, vol. 17, 171
- **HOUSEMAN E** ; **ACCOMANDO W** ; **KOESTLER D** ; **CHRISTENSEN B** ; **MARSIT C** ; **NELSON H** ; **WIENCKE J** ; **KELSEY K.** DNA methylation arrays as surrogate measures of cell mixture distribution.. *BMC Bioinformatics*, 2012, vol. 13, 86
- **QUACH A** ; **LEVINE ME** ; **TANAKA T** ; **LU AT** ; **CHEN BH** ; **FERRUCCI L** ; **RITZ B** ; **BANDINELLI S** ; **NEUHOUSER ML** ; **BEASLEY JM et al.** Epigenetic clock analysis of diet, exercise, education, and lifestyle factors.. *Aging (Albany NY)*, 2017
- **JYLHAVA J** ; **PEDERSEN NL** ; **HAGG S.** Biological Age Predictors.. *EBioMedicine*, 2017, vol. 21, 29-36
- **WONG HK** ; **CHEUNG TT** ; **CHEUNG BM.** Adreno-medullin and cardiovascular diseases.. *JRSM Cardiovasc Dis*, 2012, 1
- **LIABEUF S** ; **LENGLET A** ; **DESJARDINS L** ; **NEIRYNCK N** ; **GLORIEUX G** ; **LEMKE HD** ; **VANHOLDER R** ; **DIOUF M** ; **CHOUKROUN G** ; **MASSY ZA.** European Uremic Toxin Work G: Plasma beta-2 microglobulin is associated with cardiovascular disease in uremic patients.. *Kidney Int*, 2012, vol. 82, 1297-1303
- **FERGUSON TW** ; **KOMENDA P** ; **TANGRI N.** Cystatin C as a biomarker for estimating glomerular filtration rate.. *Curr Opin Nephrol Hypertens*, 2015, vol. 24, 295-300
- **LARRAYOZ IM** ; **FERRERO H** ; **MARTISOVA E** ; **GIL-BEA FJ** ; **RAMIREZ MJ** ; **MARTINEZ A.** Adrenomedullin Contributes to Age-Related Memory Loss in Mice and Is Elevated in Aging Human Brains.. *Front Mol Neurosci*, 2017, vol. 10, 384
- **SMITH LK** ; **HE Y** ; **PARK JS** ; **BIERI G** ; **SNETHLAGE CE** ; **LIN K** ; **GONTIER G** ; **WABL R** ; **PLAMBECK KE** ; **UDEOCHU J et al.** beta2-microglobulin is a systemic pro-aging factor that impairs cognitive function and neurogenesis.. *Nat Med*, 2015, vol. 21, 932-937
- **FUJITA Y** ; **TANIGUCHI Y** ; **SHINKAI S** ; **TANAKA M** ; **ITO M.** Secreted growth differentiation factor 15 as a potential biomarker for mitochondrial dysfunctions in aging and age-related disorders.. *Geriatr Gerontol Int*, 2016, vol. 16 (1), 17-29
- **CESARI M** ; **PAHOR M** ; **INCALZI RA.** Plasminogen activator inhibitor-1 (PAI-1): a key factor linking fibrinolysis and age-related subclinical and clinical conditions.. *Cardiovasc Ther*, 2010, vol. 28, e72-91

- **KHAN SS** ; **SHAH SJ** ; **KLYACHKO E** ; **BALDRIDGE AS** ; **EREN M** ; **PLACE AT** ; **AVIV A** ; **PUTERMAN E** ; **LLOYD-JONES DM** ; **HEIMAN M et al.** A null mutation in <em>SERPINE1</em> protects against biological aging in humans.. *Science Advances*, 2017, 3
- **ASHUTOSH, CHAO C** ; **BORGMANN K** ; **BREW K** ; **GHORPADE A**. Tissue inhibitor of metalloproteinases-1 protects human neurons from staurosporine and HIV-1-induced apoptosis: mechanisms and relevance to HIV-1-associated dementia.. *Cell Death Dis*, 2012, vol. 3, e332
- **RIDKER PM**. High-sensitivity C-reactive protein: potential adjunct for global risk assessment in the primary prevention of cardiovascular disease.. *Circulation*, 2001, vol. 103, 1813-1818
- **ANDERSSON C** ; **ENSERRO D** ; **SULLIVAN L** ; **WANG TJ** ; **JANUZZI JL, JR.** ; **BENJAMIN EJ** ; **VITA JA** ; **HAMBURG NM** ; **LARSON MG** ; **MITCHELL GF**. Relations of circulating GDF-15, soluble ST2, and troponin-I concentrations with vascular function in the community: The Framingham Heart Study.. *Atherosclerosis*, 2016, vol. 248, 245-251
- **ZOU H** ; **HASTIE T**. *Regularization and variable selection via the elastic net*, 2005, vol. B 67, 301
- *Journal of the Royal Statistical Society Series B-Statistical Methodology*, 2005, vol. 67, 768-768
- **ALMASY L** ; **BLANGERO J**. Multipoint quantitative-trait linkage analysis in general pedigrees.. *Am J Hum Genet*, 1998, vol. 62, 1198-1211
- **ZIYATDINOV A** ; **BRUNEL H** ; **MARTINEZ-PEREZ A** ; **BUIL A** ; **PERERA A** ; **SORIA JM**. solarius: an R interface to SOLAR for variance component analysis in pedigrees.. *Bioinformatics*, 2016, vol. 32, 1901-1902
- **HORVATH S** ; **LEVINE AJ**. HIV-1 infection accelerates age according to the epigenetic clock.. *J Infect Dis*, 2015
- **LANGFELDER P** ; **HORVATH S**. WGCNA: an R package for weighted correlation network analysis.. *BMC Bioinformatics*, 2008, vol. 9, 559
- **DAWBER, T.R.** ; **MEADORS, G.F.** ; **MOORE, F.E., JR.** Epidemiological approaches to heart disease: the Framingham Study.. *Am J Public Health Nations Health*, 1951, vol. 41, 279-81
- **KANNEL, W.B.** ; **FEINLEIB, M.** ; **MCNAMARA, P.M.** ; **GARRISON, R.J.** ; **CASTELLI, W.P.** An investigation of coronary heart disease in families. The Framingham offspring study.. *Am J Epidemiol*, 1979, vol. 110, 281-90
- **RYEE, M.J. et al.** Minfi: a flexible and comprehensive Bioconductor package for the analysis of Infinium DNA methylation microarrays.. *Bioinformatics*, 2014, vol. 30, 1363-9

- Design of the Women's Health Initiative clinical trial and observational study. The Women's Health Initiative Study Group.. *Control Clin Trials*, 1998, vol. 19, 61-109
- **ANDERSON, G. et al.** Implementation of the women's health initiative study design.. *Ann Epidemiol*, 2003, vol. 13, S5-17
- Epigenetic Mechanisms of PM-Mediated CVD Risk (WHI-EMPC; R01-ES02836). **WHITSEL, E.** Research Portfolio Online Reporting Tools. National Institutes of Health. U.S. Department of Health and Human Services, 2016
- **TESCHENDORFF, A.E. et al.** A beta-mixture quantile normalization method for correcting probe design bias in Illumina Infinium 450 k DNA methylation data.. *Bioinformatics*, 2013, vol. 29, 189-196
- **JOHNSON, W.E.** ; **RABINOVIC, A.** ; **LI, C.** Adjusting batch effects in microarray expression data using Empirical Bayes methods. *Biostatistics*, 2007, vol. 8, 118-127
- **PATTERSON, R.E. et al.** Measurement characteristics of the Women's Health Initiative food frequency questionnaire.. *Annals of epidemiology*, 1999, vol. 9, 178-187
- **TAYLOR, H.A., JR. et al.** Toward resolution of cardiovascular health disparities in African Americans: design and methods of the Jackson Heart Study.. *Ethn Dis*, 2005, vol. 15, S6-4, 17
- **MOORE, A.Z. et al.** Change in Epigenome-Wide DNA Methylation Over 9 Years and Subsequent Mortality: Results From the InCHIANTI Study.. *J Gerontol A Biol Sci Med Sci*, 2015
- **WONG, H.K.** ; **CHEUNG, T.T.** ; **CHEUNG, B.M.** Adrenomedullin and cardiovascular diseases.. *JRSM Cardiovasc Dis*, 2012, vol. 1
- **LARRAYOZ, I.M. et al.** Adrenomedullin Contributes to Age-Related Memory Loss in Mice and Is Elevated in Aging Human Brains.. *Front Mol Neurosci*, 2017, vol. 10, 384
- **LIABEUF, S. et al.** Plasma beta-2 microglobulin is associated with cardiovascular disease in uremic patients.. *Kidney Int*, 2012, vol. 82, 1297-303
- **SMITH, L.K. et al.** beta2-microglobulin is a systemic pro-aging factor that impairs cognitive function and neurogenesis.. *Nat Med*, 2015, vol. 21, 932-7
- **FERGUSON, T.W.** ; **KOMENDA, P.** ; **TANGRI, N.** Cystatin C as a biomarker for estimating glomerular filtration rate.. *Curr Opin Nephrol Hypertens*, 2015, vol. 24, 295-300
- **VAN DER LAAN, S.W. et al.** Cystatin C and Cardiovascular Disease: A Mendelian Randomization Study.. *J Am Coll Cardiol*, 2016, vol. 68, 934-45
- **PATERSON, R.W. et al.** Cerebrospinal fluid markers including trefoil factor 3 are associated with neurodegeneration in amyloid-positive individuals.. *Transl Psychiatry*, 2014, vol. 4, e419

- **FUJITA, Y.** ; **TANIGUCHI, Y.** ; **SHINKAI, S.** ; **TANAKA, M.** ; **ITO, M.** Secreted growth differentiation factor 15 as a potential biomarker for mitochondrial dysfunctions in aging and age-related disorders.. *Geriatr Gerontol Int*, 2016, vol. 16 (1), 17-29
- **MAIOLI, S. et al.** Alterations in brain leptin signalling in spite of unchanged CSF leptin levels in Alzheimer's disease.. *Aging Cell*, 2015, vol. 14, 122-9
- **CESARI, M.** ; **PAHOR, M.** ; **INCALZI, R.A.** Plasminogen activator inhibitor-1 (PAI-1): a key factor linking fibrinolysis and age-related subclinical and clinical conditions.. *Cardiovasc Ther*, 2010, vol. 28, e72-91
- **ASHUTOSH, CHAO, C.** ; **BORGMANN, K.** ; **BREW, K.** ; **GHORPADE, A.** Tissue inhibitor of metalloproteinases-1 protects human neurons from staurosporine and HIV-1-induced apoptosis: mechanisms and relevance to HIV-1-associated dementia.. *Cell Death Dis*, 2012, vol. 3, e332
- **HOUSEMAN, E. et al.** DNA methylation arrays as surrogate measures of cell mixture distribution.. *BMC Bioinformatics*, 2012, vol. 13, 86
- **HORVATH, S.** DNA methylation age of human tissues and cell types.. *Genome Biol*, 2013, vol. 14, R115
- **HORVATH, S.** ; **LEVINE, A.J.** HIV-1 Infection Accelerates Age According to the Epigenetic Clock.. *J Infect Dis*, 2015, vol. 212, 1563-73
- **HORVATH, S. et al.** An epigenetic clock analysis of race/ethnicity, sex, and coronary heart disease.. *Genome Biol*, 2016, vol. 17, 171
- **HANNUM, G. et al.** Genome-wide methylation profiles reveal quantitative views of human aging rates.. *Mol Cell*, 2013, vol. 49, 359-67
- **LEVINE, M.E. et al.** An epigenetic biomarker of aging for lifespan and healthspan.. *Aging (Albany NY)*, 2018, vol. 10, 573-591
- **ZHANG, Y. et al.** DNA methylation signatures in peripheral blood strongly predict all-cause mortality.. *Nat Commun*, 2017, vol. 8, 14617
- **HORVATH, S.** ; **LEVINE, A.J.** HIV-1 infection accelerates age according to the epigenetic clock.. *J Infect Dis*, 2015
- **LANGFELDER, P.** ; **HORVATH, S.** WGCNA: an R package for weighted correlation network analysis.. *BMC Bioinformatics*, 2008, vol. 9, 559